(19) Europäisches Patentamt
European Patent Office
Office européen des brevets

(11) Veröffentlichungsnummer : **0 348 766 B1**

(12) # EUROPÄISCHE PATENTSCHRIFT

(45) Veröffentlichungstag der Patentschrift : **17.04.91 Patentblatt 91/16**

(21) Anmeldenummer : **89111039.7**

(22) Anmeldetag : **19.06.89**

(51) Int. Cl.⁵ : **C07C 69/734,** C07C 69/732, C07C 69/74, C07C 69/65, A01N 37/38, A01N 37/10, A01N 53/00, C07C 69/78, C07C 69/618, C07C 69/736, C07D 277/74

(54) **Alfa-Aryl-acrylsäureester und diese enthaltende Fungizide.**

(30) Priorität : **25.06.88 DE 3821503**

(43) Veröffentlichungstag der Anmeldung : **03.01.90 Patentblatt 90/01**

(45) Bekanntmachung des Hinweises auf die Patenterteilung : **17.04.91 Patentblatt 91/16**

(84) Benannte Vertragsstaaten : **AT BE CH DE ES FR GB GR IT LI NL SE**

(56) Entgegenhaltungen :
**JOURNAL OF THE AMERICAN CHEMICAL SOCIETY, Band 98, Nr. 12, 9. Juni 1976, Seiten 3555-3564, The American Chemical Society, Columbus, Ohio, US; A. PADWA et al.: "Carbonyl group photochemistry via the enol form. Photoisomerization of 4-substituted 3-chromanones"**
**C.A.S. REGISTRY HANDBOOK, Nr. Sektion-1979 Supplement, Seite 1557RH, The American Chemical Society, Columbus, Ohio, US; RN-70932-72-8: "Benzeneacetic acid, 2-(methoxycarbonyl-alfa-methylene-, methyl ester C12H12O4"**

(73) Patentinhaber : **BASF Aktiengesellschaft
Carl-Bosch-Strasse 38
W-6700 Ludwigshafen (DE)**

(72) Erfinder : **Brand, Siegbert, Dr.
Hegelstrasse 39
W-6940 Weinheim (DE)**
Erfinder : **Wenderoth, Bernd, Dr.
Schwalbenstrasse 26
W-6840 Lampertheim (DE)**
Erfinder : **Schuetz, Franz, Dr.
Budapester Strasse 45
W-6700 Ludwigshafen (DE)**
Erfinder : **Sauter, Hubert, Dr.
Neckarpromenade 20
W-6800 Mannheim 1 (DE)**
Erfinder : **Ammermann, Eberhard, Dr.
Sachsenstrasse 3
W-6700 Ludwigshafen (DE)**
Erfinder : **Lorenz, Gisela, Dr.
Erlenweg 13
W-6730 Neustadt (DE)**

**EP 0 348 766 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue α-Aryl-acrylsäureester, ihre Herstellung und ihre Verwendung als Fungizide.

Es ist der α-Phenyl-acrylsäuremethylester bekannt (C.A. Reg. No. 1865-29-8).

Es wurde nun gefunden, daß neue α-Aryl-acrylsäureesterderivate der Formel I

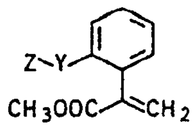

(I)

in der

Y gegebenenfalls substituiertes $C_1$-$C_4$-Alkylen, gegebenenfalls substituiertes $C_2$-$C_4$-Alkenylen, $C_2$-$C_4$-Alkinylen, $O,S(O)_m$ (m = 0, 1, 2), gegebenenfalls $C_1$-$C_4$-alkylsubstituiertes N, Oxycarbonyl, Carbonyloxy, $C_1$-$C_{10}$-Oxycarbonylalkylen, $C_1$-$C_{10}$-Carbonyloxyalkylen, $C_2$-$C_{10}$-Oxyalkylenoxy, $C_1$-$C_{10}$-Oxyalkylen, $C_1$-$C_{10}$-Alkylenoxy, $C_1$-$C_{10}$-Thioalkylen, Azo, gegebenenfalls $C_1$-$C_4$-alkylsubstituiertes Carbonylamino, gegebenenfalls $C_1$-$C_4$-alkylsubstituiertes Aminocarbonyl, gegebenenfalls $C_1$-$C_4$-alkylsubstituiertes Aminocarbonyloxy

und

Z Wasserstoff, Halogen, $C_1$-$C_{18}$-Alkyl, $C_2$-$C_{18}$-Alkenyl, $C_3$-$C_8$-Cycloalkyl, $C_2$-$C_4$-Alkinyl, Aryl, Aryl-$C_1$-$C_{10}$-alkyl, Aryl-$C_2$-$C_{10}$-alkenyl, Aryloxy, Aryloxy-$C_1$-$C_{10}$-alkyl, Alkoxy-$C_1$-$C_{10}$-alkyl, Halogen-$C_1$-$C_{10}$-alkyl, Aryloxy-$C_1$-$C_4$-alkoxy, $C_1$-$C_4$-Alkoxycarbonyl, die gegebenenfalls substituiert sind mit Halogen, Cyano, Nitro, $C_1$-$C_4$-Alkyl, $C_2$-$C_4$Alkenyl, $C_1$-$C_4$-Alkoxy, $C_1$-$C_4$-Halogenalkyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten gegebenenfalls einen aromatischen oder heteroaromatischen Ring bilden der gegebenenfalls an eine Ethenyleinheit geknüpft ist, außer den Verbindungen, in denen Y O und Z H oder $CH_3$ und in denen Z-Y $CH_3OOC$ bedeuten, eine sehr gute fungizide Wirkung haben, die besser als die der bekannten Fungizide ist.

Y bedeutet bevorzugt Methylen, Ethylen, Ethenylen, Ethinylen, O, S, Methylenoxy, Ethylenoxy, Oxymethylen, Thiomethylen, Carbonyloxy, Oxycarbonyl, Carbonyloxymethylen, – HN-CO-O –.

Z bedeutet bevorzugt Wasserstoff, Halogen, (z.B. Fluor, Chlor, Brom), $C_1$-$C_{18}$-Alkyl (z.B. Methyl, Ethyl, n- und iso-Propyl, n-, sec-, iso- und tert.-Butyl, 3-Methylbutyl, 2,2-Dimethylpropyl, 1,1-Dimethylpropyl, 3-Hexyl, n-Pentyl, n-Hexyl, n-Heptyl, n-Octyl, n-Nonyl, n-Decyl, n-Pentadecyl, n-Heptadecyl, 2,6-Dimethylhept-1-yl) ; Cyanmethyl ; $C_2$-$C_9$-Alkenyl (z.B. Vinyl, 2-Propen-2-yl, 1-Propen-1-yl, 2-Buten-2-yl, 2-Methyl-prop-1-en-1-yl, Allyl, 2-Buten-1-yl, 3-Methyl-2-buten-1-yl, 1,3-Pentadien-1-yl, 2,6-Dimethyl-1,5-heptadien-1-yl, 2,6-Dimethylhept-5-en-1-yl) ; $C_2$-Alkinyl (z.B. Ethinyl, 2-Phenylethinyl) ; $C_1$-$C_2$-Alkoxy-$C_1$-$C_2$-alkyl (Methoxymethyl, Ethoxymethyl, 1-Methoxyethyl), $C_3$-$C_6$-Cycloalkyl (z.B. Cyclopropyl, Cyclobutyl, Cyclopentyl, Cyclohexyl, 1-Methylcyclopropyl), wobei z.B. folgende substituierte Cyclopropylreste gemeint sind :

2,2-Dichlor-1-methyl-cyclopropyl (A1)
2,2-Dichlor-3,3-dimethyl-cyclopropyl (A2)
2,2,3,3-Tetramethylcyclopropyl (A3)
2-(2′-Methyl-1′-propenyl-)-3,3-dimethylcyclopropyl (A4)
2-(2′, 2′-Difluorvinyl-)-3,3-dimethylcyclopropyl (A5)
2-(2′, 2′-Dichlorvinyl)-3,3-dimethylcyclopropyl (A6)
2-(2′, 2′-Dibromvinyl)-3,3-dimethylcyclopropyl (A7)
2-Phenylcyclopropyl (A8)
2-(4′-Chlorphenyl)cyclopropyl (A9)
2,2-Dichlor-3-phenylcyclopropyl (A10)
2-Carbomethoxy-cyclopropyl (A11)

und Halogen-$C_1$-$C_2$-alkyl (z.B. Chlormethyl, 1-Chlorethyl, Dichlormethyl, Trichlormethyl, Brommethyl, Trifluormethyl), Phenyl, substituiertes Phenyl, wie Halogenphenyl (z.B. 2-Fluor-, 3-Fluor-, 4-Fluor-, 6-Fluor, 2-Chlor-, 2,4-Difluor-, 2,6-Difluor-, 2,3,4,5,6-Pentafluor-, 2-Chlor-, 3-Chlor-, 4-Chlor-, 2,4-Dichlor-, 2,5-Dichlor-, 2,6-Dichlor-, 3,4-Dichlor-, 3,5-Dichlor-, 2,4,5-Trichlor-, 2,3,4,5,6-Pentachlor-, 2-Brom-, 3-Brom-, 4-Bromphenyl) ;

$C_1$-$C_4$-Alkylphenyl (z.B. 2-Methyl-, 3-Methyl, 4-Methyl-, 2,3-Dimethyl-, 2,4-Dimethyl-, 2,5-Dimethyl-, 2,6-Dimethyl-, 3,4-Dimethyl-, 3,5-Dimethyl-, 2,4,6-Trimethyl-, 4-t-Butyl-Phenyl) ; Arylphenyl (z.B. 2-Phenyl-, 4-Phe-

nylphenyl) ; Halogen-$C_1$-alkylphenyl (z.B. 2-Trifluor-, 3-Trifluor-, 4-Trifluormethylphenyl) ; $C_1$-$C_4$-Alkoxyphenyl (z.B. 2-Methoxy-, 3-Methoxy-, 4-Methoxy-, 3,4-Dimethoxy-, 3,4,5,-Trimethoxy-, 4-t-Butoxyphenyl) ; 2-, 3-, 4-Phenoxyphenyl ;

substituiertes Benzyl (z.B. Halogenbenzyl, 2-Fluor-, 3-Fluor-, 4-Fluor-, 2-Chlor-, 3-Chlor-, 4-Chlor-, 2-Chlor-, 6-Fluor-, 2,4-Dichlor-, 2,6-Dichlor-, 3,5-Dichlor-, 2,4,6-Trichlor-, 2-Brom-, 3-Brom-, 4-Brom-, 2-Methyl-, 3-Methyl-, 4-Methyl-, 4-t-Butyl-, 2,4-Dimethyl-, 2,6-Dimethyl, 2,4,6-Trimethyl-, 2-Methoxy, 3-Methoxy-, 4-Methoxy-, 2-Trifluormethyl-, 3-Trifluormethyl-, 4-Trifluormethyl-, 4-t-Butoxy-, 4-Phenoxy-, 4-Phenylbenzyl, α-Methyl-α-ethyl, α-iso-Propyl-, α-Hydroxy-, 2-Methoxy-α-hydroxy-, 4-Methoxy-α-hydroxy-, 3,4-Dimethoxy-α-hydroxy, 2-Methoxy-α-methoxy-, 4-Methoxy-α-methoxy-, 3,4-Dimethoxy-α-methoxybenzyl) ; gegebenenfalls substituiertes Phenethyl (z.B. Phenyl-, 1-methyl-2-phenyl-, 2-(para-t-Butylphenyl-), 2-(para-t-Butylphenyl)-1-Methyl-, 2-(ortho-Chlorphenyl)-, 2-(meta-Chlorphenyl)-, 2-(para-Chlorphenyl)-ethyl) ; gegebenenfalls substituiertes Styryl (z.B. Styryl ; 2′-Chlor-, 3′-Chlor-, 4′-Chlor-, 2′,4′-Dichlor-, 2′-Fluor-, 4′-Fluor-, 2′-Methyl-, 4′-Methyl-, 4′-t-Butyl-, 2′-Methoxy-, 4′-Methoxy-, 4′-Phenoxy-styryl) ;

Phenyl-$C_3$-$C_6$-alkyl (z.B. 3-Phenylpropyl, 2-Methyl-3-Phenylpropyl, 4-Phenylbutyl, 5-Phenylpentyl, 6-Phenylhexyl, 3-(4′-t-Butylphenyl)-2-Methyl-propyl) ;

Aryloxy, wie substituiertes Phenoxy (z.B. 2-Chlor-, 3-Chlor-, 4-Chlor-, 2-Methyl-, 4-Methyl-, 2-Methoxy- 4-Methoxy-, 2-Trifluormethyl-, 4-Trifluormethyl-phenoxy) ; Aryloxy-$C_1$-$C_6$-alkyl, wie gegebenenfalls substituiertes Phenoxymethyl (z.B. 2-Chlor-, 4-Chlor-, 2-Methyl-, 4-Methyl-, 2-Methoxy-, 4-Methoxy-, 4-t-Butyl-phenoxymethyl) ; gegebenenfalls substituiertes Phenoxyethyl (z.B. 2-Chlor-, 4-Chlor-, 4-Fluor-, 2-Methyl-, 4-Methyl-, 2-Methoxy-, 4-Methoxy-, 4-t-Butyl-phenoxyethyl), gegebenenfalls substituiertes $C_3$-$C_6$-Phenoxyalkyl (z.B. 3-Phenoxypropyl, 3-(ortho-Chlorphenoxy)propyl, 3-(para-Chlor-phenoxy)propyl, 4-Phenoxybutyl, 4-(ortho-Chlorphenoxy)-butyl, 4-(para-Chlorphenoxybutyl), 5-Phenoxypentyl, 5-(ortho-Chlorphenoxy)pentyl, 5-(para-Chlorphenoxy)pentyl, 6-Phenoxyhexyl) ; Phenoxyethoxy ; Methoxycarbonyl, t-Butoxycarbonyl ; gegebenenfalls substituiertes Heteroaryl (z.B. Furyl, 2-Furyl, 3-Furyl, 5-Nitro-2- und 3-furyl, 5-Chlor-2- und 3-furyl, Benzofuran-2- und 3-yl, Thienyl, 2-Thienyl, 3-Thienyl, 5-Nitro-2- und 3-thienyl, 5-Chlor-2- und 3-thienyl, Benzothien-2- und 3-yl.

N-Methyl-pyrrol-2- und 3-yl, N-Methyl-pyrazol-3-, 4- und 5-yl, N-Methylimidazol-2-, 4- und 5-yl, 1-Methyl-1,2,3-triazol-4- und 5-yl, 1-Methyl-1,2,4-triazol-3- und 5-yl, 1-Methyl-tetrazol-5-yl, Isoxazol-3-, 4- und 5-yl, Benzisoxazol-3-yl, Benzoxazol-2-yl, Oxazol-2-, 4- und 5-yl, Thiazol-2-, 4- und 5-yl, Benzothiazol-2-yl, Benzisothiazol-3-yl, Isothiazol-3-, 4- und 5-yl, 1,2,3-Thiadiazol-4-yl, 1,2,4-Thiadiazol-5-yl, 1,3,4-Thiadiazol-3-yl, 1,3-Thiazolo-[4,5-b]-pyridin-2-yl) ; substituiertes Hetarylalkenyl (z.B. 2-(2′- und 3′-Furyl)-, 2-(5′-Nitro-2′- und 3′-furyl)-, 2-(2′- und 3′- Thienyl)-, 2-(5′-Nitro-2′- und 3′-thienyl)ethenyl).

Die Verbindungen der Formel I können z.B. hergestellt werden durch die in Schema 1 gezeigten Umsetzungen.

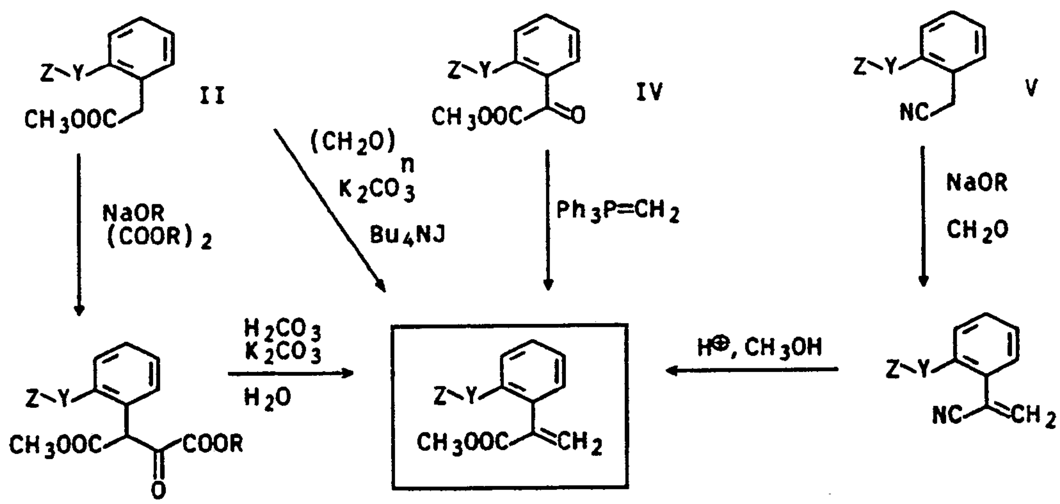


II IV V

III I VI

R=$CH_3$, $C_2H_5$ Schema 1

Die Phenylessigsäureester der Formel II werden in an sich bekannter Weise über die 3-Aryl-2-Oxo-bernsteinsäureester III in die α-Arylacrylsäuremethylester I umgewandelt (vgl. E. Galantay et al., J. Org. Chem. 35,

4277 (1970)). Dazu acyliert man die Phenylessigester (II) mit Dialkyloxalat mit Hilfe einer Base, wie z.B. Natriummethanolat oder Natriumethanolat zu den Diestern III und addiert hieran Formaldehyd. Durch Hydrolyse mit wässiger Base, wie z.B. Kaliumcarbonat erhält man die Atropasäurederivate I. Direkt lassen sich die Verbindungen der Formel I aus Phenylessigestern II mit Paraformaldehyd, Kaliumcarbonat und katalytischen Mengen eines Phasentransferkatalysators, wie z.B. Tetrabutylammoniumiodid in z.B. Toluol herstellen. (s. W. Seitz, A. Michel, DE 3 317 356).

Die α-Ketocarbonsäureester der Formel IV lassen sich über eine Wittigreaktion mit Methylentriphenylphosphoran in an sich bekannter Weise in Gegenwart einer Base wie z.B. n-Butyllithium, Kalium-tert-butylat, Natriumhydrid oder Natriummethylat in die Acrylester der Formel I umwandeln (vgl. G. Wittig, U. Schöllkopf, Org. Synth., Coll. Vol. V 751 (1973)).

Die Herstellung der α-Ketoester der Formel IV ist bekannt (EP 178826). So setzt man z.B. die aromatischen Grignardverbindungen VII mit Imidazoliden der Formel VIII um (J.S. Nimitz, H.S. Mosher, J. Org. Chem. 46, 211 (1981)) wobei die Reste Y und Z die oben genannten Bedeutungen haben.

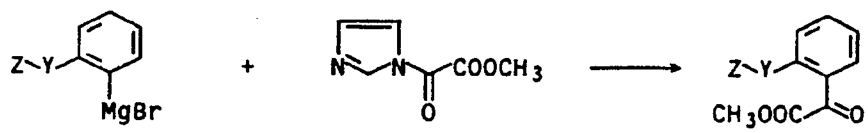

VII VIII IV

Die Oxidation substituierter Acetophenone (IX) mit Kaliumpermanganat in Pyridin/Wasser/Kaliumhydroxid

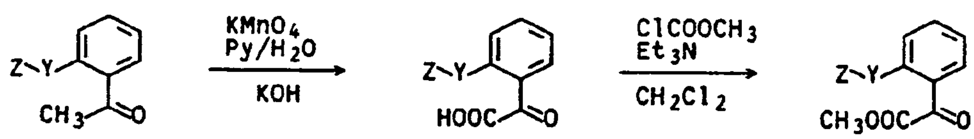


IX X IV

(vgl. J.W.F. Mc Omie, S.D. Thata, J. Chem. Soc. 1965, 5298) liefert die Phenylketosäuren X, die sich mit z.B. Chlorameisensäuremethylester/Triethylamin zu den α-Ketoestern IV verestern lassen (vgl. J.M. Domagala, Tetrahedron Lett. 21, 4957 (1980)).

Die Benzylcyanide V kann man in die α-Arylacrylnitrile der Formel VI überführen, indem man sie mit einer Base, wie z.B. Natriummethanolat und Formaldehyd behandelt (vgl. J.M. Stewart, C.H. Chang, J. Org. Chem. 21, 635 (1956)).

Die Umwandlung der Nitrile VI zu den Acrylestern der Formel I erfolgt mit methanolischer Mineralsäure, wie z.B. Salzsäure oder Schwefelsäure (vgl. E.N. Zil'berman Russ. Chem. Rev. 31, 615 (1962)).

Die Herstellung der neuen Verbindungen der Formel I wird durch folgende Beispiele erläutert :

Beispiel 1

Herstellung von 2-[2'-Cinnamoyloxymethyl)phenyl]-acrylsäuremethylester (Nr. 866)

a) Herstellung von ortho-Brommethylphenylglyoxylsäuremethylester

50,0 g (0,28 mol) ortho-Methyl-Phenylglyoxylsäuremethylester werden zusammen mit 50,0 g (0,28 mol) frisch kristallisiertem N-Bromsuccinimid in 3 Liter Tetrachlorkohlenstoff gelöst. Man belichtet eine Stunde mit einer 300 W-Hg-Dampf-Lampe, engt die Reaktionsmischung auf ca. 1 Liter ein und wäscht die organische Phase mit 3 × 200 ml Wasser. Nach Trocknen über Natriumsulfat und Abdestillieren des Lösemittels wird der Rückstand an Kieselgel mit Methyl-tert.-butyl-ether/Hexan 1 : 9 chromatographiert. Man erhält 10 g Ketoester (II, YZ = $CH_3$) und 21,1 g Benzylbromid (II, Y-Z = $CH_2Br$) (37% bezogen auf umgesetzten Ketoester) als gelbes Öl.

$^1H$ ($CDCl_3$) : δ = 3,97 (s, 3H), 4,90 (s, 2H), 7,4-7,8 (m, 4H)

IR (Film) : 2955 ; 1740, 1690 ; 1435, 1318, 1207, 999 $cm^{-1}$.

b) Herstellung von 2-(Cinnamoylmethyl)-phenylglyoxylsäuremethylester

10,4 g (56 mmol) trans-Zimtsäure-Kaliumsalz wird mit 12,0 g (47 mmol) ortho-Brommethylphenylglyoxylsäuremethylester in 250 ml N-Methylpyrrolidon gelöst und nach Zusatz einer Spatelspitze Kaliumiodid 15 h bei Raumtemp. (23°C) gerührt. Dann gießt man auf 500 ml Eiswasser, extrahiert mit 3 × 200 ml Methyl-tert.-Butylether, wäscht die org. Phasen mit Wasser, trocknet sie über Natriumsulfat und engt sie am Rotationsverdampfer ein. Die Ausbeute an Ketoster beträgt 15,0 g (99%). Er ist dünnschichtchromatographisch nahezu rein.
$^1$H-NMR ($CDCl_3$) : δ = 3.97 (s ; 3H) ; 5.62 (s ; 2H) ; 6.55 (d, J = 10 Hz ; 1H) ; 7.3-7.85 (m ; 9H) ; 7.77 (d, J = 10 Hz ; 1H)
IR (Film) : 1728, 1688, 1638, 1210, 1168, 998, 978, 765 $cm^{-1}$

c) Herstellung von 2-[2'-(Cinnamoyloxymethyl)phenyl]-acrylsäuremethylester (Nr. 866)

10,5 g (26 mmol) Methyltriphenylphosphoniumiodid werden unter Stickstoffatmosphäre in 100 ml wasserfreiem Tetrahydrofuran vorgelegt. Bei 0°C tropft man 16,5 ml einer 1,6 M Lösung von n-Butyllithium in n-Hexan zu und läßt 1 h bei 0°C nachrühren. Nach dem Abkühlen auf – 78°C werden 7,5 g (23 mmol) des gemäß b) erhaltenen Ketoesters in 50 ml Tetrahydrofuran langsam zugetropft. Man läßt auf Raumtemperatur kommen und rührt 12 h nach. Es wird in Ammoniumchloridlösung eingegossen und mit Methyl-tert.-Butylether extrahiert. Die organischen Phasen wäscht man mit Wasser, trocknet über Natriumsulfat und engt ein. Das Produkt wird durch Säulenchromatographie an Kieselgel mit MtBE/n-Hexan 1 : 9 gewonnen. Man erhält 2,1 g (28%) des obengenannten Acrylesters als Öl.
$^1$H-NMR ($CDCl_3$) : δ = 3.75 (s ; 3H) ; 5.18 (s ; 2H) ; 5.8 (d, J = 1 Hz ; 1H) ; 6.42 (d, J = 10 Hz ; 1H) ; 6.60 (d, J = 1 Hz ; 1H) ; 7.15-7.60 (m ; 9H) ; 7.68 (d, J = 10 Hz ; 1H)
IR (Film) : 1719, 1637, 1311, 1204, 1164, 768 $cm^{-1}$

Beispiel 2

Herstellung der Vorprodukte

Herstellung von 2-Methylphenylglyoxylsäuremethylester

1,4 g (10 mmol) ortho-Methylacetophenon, 2,0 g Kaliumhydroxid, 200 ml Pyridin und 100 ml Wasser werden bei +10°C vorgelegt. Dazu tropft man 5 g Kaliumpermanganat in 250 ml Wasser und rührt 2 Stunden bei dieser Temperatur nach. Sodann tropft man soviel Natriumbisulfitlauge zu bis die Rotfärbung der Mischung verschwindet. Es wird filtriert, das Filtrat mit konz. Salzsäure angesäuert, erneut filtriert und mit 5 × 50 ml Methyl-tert.-Butylether extrahiert. Nach Trocknen über Natriumsulfat und Einengen wird direkt weiter umgesetzt (Verhältnis Ketosäure/Benzoesäure 8-9 : 1 laut $^1$H-NMR). 2,0 g des obigen Gemisches werden in 20 ml Dichlormethan vorgelegt und mit 1,6 g (16 mmol) Triethylamin versetzt. 1,5 g (16 mmol) Chlorameisensäuremethylester werden bei Raumtemperatur zugetropft. Nach 1 h bei dieser Temperatur extrahiert man mit Phosphatpuffer (pH 7), engt ein und destilliert.
Sdp. 95-99°C/0,4 mbar ; Ausbeute : 0,92 g (51%)
IR (Film) : 1740, 1686, 1602, 1457, 1317, 1285, 1203, 1002, 739 $cm^{-1}$

Beispiel 3

Herstellung von 2-[2'-(Benzyloxy)phenyl]acrylsäuremethylester (Nr. 446)

25,6 g (0,10 mol) ortho-Benzyloxyphenylessigsäuremethylester, 4,5 g (0,15 mol) Paraformaldehyd, 23,5 g (0,16 mol) Kaliumcarbonat und 600 mg (2 mmol) Tetrabutylammoniumiodid werden zu 50 ml Toluol gegeben. Nach 3-4 Stunden Rühren bei 80-85°C wird abgekühlt und 50 ml Wasser zugegeben. Nach Schütteln und Trennen der Phasen wird die wäßrige Phase mit 2 × 50 ml Toluol und das Toluol und die organische Phase mit 2 × 50 ml gesätt. Kochsalzlösung extrahiert. Trocknen mit $Na_2SO_4$ und Einengen führt zu einem gelblichen Öl, das am Kieselgel mit Methyl-tert.Butylether/Hexan 1 : 2 chromatographiert wird. Ausbeute : 16,4 g (61%).
IR (Film) : 1727, 1602, 1495, 1451, 1276, 1242, 1268, 753, 735, 696 $cm^{-1}$
$^1$H-NMR ($CDCl_3$) : δ = 3.60 (s ; 3H) ; 5.07 (s ; 2H) ; 5.23 (d, J = 1 Hz ; 1H) ; 6.26 (d, J = 1 Hz ; 1H) ; 6.8-7.0 (m; 2H) ; 7.1-7.4 (m ; 7H)

Beispiel 4

Herstellung von 2-(2'-Methoxycarbonyl-ethen-1'-yl)-benzyl-triphenylphosphoniumbromid (Nr. 926)

14 g (80 mmol) 2-(2'-Methylphenyl)acrylsäuremethylester, 28.5 g (160 mmol) N-Bromsuccinimid und 500 ml Tetrachlorkohlenstoff werden zusammengegeben und 3 h mit einer Quecksilberdampflampe (300 W) unter Rückfluß bestrahlt. Nach dem Abkühlen wird mit Wasser gewaschen, mit Natriumsulfat getrocknet und eingeengt. Das Rohprodukt (2-(2'-Brommethylphenyl)-acrylsäuremethylester (Nr. 925)) wird in 500 ml Tetrahydrofuran gelöst, mit 21 g (80 mmol) Triphenylphosphan versetzt und 15 h bei 23°C, dann 4 h bei 70°C gekocht. Die Mischung wird gekühlt, abgesaugt, mit Methyl-tert.-Butylether gewaschen und i.Vak. getrocknet. Ausbeute an weißem Pulver : 16,0 g (39%).
Fp. : 145-147°C
$^1$H-NMR ($CDCl_3$ ; Nr. 925) : $\delta$ = 3.78 (s ; 3H) ; 4.42 (s ; 2H) ; 5.90 (d, J=1 Hz ; 1H) ; 6.65 (d, J=1 Hz ; 1H) ; 7.1-7.5 (m ; 4H)
$^1$H-NMR ($CDCl_3$ ; Nr. 926) : $\delta$ = 3.62 (s ; 3H) ; 5.07 (sbr ; 1H) ; 5.15 (d. J = 1 Hz ; 2H) ; 6.27 (sbr ; 1H) ; 7.0-7.85 (m ; 19H)

| Nr. | Z | Y | Daten |
|---|---|---|---|
| 1 | H | -CH=CH- | |
| 2 | $CH_3$ | -CH=CH- | |
| 3 | $C_2H_5$ | -CH=CH- | |
| 4 | $n-C_3H_7$ | -CH=CH- | |
| 5 | $iso-C_3H_7$ | -CH=CH- | |
| 6 | $n-C_4H_9$ | -CH=CH- | |
| 7 | $(CH_3)_2CHCH_2$ | -CH=CH- | |
| 8 | $CH_3CH_2CH(CH_3)$ | -CH=CH- | |
| 9 | $tert.C_4H_9$ | -CH=CH- | |
| 10 | $CH_3CH_2C(CH_3)_2$ | -CH=CH- | |
| 11 | $(CH_3)_3CCH_2$ | -CH=CH- | |
| 12 | $(CH_3)_2CHCH_2CH_2$ | -CH=CH- | |
| 13 | $CH_3CH_2CH_2CH(C_2H_5)$ | -CH=CH- | |
| 14 | $n-C_5H_{11}$ | -CH=CH- | |
| 15 | $n-C_6H_{13}$ | -CH=CH- | |
| 16 | $n-C_7H_{15}$ | -CH=CH- | |
| 17 | $n-C_8H_{17}$ | -CH=CH- | |
| 18 | $n-C_9H_{19}$ | -CH=CH- | |
| 19 | $n-C_{10}H_{21}$ | -CH=CH- | |
| 20 | $n-C_{15}H_{31}$ | -CH=CH- | |
| 21 | $n-C_{17}H_{35}$ | -CH=CH- | |
| 22 | $CH_2=CH$ | -CH=CH- | |
| 23 | $CH_2=C(CH_3)$ | -CH=CH- | |
| 24 | $CH_3CH=CH$ | -CH=CH- | |
| 25 | $CH_3CH=C(CH_3)$ | -CH=CH- | |

| Nr. | Z | Y | Daten |
|---|---|---|---|
| 26 | $(CH_3)_2C=CH$ | -CH=CH- | |
| 27 | $CH_2=CHCH_2$ | -CH=CH- | |
| 28 | $CH_3CH=CHCH_2$ | -CH=CH- | |
| 29 | $(CH_3)_2C=CHCH_2$ | -CH=CH- | |
| 30 | $CH_3CH=CH-CH=CH$ | -CH=CH- | |
| 31 | $(CH_3)_2C=CHCH_2CH_2C(CH_3)=CH$ | -CH=CH- | |
| 32 | $(CH_3)_2CHCH_2CH_2CH_2CH(CH_3)CH_2$ | -CH=CH- | |
| 33 | $(CH_3)_2C=CHCH_2CH_2CH(CH_3)CH_2$ | -CH=CH- | |
| 34 | $HC \equiv C$ | -CH=CH- | |
| 35 | $C_6H_5-C \equiv C$ | -CH=CH- | |
| 36 | $CH_3OCH_2$ | -CH=CH- | |
| 37 | $C_2H_5OCH_2$ | -CH=CH- | |
| 38 | $CH_3CH(OCH_3)$ | -CH=CH- | |
| 39 | $CN-CH_2$ | -CH=CH- | |
| 40 | Cyclopropyl | -CH=CH- | |
| 41 | Cyclobutyl | -CH=CH- | |
| 42 | Cyclopentyl | -CH=CH- | |
| 43 | Cyclohexyl | -CH=CH- | |
| 44 | 1-Methylcyclopropyl | -CH=CH- | |
| 45 | 2,2-Dichlor-1-Methyl-cyclopropyl | -CH=CH- | |
| 46 | 2,2-Dichlor-3,3-Dimethyl-cyclopropyl | -CH=CH- | |
| 47 | 2,2,3,3-Tetramethylcyclopropyl | -CH=CH- | |
| 48 | 2-(2'-Methyl-1'-propenyl-)-3,3-Dimethylcyclopropyl | -CH=CH- | |
| 49 | 2-(2',2'-Difluorvinyl)-3,3-Dimethylcyclopropyl | -CH=CH- | |
| 50 | 2-(2',2'-Dichlorvinyl)-3,3-Dimethylcyclopropyl | -CH=CH- | |

| Nr. | Z | Y | Daten |
|---|---|---|---|
| 51 | 2-(2',2'Dibromvinyl)-3,3-Dimethylcyclopropyl | -CH=CH- | |
| 52 | 2-Phenylcyclopropyl | -CH=CH- | |
| 53 | 2-(4'-Chlorphenyl)cyclopropyl | -CH=CH- | |
| 54 | 2,2-Dichlor-3-Phenylcyclopropyl | -CH=CH- | |
| 55 | 2-Carbomethoxy-cyclopropyl | -CH=CH- | |
| 56 | $ClCH_2$ | -CH=CH- | |
| 57 | $Cl_2CH$ | -CH=CH- | |
| 58 | $Cl_3C$ | -CH=CH- | |
| 59 | $BrCH_2$ | -CH=CH- | |
| 60 | $CF_3$ | -CH=CH- | |
| 61 | $CH_3CH(Cl)$ | -CH=CH- | |
| 62 | $C_6H_5$ (=Phenyl) | -CH=CH- | |
| 63 | $2-CH_3-C_6H_4$ | -CH=CH- | |
| 64 | $3-CH_3-C_6H_4$ | -CH=CH- | |
| 65 | $4-CH_3-C_6H_4$ | -CH=CH- | |
| 66 | $2,3-(CH_3)_2-C_6H_3$ | -CH=CH- | |
| 67 | $2,4-(CH_3)_2-C_6H_3$ | -CH=CH- | |
| 68 | $2,6-(CH_3)_2-C_6H_3$ | -CH=CH- | |
| 69 | $3,4-(CH_3)_2-C_6H_3$ | -CH=CH- | |
| 70 | $3,5-(CH_3)_2-C_6H_3$ | -CH=CH- | |
| 71 | $2,4,6-(CH_3)_2-C_6H_2$ | -CH=CH- | |
| 72 | $4-t-C_4H_9-C_6H_4$ | -CH=CH- | |
| 73 | $2-C_6H_5-C_6H_4$ | -CH=CH- | |
| 74 | $4-C_6H_5-C_6H_4$ | -CH=CH- | |
| 75 | $2-Cl-C_6H_4$ | -CH=CH- | |

| Nr. | Z | Y | Daten |
|---|---|---|---|
| 76 | $3-Cl-C_6H_4$ | $-CH=CH-$ | |
| 77 | $4-Cl-C_6H_4$ | $-CH=CH-$ | |
| 78 | $2,4-Cl_2-C_6H_3$ | $-CH=CH-$ | |
| 79 | $2,5-Cl_2-C_6H_3$ | $-CH=CH-$ | |
| 80 | $2,6-Cl_2-C_6H_3$ | $-CH=CH-$ | |
| 81 | $3,4-Cl_2-C_6H_3$ | $-CH=CH-$ | |
| 82 | $3,5-Cl_2-C_6H_3$ | $-CH=CH-$ | |
| 83 | $2,4,5-Cl_3-C_6H_2$ | $-CH=CH-$ | |
| 84 | $2,3,4,5,6-Cl_5-C_6$ | $-CH=CH-$ | |
| 85 | $6-F,2-Cl-C_6H_3$ | $-CH=CH-$ | |
| 86 | $2-F-C_6H_4$ | $-CH=CH-$ | |
| 87 | $3-F-C_6H_4$ | $-CH=CH-$ | |
| 88 | $4-F-C_6H_4$ | $-CH=CH-$ | |
| 89 | $2,4-F_2-C_6H_3$ | $-CH=CH-$ | |
| 90 | $2,6-F_2-C_6H_3$ | $-CH=CH-$ | |
| 91 | $2,3,4,5,6-F_5-C_6$ | $-CH=CH-$ | |
| 92 | $2-Br-C_6H_4$ | $-CH=CH-$ | |
| 93 | $3-Br-C_6H_4$ | $-CH=CH-$ | |
| 94 | $4-Br-C_6H_4$ | $-CH=CH-$ | |
| 95 | $2-CF_3-C_6H_4$ | $-CH=CH-$ | |
| 96 | $3-CF_3-C_6H_4$ | $-CH=CH-$ | |
| 97 | $4-CF_3-C_6H_4$ | $-CH=CH-$ | |
| 98 | $2-OCH_3-C_6H_4$ | $-CH=CH-$ | |
| 99 | $3-OCH_3-C_6H_4$ | $-CH=CH-$ | |
| 100 | $4-OCH_3-C_6H_4$ | $-CH=CH-$ | |

| Nr. | Z | Y | Daten |
|---|---|---|---|
| 101 | 3,4-$(OCH_3)_2$-$C_6H_3$ | -CH=CH- | |
| 102 | 3,4,5-$(OCH_3)_3$-$C_6H_2$ | -CH=CH- | |
| 103 | 4-t-$C_4H_9O$-$C_6H_4$ | -CH=CH- | |
| 104 | 2-Phenoxy-$C_6H_4$ | -CH=CH- | |
| 105 | 3-Phenoxy-$C_6H_4$ | -CH=CH- | |
| 106 | 4-Phenoxy-$C_6H_4$ | -CH=CH- | |
| 107 | $C_6H_5$-$CH_2$ | -CH=CH- | |
| 108 | 2-F-$C_6H_4$-$CH_2$ | -CH=CH- | |
| 109 | 3-F-$C_6H_4$-$CH_2$ | -CH=CH- | |
| 110 | 4-F-$C_6H_4$-$CH_2$ | -CH=CH- | |
| 111 | 2-Cl-$C_6H_4$-$CH_2$ | -CH=CH- | |
| 112 | 3-Cl-$C_6H_4$-$CH_2$ | -CH=CH- | |
| 113 | 4-Cl-$C_6H_4$-$CH_2$ | -CH=CH- | |
| 114 | 2-Cl,6F-$C_6H_3$-$CH_2$ | -CH=CH- | |
| 115 | 2,4-$Cl_2$-$C_6H_3$-$CH_2$ | -CH=CH- | |
| 116 | 2,6-$Cl_2$-$C_6H_3$-$CH_2$ | -CH=CH- | |
| 117 | 3,5-$Cl_2$-$C_6H_3$-$CH_2$ | -CH=CH- | |
| 118 | 2,4,6-$Cl_3$-$C_6H_2$-$CH_2$ | -CH=CH- | |
| 119 | 2-$CH_3$-$C_6H_4$-$CH_2$ | -CH=CH- | |
| 120 | 3-$CH_3$-$C_6H_4$-$CH_2$ | -CH=CH- | |
| 121 | 4-$CH_3$-$C_6H_4$-$CH_2$ | -CH=CH- | |
| 122 | 4-t-$C_4H_9$-$C_6H_4$-$CH_2$ | -CH=CH- | |
| 123 | 2,4-$(CH_3)_2$-$C_6H_3$-$CH_2$ | -CH=CH- | |
| 124 | 2,6-$(CH_3)_2$-$C_6H_3$-$CH_2$ | -CH=CH- | |
| 125 | 2,4,6-$(CH_3)_3$-$C_6H_2$-$CH_2$ | -CH=CH- | |

| Nr. | Z | Y | Daten |
|---|---|---|---|
| 126 | $2-OCH_3-C_6H_4-CH_2$ | $-CH=CH-$ | |
| 127 | $3-OCH_3-C_6H_4-CH_2$ | $-CH=CH-$ | |
| 128 | $4-OCH_3-C_6H_4-CH_2$ | $-CH=CH-$ | |
| 129 | $2-CF_3-C_6H_4-CH_2$ | $-CH=CH-$ | |
| 130 | $3-CF_3-C_6H_4-CH_2$ | $-CH=CH-$ | |
| 131 | $4-CF_3-C_6H_4-CH_2$ | $-CH=CH-$ | |
| 132 | $4-O-t-C_4H_9-C_6H_4-CH_2$ | $-CH=CH-$ | |
| 133 | $4-O-C_6H_5-C_6H_4-CH_2$ | $-CH=CH-$ | |
| 134 | $4-C_6H_5-C_6H_4-CH_2$ | $-CH=CH-$ | |
| 135 | $2-Br-C_6H_4-CH_2$ | $-CH=CH-$ | |
| 136 | $3-Br-C_6H_4-CH_2$ | $-CH=CH-$ | |
| 137 | $4-Br-C_6H_4-CH_2$ | $-CH=CH-$ | |
| 138 | $C_6H_5-CH(CH_3)$ | $-CH=CH-$ | |
| 139 | $C_6H_5-CH(C_2H_5)$ | $-CH=CH-$ | |
| 140 | $C_6H_5-CH(iso-C_3H_7)$ | $-CH=CH-$ | |
| 141 | $C_6H_5-CH(OH)$ | $-CH=CH-$ | |
| 142 | $2-OCH_3-C_6H_4-CH(OH)$ | $-CH=CH-$ | |
| 143 | $4-OCH_3-C_6H_4-CH(OH)$ | $-CH=CH-$ | |
| 144 | $3,4-(OCH_3)_2-C_6H_3-CH(OH)$ | $-CH=CH-$ | |
| 145 | $2-OCH_3-C_6H_4-CH(OCH_3)$ | $-CH=CH-$ | |
| 146 | $4-OCH_3-C_6H_4-CH(OCH_3)$ | $-CH=CH-$ | |
| 147 | $3,4-(OCH_3)_2-C_6H_3-CH(OCH_3)$ | $-CH=CH-$ | |
| 148 | $C_6H_5-CH_2CH_2$ | $-CH=CH-$ | |
| 149 | $C_6H_5-CH_2CH(CH_3)$ | $-CH=CH-$ | |
| 150 | $4-t-C_4H_9-C_6H_4-CH_2CH_2$ | $-CH=CH-$ | |

| Nr. | Z | Y | Daten |
|---|---|---|---|
| 151 | 4-t-$C_4H_9$-$C_6H_4$- $CH_2CH(CH_3)$ | -CH=CH- | |
| 152 | 2-Cl-$C_6H_4$-$CH_2CH_2$ | -CH=CH- | |
| 153 | 3-Cl-$C_6H_4$-$CH_2CH_2$ | -CH=CH- | |
| 154 | 4-Cl-$C_6H_4$-$CH_2CH_2$ | -CH=CH- | |
| 155 | $C_6H_5$-CH=CH | -CH=CH- | |
| 156 | 2-Cl-$C_6H_4$-CH=CH | -CH=CH- | |
| 157 | 3-Cl-$C_6H_4$-CH=CH | -CH=CH- | |
| 158 | 4-Cl-$C_6H_4$-CH=CH | -CH=CH- | |
| 159 | 2,4-$Cl_2$-$C_6H_3$-CH=CH | -CH=CH- | |
| 160 | 2-F-$C_6H_4$-CH=CH | -CH=CH- | |
| 161 | 4-F-$C_6H_4$-CH=CH | -CH=CH- | |
| 162 | 2-$CH_3$-$C_6H_4$-CH=CH | -CH=CH- | |
| 163 | 4-$CH_3$-$C_6H_4$-CH=CH | -CH=CH- | |
| 164 | 4-t-$C_4H_9$-$C_6H_4$-CH=CH | -CH=CH- | |
| 165 | 2-$OCH_3$-$C_6H_4$-CH=CH | -CH=CH- | |
| 166 | 4-$OCH_3$-$C_6H_4$-CH=CH | -CH=CH- | |
| 167 | 4-Phenoxy-$C_6H_4$-CH=CH | -CH=CH- | |
| 168 | $C_6H_5$-$(CH_2)_3$ | -CH=CH- | |
| 169 | $C_6H_5$-$CH_2CH(CH_3)CH_2$ | -CH=CH- | |
| 170 | $C_6H_5$-$(CH_2)_4$ | -CH=CH- | |
| 171 | $C_6H_5$-$(CH_2)_5$ | -CH=CH- | |
| 172 | $C_6H_5$-$(CH_2)_6$ | -CH=CH- | |
| 173 | 4-t-$C_4H_9$-$C_6H_4$-$CH_2CH(CH_3)CH_2$ | -CH=CH- | |

| Nr. | Z | Y | Daten |
|---|---|---|---|
| 174 | H | $-CH_2CH_2-$ | |
| 175 | $CH_3$ | $-CH_2CH_2-$ | |
| 176 | $C_2H_5$ | $-CH_2CH_2-$ | |
| 177 | $n-C_3H_7$ | $-CH_2CH_2-$ | |
| 178 | $iso-C_3H_7$ | $-CH_2CH_2-$ | |
| 179 | $n-C_4H_9$ | $-CH_2CH_2-$ | |
| 180 | $(CH_3)_2CHCH_2$ | $-CH_2CH_2-$ | |
| 181 | $CH_3CH_2CH(CH_3)$ | $-CH_2CH_2-$ | |
| 182 | $tert.C_4H_9$ | $-CH_2CH_2-$ | |
| 183 | $CH_3CH_2C(CH_3)_2$ | $-CH_2CH_2-$ | |
| 184 | $(CH_3)_3CCH_2$ | $-CH_2CH_2-$ | |
| 185 | $(CH_3)_2CHCH_2CH_2$ | $-CH_2CH_2-$ | |
| 186 | $CH_3CH_2CH_2CH(C_2H_5)$ | $-CH_2CH_2-$ | |
| 187 | $n-C_5H_{11}$ | $-CH_2CH_2-$ | |
| 188 | $n-C_6H_{13}$ | $-CH_2CH_2-$ | |
| 189 | $n-C_7H_{15}$ | $-CH_2CH_2-$ | |
| 190 | $n-C_8H_{17}$ | $-CH_2CH_2-$ | |
| 191 | $n-C_9H_{19}$ | $-CH_2CH_2-$ | |
| 192 | $n-C_{10}H_{21}$ | $-CH_2CH_2-$ | |
| 193 | $n-C_{15}H_{31}$ | $-CH_2CH_2-$ | |
| 194 | $n-C_{17}H_{35}$ | $-CH_2CH_2-$ | |
| 195 | $CH_2=CH$ | $-CH_2CH_2-$ | |
| 196 | $CH_2=C(CH_3)$ | $-CH_2CH_2-$ | |
| 197 | $CH_3CH=CH$ | $-CH_2CH_2-$ | |

| Nr. | Z | Y | Daten |
|---|---|---|---|
| 198 | $CH_3CH=C(CH_3)$ | $-CH_2CH_2-$ | |
| 199 | $(CH_3)_2C=CH$ | $-CH_2CH_2-$ | |
| 200 | $CH_2=CHCH_2$ | $-CH_2CH_2-$ | |
| 201 | $CH_3CH=CHCH_2$ | $-CH_2CH_2-$ | |
| 202 | $(CH_3)_2C=CHCH_2$ | $-CH_2CH_2-$ | |
| 203 | $CH_3CH=CH-CH=CH$ | $-CH_2CH_2-$ | |
| 204 | $(CH_3)_2C=CHCH_2CH_2C(CH_3)=CH$ | $-CH_2CH_2-$ | |
| 205 | $(CH_3)_2CHCH_2CH_2CH_2CH(CH_3)CH_2$ | $-CH_2CH_2-$ | |
| 206 | $(CH_3)_2C=CHCH_2CH_2CH(CH_3)CH_2$ | $-CH_2CH_2-$ | |
| 207 | $HC\equiv C$ | $-CH_2CH_2-$ | |
| 208 | $C_6H_5-C\equiv C$ | $-CH_2CH_2-$ | |
| 209 | $CH_3OCH_2$ | $-CH_2CH_2-$ | |
| 210 | $C_2H_5OCH_2$ | $-CH_2CH_2-$ | |
| 211 | $CH_3CH(OCH_3)$ | $-CH_2CH_2-$ | |
| 212 | $CN-CH_2$ | $-CH_2CH_2-$ | |
| 213 | Cyclopropyl | $-CH_2CH_2-$ | |
| 214 | Cyclobutyl | $-CH_2CH_2-$ | |
| 215 | Cyclopentyl | $-CH_2CH_2-$ | |
| 216 | Cyclohexyl | $-CH_2CH_2-$ | |
| 217 | 1-Methylcyclopropyl | $-CH_2CH_2-$ | |
| 218 | 2,2-Dichlor-1-Methyl-cyclopropyl | $-CH_2CH_2-$ | |
| 219 | 2,2-Dichlor-3,3-Dimethyl-cyclopropyl | $-CH_2CH_2-$ | |
| 220 | 2,2,3,3-Tetramethylcyclopropyl | $-CH_2CH_2-$ | |
| 221 | 2-(2'-Methyl-1'-propenyl-)-3,3-Dimethylcyclopropyl | $-CH_2CH_2-$ | |
| 222 | 2-(2',2'-Difluorvinyl)-3,3-Dimethylcyclopropyl | $-CH_2CH_2-$ | |

| Nr. | Z | Y | Daten |
|---|---|---|---|
| 223 | 2-(2',2'-Dichlorvinyl)-3,3-Dimethylcyclopropyl | $-CH_2CH_2-$ | |
| 224 | 2-(2',2'Dibromvinyl)-3,3-Dimethylcyclopropyl | $-CH_2CH_2-$ | |
| 225 | 2-Phenylcyclopropyl | $-CH_2CH_2-$ | |
| 226 | 2-(4'-Chlorphenyl)cyclopropyl | $-CH_2CH_2-$ | |
| 227 | 2,2-Dichlor-3-Phenylcyclopropyl | $-CH_2CH_2-$ | |
| 228 | 2-Carbomethoxy-cyclopropyl | $-CH_2CH_2-$ | |
| 229 | $ClCH_2$ | $-CH_2CH_2-$ | |
| 230 | $Cl_2CH$ | $-CH_2CH_2-$ | |
| 231 | $Cl_3C$ | $-CH_2CH_2-$ | |
| 232 | $BrCH_2$ | $-CH_2CH_2-$ | |
| 233 | $CF_3$ | $-CH_2CH_2-$ | |
| 234 | $CH_3CH(Cl)$ | $-CH_2CH_2-$ | |
| 235 | $C_6H_5$ (=Phenyl) | $-CH_2CH_2-$ | |
| 236 | $2-CH_3-C_6H_4$ | $-CH_2CH_2-$ | |
| 237 | $3-CH_3-C_6H_4$ | $-CH_2CH_2-$ | |
| 238 | $4-CH_3-C_6H_4$ | $-CH_2CH_2-$ | |
| 239 | $2,3-(CH_3)_2-C_6H_3$ | $-CH_2CH_2-$ | |
| 240 | $2,4-(CH_3)_2-C_6H_3$ | $-CH_2CH_2-$ | |
| 241 | $2,6-(CH_3)_2-C_6H_3$ | $-CH_2CH_2-$ | |
| 242 | $3,4-(CH_3)_2-C_6H_3$ | $-CH_2CH_2-$ | |
| 243 | $3,5-(CH_3)_2-C_6H_3$ | $-CH_2CH_2-$ | |
| 244 | $2,4,6-(CH_3)_2-C_6H_2$ | $-CH_2CH_2-$ | |
| 245 | $4-t-C_4H_9-C_6H_4$ | $-CH_2CH_2-$ | |
| 246 | $2-C_6H_5-C_6H_4$ | $-CH_2CH_2-$ | |
| 247 | $4-C_6H_5-C_6H_4$ | $-CH_2CH_2-$ | |

| Nr. | Z | Y | Daten |
|---|---|---|---|
| 248 | 2-Cl-$C_6H_4$ | $-CH_2CH_2-$ | |
| 249 | 3-Cl-$C_6H_4$ | $-CH_2CH_2-$ | |
| 250 | 4-Cl-$C_6H_4$ | $-CH_2CH_2-$ | |
| 251 | 2,4-$Cl_2$-$C_6H_3$ | $-CH_2CH_2-$ | |
| 252 | 2,5-$Cl_2$-$C_6H_3$ | $-CH_2CH_2-$ | |
| 253 | 2,6-$Cl_2$-$C_6H_3$ | $-CH_2CH_2-$ | |
| 254 | 3,4-$Cl_2$-$C_6H_3$ | $-CH_2CH_2-$ | |
| 255 | 3,5-$Cl_2$-$C_6H_3$ | $-CH_2CH_2-$ | |
| 256 | 2,4,5-$Cl_3$-$C_6H_2$ | $-CH_2CH_2-$ | |
| 257 | 2,3,4,5,6-$Cl_5$-$C_6$ | $-CH_2CH_2-$ | |
| 258 | 6-F,2-Cl-$C_6H_3$ | $-CH_2CH_2-$ | |
| 260 | 2-F-$C_6H_4$ | $-CH_2CH_2-$ | |
| 261 | 3-F-$C_6H_4$ | $-CH_2CH_2-$ | |
| 262 | 4-F-$C_6H_4$ | $-CH_2CH_2-$ | |
| 263 | 2,4-$F_2$-$C_6H_3$ | $-CH_2CH_2-$ | |
| 264 | 2,6-$F_2$-$C_6H_3$ | $-CH_2CH_2-$ | |
| 265 | 2,3,4,5,6-$F_5$-$C_6$ | $-CH_2CH_2-$ | |
| 266 | 2-Br-$C_6H_4$ | $-CH_2CH_2-$ | |
| 267 | 3-Br-$C_6H_4$ | $-CH_2CH_2-$ | |
| 268 | 4-Br-$C_6H_4$ | $-CH_2CH_2-$ | |
| 269 | 2-$CF_3$-$C_6H_4$ | $-CH_2CH_2-$ | |
| 270 | 3-$CF_3$-$C_6H_4$ | $-CH_2CH_2-$ | |
| 271 | 4-$CF_3$-$C_6H_4$ | $-CH_2CH_2-$ | |
| 272 | 2-$OCH_3$-$C_6H_4$ | $-CH_2CH_2-$ | |
| 273 | 3-$OCH_3$-$C_6H_4$ | $-CH_2CH_2-$ | |

| Nr. | Z | Y | Daten |
|---|---|---|---|
| 277 | 4-t-$C_4H_9O$-$C_6H_4$ | -$CH_2CH_2$- | |
| 278 | 2-Phenoxy-$C_6H_4$ | -$CH_2CH_2$- | |
| 279 | 3-Phenoxy-$C_6H_4$ | -$CH_2CH_2$- | |
| 280 | 4-Phenoxy-$C_6H_4$ | -$CH_2CH_2$- | |
| 281 | $C_6H_5$-$CH_2$ | -$CH_2CH_2$- | |
| 282 | 2-F-$C_6H_4$-$CH_2$ | -$CH_2CH_2$- | |
| 283 | 3-F-$C_6H_4$-$CH_2$ | -$CH_2CH_2$- | |
| 284 | 4-F-$C_6H_4$-$CH_2$ | -$CH_2CH_2$- | |
| 285 | 2-Cl-$C_6H_4$-$CH_2$ | -$CH_2CH_2$- | |
| 286 | 3-Cl-$C_6H_4$-$CH_2$ | -$CH_2CH_2$- | |
| 287 | 4-Cl-$C_6H_4$-$CH_2$ | -$CH_2CH_2$- | |
| 288 | 2-Cl,6F-$C_6H_3$-$CH_2$ | -$CH_2CH_2$- | |
| 289 | 2,4-$Cl_2$-$C_6H_3$-$CH_2$ | -$CH_2CH_2$- | |
| 290 | 2,6-$Cl_2$-$C_6H_3$-$CH_2$ | -$CH_2CH_2$- | |
| 291 | 3,5-$Cl_2$-$C_6H_3$-$CH_2$ | -$CH_2CH_2$- | |
| 292 | 2,4,6-$Cl_3$-$C_6H_2$-$CH_2$ | -$CH_2CH_2$- | |
| 293 | 2-$CH_3$-$C_6H_4$-$CH_2$ | -$CH_2CH_2$- | |
| 294 | 3-$CH_3$-$C_6H_4$-$CH_2$ | -$CH_2CH_2$- | |
| 295 | 4-$CH_3$-$C_6H_4$-$CH_2$ | -$CH_2CH_2$- | |
| 296 | 4-t-$C_4H_9$-$C_6H_4$-$CH_2$ | -$CH_2CH_2$- | |
| 297 | 2,4-$(CH_3)_2$-$C_6H_3$-$CH_2$ | -$CH_2CH_2$- | |
| 298 | 2,6-$(CH_3)_2$-$C_6H_3$-$CH_2$ | -$CH_2CH_2$- | |
| 299 | 2,4,6-$(CH_3)_3$-$C_6H_2$-$CH_2$ | -$CH_2CH_2$- | |
| 300 | 2-$OCH_3$-$C_6H_4$-$CH_2$ | -$CH_2CH_2$- | |
| 301 | 3-$OCH_3$-$C_6H_4$-$CH_2$ | -$CH_2CH_2$- | |
| 302 | 4-$OCH_3$-$C_6H_4$-$CH_2$ | -$CH_2CH_2$- | |

| Nr. | Z | Y | Daten |
|---|---|---|---|
| 302 | $2-CF_3-C_6H_4-CH_2$ | $-CH_2CH_2-$ | |
| 303 | $3-CF_3-C_6H_4-CH_2$ | $-CH_2CH_2-$ | |
| 304 | $4-CF_3-C_6H_4-CH_2$ | $-CH_2CH_2-$ | |
| 305 | $4-O-t-C_4H_9-C_6H_4-CH_2$ | $-CH_2CH_2-$ | |
| 306 | $4-O-C_6H_5-C_6H_4-CH_2$ | $-CH_2CH_2-$ | |
| 307 | $4-C_6H_5-C_6H_4-CH_2$ | $-CH_2CH_2-$ | |
| 308 | $2-Br-C_6H_4-CH_2$ | $-CH_2CH_2-$ | |
| 309 | $3-Br-C_6H_4-CH_2$ | $-CH_2CH_2-$ | |
| 310 | $4-Br-C_6H_4-CH_2$ | $-CH_2CH_2-$ | |
| 311 | $C_6H_5-CH(CH_3)$ | $-CH_2CH_2-$ | |
| 312 | $C_6H_5-CH(C_2H_5)$ | $-CH_2CH_2-$ | |
| 313 | $C_6H_5-CH(iso-C_3H_7)$ | $-CH_2CH_2-$ | |
| 314 | $C_6H_5-CH(OH)$ | $-CH_2CH_2-$ | |
| 315 | $2-OCH_3-C_6H_4-CH(OH)$ | $-CH_2CH_2-$ | |
| 316 | $4-OCH_3-C_6H_4-CH(OH)$ | $-CH_2CH_2-$ | |
| 317 | $3,4-(OCH_3)_2-C_6H_3-CH(OH)$ | $-CH_2CH_2-$ | |
| 318 | $2-OCH_3-C_6H_4-CH(OCH_3)$ | $-CH_2CH_2-$ | |
| 319 | $4-OCH_3-C_6H_4-CH(OCH_3)$ | $-CH_2CH_2-$ | |
| 320 | $3,4-(OCH_3)_2-C_6H_3-CH(OCH_3)$ | $-CH_2CH_2-$ | |
| 321 | $C_6H_5-CH_2CH_2$ | $-CH_2CH_2-$ | |
| 322 | $C_6H_5-CH_2CH(CH_3)$ | $-CH_2CH_2-$ | |
| 323 | $4-t-C_4H_9-C_6H_4-CH_2CH_2$ | $-CH_2CH_2-$ | |
| 324 | $4-t-C_4H_9-C_6H_4-\ CH_2CH(CH_3)$ | $-CH_2CH_2-$ | |
| 325 | $2-Cl-C_6H_4-CH_2CH_2$ | $-CH_2CH_2-$ | |
| 326 | $3-Cl-C_6H_4-CH_2CH_2$ | $-CH_2CH_2-$ | |

| Nr. | Z | Y | Daten |
|---|---|---|---|
| 327 | $4-Cl-C_6H_4-CH_2CH_2$ | $-CH_2CH_2-$ | |
| 328 | $C_6H_5-CH=CH$ | $-CH_2CH_2-$ | |
| 329 | $2-Cl-C_6H_4-CH=CH$ | $-CH_2CH_2-$ | |
| 330 | $3-Cl-C_6H_4-CH=CH$ | $-CH_2CH_2-$ | |
| 331 | $4-Cl-C_6H_4-CH=CH$ | $-CH_2CH_2-$ | |
| 332 | $2,4-Cl_2-C_6H_3-CH=CH$ | $-CH_2CH_2-$ | |
| 333 | $2-F-C_6H_4-CH=CH$ | $-CH_2CH_2-$ | |
| 334 | $4-F-C_6H_4-CH=CH$ | $-CH_2CH_2$ | |
| 335 | $2-CH_3-C_6H_4-CH=CH$ | $-CH_2CH_2-$ | |
| 336 | $4-CH_3-C_6H_4-CH=CH$ | $-CH_2CH_2-$ | |
| 337 | $4-t-C_4H_9-C_6H_4-CH=CH$ | $-CH_2CH_2-$ | |
| 338 | $2-OCH_3-C_6H_4-CH=CH$ | $-CH_2CH_2-$ | |
| 339 | $4-OCH_3-C_6H_4-CH=CH$ | $-CH_2CH_2-$ | |
| 340 | 4-Phenoxy-$C_6H_4$-CH=CH | $-CH_2CH_2-$ | |
| 341 | $C_6H_5-(CH_2)_3$ | $-CH_2CH_2-$ | |
| 342 | $C_6H_5-CH_2CH(CH_3)CH_2$ | $-CH_2CH_2-$ | |
| 343 | $C_6H_5-(CH_2)_4$ | $-CH_2CH_2-$ | |
| 344 | $C_6H_5-(CH_2)_5$ | $-CH_2CH_2-$ | |
| 345 | $C_6H_5-(CH_2)_6$ | $-CH_2CH_2-$ | |
| 346 | $4-t-C_4H_9-C_6H_4-CH_2CH(CH_3)CH_2$ | $-CH_2CH_2-$ | |
| 347 | $C_6H_5O$ | $-CH_2CH_2-$ | |
| 348 | $2-Cl-C_6H_4O$ | $-CH_2CH_2-$ | |
| 349 | $3-Cl-C_6H_4O$ | $-CH_2CH_2-$ | |
| 350 | $4-Cl-C_6H_4O$ | $-CH_2CH_2-$ | |
| 351 | $2-CH_3-C_6H_4O$ | $-CH_2CH_2-$ | |

| Nr. | Z | Y | Daten |
|---|---|---|---|
| 352 | $4-CH_3-C_6H_4O$ | $-CH_2CH_2-$ | |
| 353 | $2-OCH_3-C_6H_4O$ | $-CH_2CH_2-$ | |
| 354 | $4-OCH_3-C_6H_4O$ | $-CH_2CH_2-$ | |
| 355 | $2-CF_3-C_6H_4O$ | $-CH_2CH_2-$ | |
| 356 | $4-CF_3-C_6H_4O$ | $-CH_2CH_2-$ | |
| 357 | $C_6H_5OCH_2$ | $-CH_2CH_2-$ | |
| 358 | $2-Cl-C_6H_4OCH_2$ | $-CH_2CH_2-$ | |
| 359 | $4-Cl-C_6H_4OCH_2$ | $-CH_2CH_2-$ | |
| 360 | $2-CH_3-C_6H_4OCH_2$ | $-CH_2CH_2-$ | |
| 361 | $4-CH_3-C_6H_4OCH_2$ | $-CH_2CH_2-$ | |
| 362 | $2-OCH_3-C_6H_4OCH_2$ | $-CH_2CH_2-$ | |
| 363 | $4-OCH_3-C_6H_4OCH_2$ | $-CH_2CH_2-$ | |
| 364 | $4-t-C_4H_9-C_6H_4OCH_2$ | $-CH_2CH_2-$ | |
| 365 | $C_6H_5OCH_2CH_2$ | $-CH_2CH_2-$ | |
| 366 | $2-Cl-C_6H_4OCH_2CH_2$ | $-CH_2CH_2-$ | |
| 367 | $4-Cl-C_6H_4OCH_2CH_2$ | $-CH_2CH_2-$ | |
| 368 | $4-F-C_6H_4OCH_2CH_2$ | $-CH_2CH_2-$ | |
| 369 | $2-CH_3-C_6H_4OCH_2CH_2$ | $-CH_2CH_2-$ | |
| 370 | $4-CH_3-C_6H_4OCH_2CH_2$ | $-CH_2CH_2-$ | |
| 371 | $2-OCH_3-C_6H_4OCH_2CH_2$ | $-CH_2CH_2-$ | |
| 372 | $4-OCH_3-C_6H_4OCH_2CH_2$ | $-CH_2CH_2-$ | |
| 373 | $4-t-C_4H_9-C_6H_4-OCH_2CH_2$ | $-CH_2CH_2-$ | |
| 374 | $C_6H_5O(CH_2)_3$ | $-CH_2CH_2-$ | |
| 375 | $2-Cl-C_6H_4O(CH_2)_3$ | $-CH_2CH_2-$ | |
| 376 | $4-Cl-C_6H_4O(CH_2)_3$ | $-CH_2CH_2-$ | |

| Nr. | Z | Y | Daten |
|---|---|---|---|
| 377 | $C_6H_5O(CH_2)_4$ | $-CH_2CH_2-$ | |
| 378 | $2-Cl-C_6H_4-O(CH_2)_4$ | $-CH_2CH_2-$ | |
| 379 | $4-Cl-C_6H_4-O(CH_2)_4$ | $-CH_2CH_2-$ | |
| 380 | $C_6H_5O(CH_2)_5$ | $-CH_2CH_2-$ | |
| 381 | $2-Cl-C_6H_4O(CH_2)_5$ | $-CH_2CH_2-$ | |
| 382 | $4-Cl-C_6H_4O(CH_2)_5$ | $-CH_2CH_2-$ | |
| 383 | $C_6H_5O(CH_2)_6$ | $-CH_2CH_2-$ | |
| 384 | $C_6H_5OCH_2CH_2O$ | $-CH_2CH_2-$ | |
| ~~385~~ | ~~H~~ | ~~$-CH_2O-$~~ | |
| 386 | $CH_3$ | $-CH_2O-$ | |
| 387 | $C_2H_5$ | $-CH_2O-$ | |
| 388 | $n-C_3H_7$ | $-CH_2O-$ | |
| 389 | $iso-C_3H_7$ | $-CH_2O-$ | |
| 390 | $n-C_4H_9$ | $-CH_2O-$ | |
| 391 | $(CH_3)_2CHCH_2$ | $-CH_2O-$ | |
| 392 | $CH_3CH_2CH(CH_3)$ | $-CH_2O-$ | |
| 393 | $tert.C_4H_9$ | $-CH_2O-$ | |
| 394 | $CH_3CH_2C(CH_3)_2$ | $-CH_2O-$ | |
| 395 | $(CH_3)_3CCH_2$ | $-CH_2O-$ | |
| 396 | $(CH_3)_2CHCH_2CH_2$ | $-CH_2O-$ | |
| 397 | $CH_3CH_2CH_2CH(C_2H_5)$ | $-CH_2O-$ | |
| 398 | $n-C_5H_{11}$ | $-CH_2O-$ | |
| 399 | $n-C_6H_{13}$ | $-CH_2O-$ | |
| 400 | $n-C_7H_{15}$ | $-CH_2O-$ | |
| 401 | $n-C_8H_{17}$ | $-CH_2O-$ | |

| Nr. | Z | Y | Daten |
|---|---|---|---|
| 402 | n-$C_9H_{19}$ | $-CH_2O-$ | |
| 403 | n-$C_{10}H_{21}$ | $-CH_2O-$ | |
| 404 | n-$C_{15}H_{31}$ | $-CH_2O-$ | |
| 405 | n-$C_{17}H_{35}$ | $-CH_2O-$ | |
| 406 | $CH_2=CH$ | $-CH_2O-$ | |
| 407 | $CH_2=C(CH_3)$ | $-CH_2O-$ | |
| 408 | $CH_3CH=CH$ | $-CH_2O-$ | |
| 409 | $CH_3CH=C(CH_3)$ | $-CH_2O-$ | |
| 410 | $(CH_3)_2C=CH$ | $-CH_2O-$ | |
| 411 | $CH_2=CHCH_2$ | $-CH_2O-$ | |
| 412 | $CH_3CH=CHCH_2$ | $-CH_2O-$ | |
| 413 | $(CH_3)_2C=CHCH_2$ | $-CH_2O-$ | |
| 414 | $CH_3CH=CH-CH=CH$ | $-CH_2O-$ | |
| 415 | $(CH_3)_2C=CHCH_2CH_2C(CH_3)=CH$ | $-CH_2O-$ | |
| 416 | $(CH_3)_2CHCH_2CH_2CH_2CH(CH_3)CH_2$ | $-CH_2O-$ | |
| 417 | $(CH_3)_2C=CHCH_2CH_2CH(CH_3)CH_2$ | $-CH_2O-$ | |
| 418 | $HC\equiv C$ | $-CH_2O-$ | |
| 419 | $C_6H_5-C\equiv C$ | $-CH_2O-$ | |
| 420 | $CH_3OCH_2$ | $-CH_2O-$ | |
| 421 | $C_2H_5OCH_2$ | $-CH_2O-$ | |
| 422 | $CH_3CH(OCH_3)$ | $-CH_2O-$ | |
| 423 | $CN-CH_2$ | $-CH_2O-$ | |
| 424 | Cyclopropyl | $-CH_2O-$ | |
| 425 | Cyclobutyl | $-CH_2O-$ | |
| 426 | Cyclopentyl | $-CH_2O-$ | |

| Nr. | Z | Y | Daten |
|---|---|---|---|
| 427 | Cyclohexyl | $-CH_2O-$ | |
| 428 | 1-Methylcyclopropyl | $-CH_2O-$ | |
| 429 | 2,2-Dichlor-1-Methyl-cyclopropyl | $-CH_2O-$ | |
| 430 | 2,2-Dichlor-3,3-Dimethyl-cyclopropyl | $-CH_2O-$ | |
| 431 | 2,2,3,3-Tetramethylcyclopropyl | $-CH_2O-$ | |
| 432 | 2-(2'-Methyl-1'-propenyl-)-3,3-Dimethylcyclopropyl | $-CH_2O-$ | |
| 433 | 2-(2',2'-Difluorvinyl)-3,3-Dimethylcyclopropyl | $-CH_2O-$ | |
| 434 | 2-(2',2'-Dichlorvinyl)-3,3-Dimethylcyclopropyl | $-CH_2O-$ | |
| 435 | 2-(2',2'Dibromvinyl)-3,3-Dimethylcyclopropyl | $-CH_2O-$ | |
| 436 | 2-Phenylcyclopropyl | $-CH_2O-$ | |
| 437 | 2-(4'-Chlorphenyl)cyclopropyl | $-CH_2O-$ | |
| 438 | 2,2-Dichlor-3-Phenylcyclopropyl | $-CH_2O-$ | |
| 439 | 2-Carbomethoxy-cyclopropyl | $-CH_2O-$ | |
| 440 | $Cl_2CH$ | $-CH_2O-$ | |
| 441 | $Cl_2CH$ | $-CH_2O-$ | |
| 442 | $Cl_3C$ | $-CH_2O-$ | |
| 443 | $BrCH_2$ | $-CH_2O-$ | |
| 444 | $CF_3$ | $-CH_2O-$ | |
| 445 | $CH_3CH(Cl)$ | $-CH_2O-$ | |
| 446 | $C_6H_5$ (=Phenyl) | $-CH_2O-$ | Öl; IR (Film): 1727, 1602, 1495, 1451, 1276, 1242, 1208, 753, 735, 696 $cm^{-1}$ |
| 447 | $2-CH_3-C_6H_4$ | $-CH_2O-$ | |
| 448 | $3-CH_3-C_6H_4$ | $-CH_2O-$ | Öl; IR (Film): 1738, 1602, 1495, 1242, 1160 1018, 753 $cm^{-1}$ |
| 449 | $4-CH_3-C_6H_4$ | $-CH_2O-$ | |

| Nr. | Z | Y | Daten |
|---|---|---|---|
| 450 | $2,3-(CH_3)_2-C_6H_3$ | $-CH_2O-$ | |
| 451 | $2,4-(CH_3)_2-C_6H_3$ | $-CH_2O-$ | |
| 452 | $2,6-(CH_3)_2-C_6H_3$ | $-CH_2O-$ | |
| 453 | $3,4-(CH_3)_2-C_6H_3$ | $-CH_2O-$ | |
| 454 | $3,5-(CH_3)_2-C_6H_3$ | $-CH_2O-$ | |
| 455 | $2,4,6-(CH_3)_2-C_6H_2$ | $-CH_2O-$ | |
| 456 | $4-t-C_4H_9-C_6H_4$ | $-CH_2O-$ | |
| 457 | $2-C_6H_5-C_6H_4$ | $-CH_2O-$ | |
| 458 | $4-C_6H_5-C_6H_4$ | $-CH_2O-$ | |
| 459 | $2-Cl-C_6H_4$ | $-CH_2O-$ | |
| 460 | $3-Cl-C_6H_4$ | $-CH_2O-$ | Öl; IR (Film): 1726, 1491, 1451, 1210, 753 $cm^{-1}$ |
| 461 | $4-Cl-C_6H_4$ | $-CH_2O-$ | |
| 462 | $2,4-Cl_2-C_6H_3$ | $-CH_2O-$ | |
| 463 | $2,5-Cl_2-C_6H_3$ | $-CH_2O-$ | |
| 464 | $2,6-Cl_2-C_6H_3$ | $-CH_2O-$ | |
| 465 | $3,4-Cl_2-C_6H_3$ | $-CH_2O-$ | |
| 466 | $3,5-Cl_2-C_6H_3$ | $-CH_2O-$ | |
| 467 | $2,4,5-Cl_3-C_6H_2$ | $-CH_2O-$ | |
| 468 | $2,3,4,5,6-Cl_5-C_6$ | $-CH_2O-$ | |
| 469 | $6-F,2-Cl-C_6H_3$ | $-CH_2O-$ | |
| 470 | $2-F-C_6H_4$ | $-CH_2O-$ | |
| 471 | $3-F-C_6H_4$ | $-CH_2O-$ | |
| 472 | $4-F-C_6H_4$ | $-CH_2O-$ | |
| 473 | $2,4-F_2-C_6H_3$ | $-CH_2O-$ | |
| 474 | $2,6-F_2-C_6H_3$ | $-CH_2O-$ | |

| Nr. | Z | Y | Daten |
|---|---|---|---|
| 475 | $2,3,4,5,6-F_5-C_6$ | $-CH_2O-$ | |
| 476 | $2-Br-C_6H_4$ | $-CH_2O-$ | |
| 477 | $3-Br-C_6H_4$ | $-CH_2O-$ | |
| 478 | $4-Br-C_6H_4$ | $-CH_2O-$ | |
| 479 | $2-CF_3-C_6H_4$ | $-CH_2O-$ | |
| 480 | $3-CF_3-C_6H_4$ | $-CH_2O-$ | |
| 481 | $4-CF_3-C_6H_4$ | $-CH_2O-$ | |
| 482 | $2-OCH_3-C_6H_4$ | $-CH_2O-$ | |
| 483 | $3-OCH_3-C_6H_4$ | $-CH_2O-$ | |
| 484 | $4-OCH_3-C_6H_4$ | $-CH_2O-$ | |
| 485 | $3,4-(OCH_3)_2-C_6H_3$ | $-CH_2O-$ | |
| 486 | $3,4,5-(OCH_3)_3-C_6H_2$ | $-CH_2O-$ | |
| 487 | $4-t-C_4H_9O-C_6H_4$ | $-CH_2O-$ | |
| 488 | 2-Phenoxy-$C_6H_4$ | $-CH_2O-$ | |
| 489 | 3-Phenoxy-$C_6H_4$ | $-CH_2O-$ | |
| 490 | 4-Phenoxy-$C_6H_4$ | $-CH_2O-$ | |
| 491 | $C_6H_5-CH_2$ | $-CH_2O-$ | |
| 492 | $2-F-C_6H_4-CH_2$ | $-CH_2O-$ | |
| 493 | $3-F-C_6H_4-CH_2$ | $-CH_2O-$ | |
| 494 | $4-F-C_6H_4-CH_2$ | $-CH_2O-$ | |
| 495 | $2-Cl-C_6H_4-CH_2$ | $-CH_2O-$ | |
| 496 | $3-Cl-C_6H_4-CH_2$ | $-CH_2O-$ | |
| 497 | $4-Cl-C_6H_4-CH_2$ | $-CH_2O-$ | |
| 498 | $2-Cl,6F-C_6H_3-CH_2$ | $-CH_2O-$ | |
| 499 | $2,4-Cl_2-C_6H_3-CH_2$ | $-CH_2O-$ | |

| Nr. | Z | Y | Daten |
|---|---|---|---|
| 500 | $2,6-Cl_2-C_6H_3-CH_2$ | $-CH_2O-$ | |
| 501 | $3,5-Cl_2-C_6H_3-CH_2$ | $-CH_2O-$ | |
| 502 | $2,4,6-Cl_3-C_6H_2-CH_2$ | $-CH_2O-$ | |
| 503 | $2-CH_3-C_6H_4-CH_2$ | $-CH_2O-$ | |
| 504 | $3-CH_3-C_6H_4-CH_2$ | $-CH_2O-$ | |
| 505 | $4-CH_3-C_6H_4-CH_2$ | $-CH_2O-$ | |
| 506 | $4-t-C_4H_9-C_6H_4-CH_2$ | $-CH_2O-$ | |
| 507 | $2,4-(CH_3)_2-C_6H_3-CH_2$ | $-CH_2O-$ | |
| 508 | $2,6-(CH_3)_2-C_6H_3-CH_2$ | $-CH_2O-$ | |
| 509 | $2,4,6-(CH_3)_3-C_6H_2-CH_2$ | $-CH_2O-$ | |
| 510 | $2-OCH_3-C_6H_4-CH_2$ | $-CH_2O-$ | |
| 511 | $3-OCH_3-C_6H_4-CH_2$ | $-CH_2O-$ | |
| 512 | $4-OCH_3-C_6H_4-CH_2$ | $-CH_2O-$ | |
| 513 | $2-CF_3-C_6H_4-CH_2$ | $-CH_2O-$ | |
| 514 | $3-CF_3-C_6H_4-CH_2$ | $-CH_2O-$ | |
| 515 | $4-CF_3-C_6H_4-CH_2$ | $-CH_2O-$ | |
| 516 | $4-O-t-C_4H_9-C_6H_4-CH_2$ | $-CH_2O-$ | |
| 517 | $4-O-C_6H_5-C_6H_4-CH_2$ | $-CH_2O-$ | |
| 518 | $4-C_6H_5-C_6H_4-CH_2$ | $-CH_2O-$ | |
| 519 | $2-Br-C_6H_4-CH_2$ | $-CH_2O-$ | |
| 520 | $3-Br-C_6H_4-CH_2$ | $-CH_2O-$ | |
| 521 | $4-Br-C_6H_4-CH_2$ | $-CH_2O-$ | |
| 522 | $C_6H_5-CH(CH_3)$ | $-CH_2O-$ | |
| 523 | $C_6H_5-CH(C_2H_5)$ | $-CH_2O-$ | |
| 524 | $C_6H_5-CH(iso-C_3H_7)$ | $-CH_2O-$ | |

| Nr. | Z | Y | Daten |
|---|---|---|---|
| 525 | $C_6H_5-CH(OH)$ | $-CH_2O-$ | |
| 526 | $2-OCH_3-C_6H_4-CH(OH)$ | $-CH_2O-$ | |
| 527 | $4-OCH_3-C_6H_4-CH(OH)$ | $-CH_2O-$ | |
| 528 | $3,4-(OCH_3)_2-C_6H_3-CH(OH)$ | $-CH_2O-$ | |
| 529 | $2-OCH_3-C_6H_4-CH(OCH_3)$ | $-CH_2O-$ | |
| 530 | $4-OCH_3-C_6H_4-CH(OCH_3)$ | $-CH_2O-$ | |
| 531 | $3,4-(OCH_3)_2-C_6H_3-CH(OCH_3)$ | $-CH_2O-$ | |
| 532 | $C_6H_5-CH_2CH_2$ | $-CH_2O-$ | |
| 533 | $C_6H_5-CH_2CH(CH_3)$ | $-CH_2O-$ | |
| 534 | $4-t-C_4H_9-C_6H_4-CH_2CH_2$ | $-CH_2O-$ | |
| 535 | $4-t-C_4H_9-C_6H_4-$ $CH_2CH(CH_3)$ | $-CH_2O-$ | |
| 536 | $2-Cl-C_6H_4-CH_2CH_2$ | $-CH_2O-$ | |
| 537 | $3-Cl-C_6H_4-CH_2CH_2$ | $-CH_2O-$ | |
| 538 | $4-Cl-C_6H_4-CH_2CH_2$ | $-CH_2O-$ | |
| 539 | $C_6H_5-CH=CH$ | $-CH_2O-$ | |
| 540 | $2-Cl-C_6H_4-CH=CH$ | $-CH_2O-$ | |
| 541 | $3-Cl-C_6H_4-CH=CH$ | $-CH_2O-$ | |
| 542 | $4-Cl-C_6H_4-CH=CH$ | $-CH_2O-$ | |
| 543 | $2,4-Cl_2-C_6H_3-CH=CH$ | $-CH_2O-$ | |
| 545 | $2-F-C_6H_4-CH=CH$ | $-CH_2O-$ | |
| 546 | $4-F-C_6H_4-CH=CH$ | $-CH_2O$ | |
| 547 | $2-CH_3-C_6H_4-CH=CH$ | $-CH_2O-$ | |
| 548 | $4-CH_3-C_6H_4-CH=CH$ | $-CH_2O-$ | |
| 549 | $4-t-C_4H_9-C_6H_4-CH=CH$ | $-CH_2O-$ | |
| 550 | $2-OCH_3-C_6H_4-CH=CH$ | $-CH_2O-$ | |

| Nr. | Z | Y | Daten |
|---|---|---|---|
| 551 | $4\text{-}OCH_3\text{-}C_6H_4\text{-}CH{=}CH$ | $-CH_2O-$ | |
| 552 | 4-Phenoxy-$C_6H_4\text{-}CH{=}CH$ | $-CH_2O-$ | |
| 553 | $C_6H_5\text{-}(CH_2)_3$ | $-CH_2O-$ | |
| 554 | $C_6H_5\text{-}CH_2CH(CH_3)CH_2$ | $-CH_2O-$ | |
| 555 | $C_6H_5\text{-}(CH_2)_4$ | $-CH_2O-$ | |
| 556 | $C_6H_5\text{-}(CH_2)_5$ | $-CH_2O-$ | |
| 557 | $C_6H_5\text{-}(CH_2)_6$ | $-CH_2O-$ | |
| 558 | $4\text{-}t\text{-}C_4H_9\text{-}C_6H_4\text{-}CH_2CH(CH_3)CH_2$ | $-CH_2O-$ | |
| 559 | H | $-OCH_2-$ | |
| 560 | $CH_3$ | $-OCH_2-$ | |
| 561 | $C_2H_5$ | $-OCH_2-$ | |
| 562 | n-$C_3H_7$ | $-OCH_2-$ | |
| 563 | iso-$C_3H_7$ | $-OCH_2-$ | |
| 564 | n-$C_4H_9$ | $-OCH_2-$ | |
| 565 | $(CH_3)_2CHCH_2$ | $-OCH_2-$ | |
| 566 | $CH_3CH_2CH(CH_3)$ | $-OCH_2-$ | |
| 567 | tert.$C_4H_9$ | $-OCH_2-$ | |
| 568 | $CH_3CH_2C(CH_3)_2$ | $-OCH_2-$ | |
| 569 | $(CH_3)_3CCH_2$ | $-OCH_2-$ | |
| 570 | $(CH_3)_2CHCH_2CH_2$ | $-OCH_2-$ | |
| 571 | $CH_3CH_2CH_2CH(C_2H_5)$ | $-OCH_2-$ | |
| 572 | n-$C_5H_{11}$ | $-OCH_2-$ | |
| 573 | n-$C_6H_{13}$ | $-OCH_2-$ | |
| 574 | n-$C_7H_{15}$ | $-OCH_2-$ | |
| 575 | n-$C_8H_{17}$ | $-OCH_2-$ | |

| Nr. | Z | Y | Daten |
|---|---|---|---|
| 576 | n-$C_9H_{19}$ | $-OCH_2-$ | |
| 577 | n-$C_{10}H_{21}$ | $-OCH_2-$ | |
| 578 | n-$C_{15}H_{31}$ | $-OCH_2-$ | |
| 579 | n-$C_{17}H_{35}$ | $-OCH_2-$ | |
| 580 | $CH_2=CH$ | $-OCH_2-$ | |
| 581 | $CH_2=C(CH_3)$ | $-OCH_2-$ | |
| 582 | $CH_3CH=CH$ | $-OCH_2-$ | |
| 583 | $CH_3CH=C(CH_3)$ | $-OCH_2-$ | |
| 584 | $(CH_3)_2C=CH$ | $-OCH_2-$ | |
| 585 | $CH_2=CHCH_2$ | $-OCH_2-$ | |
| 586 | $CH_3CH=CHCH_2$ | $-OCH_2-$ | |
| 587 | $(CH_3)_2C=CHCH_2$ | $-OCH_2-$ | |
| 588 | $CH_3CH=CH-CH=CH$ | $-OCH_2-$ | |
| 589 | $(CH_3)_2C=CHCH_2CH_2C(CH_3)=CH$ | $-OCH_2-$ | |
| 590 | $(CH_3)_2CHCH_2CH_2CH_2CH(CH_3)CH_2$ | $-OCH_2-$ | |
| 591 | $(CH_3)_2C=CHCH_2CH_2CH(CH_3)CH_2$ | $-OCH_2-$ | |
| 592 | $(CH_3)_2C=CHCH_2CH_2CH(CH_3)CH_2$ | $-OCH_2-$ | |
| 593 | $CH_3OCH_2$ | $-OCH_2-$ | |
| 594 | $C_2H_5OCH_2$ | $-OCH_2-$ | |
| 595 | $CH_3CH(OCH_3)$ | $-OCH_2-$ | |
| 596 | $CN-CH_2$ | $-OCH_2-$ | |
| 597 | Cyclopropyl | $-OCH_2-$ | |
| 598 | Cyclobutyl | $-OCH_2-$ | |
| 599 | Cyclopentyl | $-OCH_2-$ | |
| 600 | Cyclohexyl | $-OCH_2-$ | |

| Nr. | Z | Y | Daten |
|---|---|---|---|
| 601 | 1-Methylcyclopropyl | $-OCH_2-$ | |
| 602 | 2,2-Dichlor-1-Methyl-cyclopropyl | $-OCH_2-$ | |
| 603 | 2,2-Dichlor-3,3-Dimethyl-cyclopropyl | $-OCH_2-$ | |
| 604 | 2,2,3,3-Tetramethylcyclopropyl | $-OCH_2-$ | |
| 605 | 2-(2'-Methyl-1'-propenyl-)-3,3-Dimethylcyclopropyl | $-OCH_2-$ | |
| 606 | 2-(2',2'-Difluorvinyl)-3,3-Dimethylcyclopropyl | $-OCH_2-$ | |
| 607 | 2-(2',2'-Dichlorvinyl)-3,3-Dimethylcyclopropyl | $-OCH_2-$ | |
| 608 | 2-(2',2'Dibromvinyl)-3,3-Dimethylcyclopropyl | $-OCH_2-$ | |
| 609 | 2-Phenylcyclopropyl | $-OCH_2-$ | |
| 610 | 2-(4'-Chlorphenyl)cyclopropyl | $-OCH_2-$ | |
| 611 | 2,2-Dichlor-3-Phenylcyclopropyl | $-OCH_2-$ | |
| 612 | 2-Carbomethoxy-cyclopropyl | $-OCH_2-$ | |
| 613 | $Cl_2CH$ | $-OCH_2-$ | |
| 614 | $Cl_2CH$ | $-OCH_2-$ | |
| 615 | $Cl_3C$ | $-OCH_2-$ | |
| 616 | $BrCH_2$ | $-OCH_2-$ | |
| 617 | $CF_3$ | $-OCH_2-$ | |
| 618 | $CH_3CH(Cl)$ | $-OCH_2-$ | |
| 619 | $C_6H_5$ (=Phenyl) | $-OCH_2-$ | |
| 620 | $2-CH_3-C_6H_4$ | $-OCH_2-$ | |
| 621 | $3-CH_3-C_6H_4$ | $-OCH_2-$ | |
| 622 | $4-CH_3-C_6H_4$ | $-OCH_2-$ | |
| 623 | $2,3-(CH_3)_2-C_6H_3$ | $-OCH_2-$ | |
| 624 | $2,4-(CH_3)_2-C_6H_3$ | $-OCH_2-$ | |
| 625 | $2,6-(CH_3)_2-C_6H_3$ | $-OCH_2-$ | |

| Nr. | Z | Y | Daten |
|---|---|---|---|
| 626 | $3,4-(CH_3)_2-C_6H_3$ | $-OCH_2-$ | |
| 627 | $3,5-(CH_3)_2-C_6H_3$ | $-OCH_2-$ | |
| 628 | $2,4,6-(CH_3)_2-C_6H_2$ | $-OCH_2-$ | |
| 629 | $4-t-C_4H_9-C_6H_4$ | $-OCH_2-$ | |
| 630 | $2-C_6H_5-C_6H_4$ | $-OCH_2-$ | |
| 631 | $4-C_6H_5-C_6H_4$ | $-OCH_2-$ | |
| 632 | $2-Cl-C_6H_4$ | $-OCH_2-$ | Öl; $^1H$-NMR ($CDCl_3$): $\delta$ = 3.75(3H), 5.03(2H), 5.83(2H), 6.58(1H), 6.90(1H), 7.0-7.6(8H) |
| 633 | $3-Cl-C_6H_4$ | $-OCH_2-$ | |
| 634 | $4-Cl-C_6H_4$ | $-OCH_2-$ | |
| 635 | $2,4-Cl_2-C_6H_3$ | $-OCH_2-$ | |
| 636 | $2,5-Cl_2-C_6H_3$ | $-OCH_2-$ | |
| 637 | $2,6-Cl_2-C_6H_3$ | $-OCH_2-$ | |
| 638 | $3,4-Cl_2-C_6H_3$ | $-OCH_2-$ | |
| 639 | $3,5-Cl_2-C_6H_3$ | $-OCH_2-$ | |
| 640 | $2,4,5-Cl_3-C_6H_2$ | $-OCH_2-$ | |
| 641 | $2,3,4,5,6-Cl_5-C_6$ | $-OCH_2-$ | |
| 642 | $6-F,2-Cl-C_6H_3$ | $-OCH_2-$ | |
| 643 | $2-F-C_6H_4$ | $-OCH_2-$ | |
| 644 | $3-F-C_6H_4$ | $-OCH_2-$ | |
| 645 | $4-F-C_6H_4$ | $-OCH_2-$ | |
| 646 | $2,4-F_2-C_6H_3$ | $-OCH_2-$ | |
| 647 | $2,6-F_2-C_6H_3$ | $-OCH_2-$ | |
| 648 | $2,3,4,5,6-F_5-C_6$ | $-OCH_2-$ | |
| 649 | $2-Br-C_6H_4$ | $-OCH_2-$ | Öl; $^1H$-NMR ($CDCl_3$): $\delta$ = 3.77(3H), 5.03(2H), 5.83(2H), 6.60(1H), 6.83(2H), 7.1-7.7(6H) |
| 650 | $3-Br-C_6H_4$ | $-OCH_2-$ | |

| Nr. | Z | Y | Daten |
|---|---|---|---|
| 651 | 4-Br-$C_6H_4$ | $-OCH_2-$ | |
| 652 | 2-$CF_3$-$C_6H_4$ | $-OCH_2-$ | |
| 653 | 3-$CF_3$-$C_6H_4$ | $-OCH_2-$ | |
| 654 | 4-$CF_3$-$C_6H_4$ | $-OCH_2-$ | |
| 655 | 2-$OCH_3$-$C_6H_4$ | $-OCH_2-$ | |
| 656 | 3-$OCH_3$-$C_6H_4$ | $-OCH_2-$ | |
| 657 | 4-$OCH_3$-$C_6H_4$ | $-OCH_2-$ | |
| 658 | 3,4-$(OCH_3)_2$-$C_6H_3$ | $-OCH_2-$ | |
| 659 | 3,4,5-$(OCH_3)_3$-$C_6H_2$ | $-OCH_2-$ | |
| 660 | 4-t-$C_4H_9O$-$C_6H_4$ | $-OCH_2-$ | |
| 661 | 2-Phenoxy-$C_6H_4$ | $-OCH_2-$ | |
| 662 | 3-Phenoxy-$C_6H_4$ | $-OCH_2-$ | |
| 663 | 4-Phenoxy-$C_6H_4$ | $-OCH_2-$ | |
| 664 | $C_6H_5$-$CH_2$ | $-OCH_2-$ | |
| 665 | 2-F-$C_6H_4$-$CH_2$ | $-OCH_2-$ | |
| 666 | 3-F-$C_6H_4$-$CH_2$ | $-OCH_2-$ | |
| 667 | 4-F-$C_6H_4$-$CH_2$ | $-OCH_2-$ | |
| 668 | 2-Cl-$C_6H_4$-$CH_2$ | $-OCH_2-$ | |
| 669 | 3-Cl-$C_6H_4$-$CH_2$ | $-OCH_2-$ | |
| 670 | 4-Cl-$C_6H_4$-$CH_2$ | $-OCH_2-$ | |
| 671 | 2-Cl,6F-$C_6H_3$-$CH_2$ | $-OCH_2-$ | |
| 672 | 2,4-$Cl_2$-$C_6H_3$-$CH_2$ | $-OCH_2-$ | |
| 673 | 2,6-$Cl_2$-$C_6H_3$-$CH_2$ | $-OCH_2-$ | |
| 674 | 3,5-$Cl_2$-$C_6H_3$-$CH_2$ | $-OCH_2-$ | |
| 675 | 2,4,6-$Cl_3$-$C_6H_2$-$CH_2$ | $-OCH_2-$ | |

| Nr. | Z | Y | Daten |
|---|---|---|---|
| 676 | $2-CH_3-C_6H_4-CH_2$ | $-OCH_2-$ | |
| 677 | $3-CH_3-C_6H_4-CH_2$ | $-OCH_2-$ | |
| 678 | $4-CH_3-C_6H_4-CH_2$ | $-OCH_2-$ | |
| 679 | $4-t-C_4H_9-C_6H_4-CH_2$ | $-OCH_2-$ | |
| 680 | $2,4-(CH_3)_2-C_6H_3-CH_2$ | $-OCH_2-$ | |
| 681 | $2,6-(CH_3)_2-C_6H_3-CH_2$ | $-OCH_2-$ | |
| 682 | $2,4,6-(CH_3)_3-C_6H_2-CH_2$ | $-OCH_2-$ | |
| 683 | $2-OCH_3-C_6H_4-CH_2$ | $-OCH_2-$ | |
| 684 | $3-OCH_3-C_6H_4-CH_2$ | $-OCH_2-$ | |
| 685 | $4-OCH_3-C_6H_4-CH_2$ | $-OCH_2-$ | |
| 686 | $2-CF_3-C_6H_4-CH_2$ | $-OCH_2-$ | |
| 687 | $3-CF_3-C_6H_4-CH_2$ | $-OCH_2-$ | |
| 688 | $4-CF_3-C_6H_4-CH_2$ | $-OCH_2-$ | |
| 689 | $4-O-t-C_4H_9-C_6H_4-CH_2$ | $-OCH_2-$ | |
| 690 | $4-O-C_6H_5-C_6H_4-CH_2$ | $-OCH_2-$ | |
| 691 | $4-C_6H_5-C_6H_4-CH_2$ | $-OCH_2-$ | |
| 692 | $2-Br-C_6H_4-CH_2$ | $-OCH_2-$ | |
| 693 | $3-Br-C_6H_4-CH_2$ | $-OCH_2-$ | |
| 694 | $4-Br-C_6H_4-CH_2$ | $-OCH_2-$ | |
| 695 | $C_6H_5-CH(CH_3)$ | $-OCH_2-$ | |
| 696 | $C_6H_5-CH(C_2H_5)$ | $-OCH_2-$ | |
| 697 | $C_6H_5-CH(iso-C_3H_7)$ | $-OCH_2-$ | |
| 698 | $C_6H_5-CH_2CH_2$ | $-OCH_2-$ | |
| 699 | $C_6H_5-CH_2CH(CH_3)$ | $-OCH_2-$ | |
| 700 | $4-t-C_4H_9-C_6H_4-CH_2CH_2$ | $-OCH_2-$ | |

| Nr. | Z | Y | Daten |
|---|---|---|---|
| 701 | $4-t-C_4H_9-C_6H_4-\ CH_2CH(CH_3)$ | $-OCH_2-$ | |
| 702 | $2-Cl-C_6H_4-CH_2CH_2$ | $-OCH_2-$ | |
| 703 | $3-Cl-C_6H_4-CH_2CH_2$ | $-OCH_2-$ | |
| 704 | $4-Cl-C_6H_4-CH_2CH_3$ | $-OCH_2-$ | |
| 705 | $C_6H_5-CH=CH$ | $-OCH_2-$ | |
| 706 | $C_6H_5-(CH_2)_3$ | $-OCH_2-$ | |
| 707 | $C_6H_5-CH_2CH(CH_3)CH_2$ | $-OCH_2-$ | |
| 708 | $C_6H_5-(CH_2)_4$ | $-OCH_2-$ | |
| 709 | $C_6H_5-(CH_2)_5$ | $-OCH_2-$ | |
| 710 | $C_6H_5-(CH_2)_6$ | $-OCH_2-$ | |
| 711 | $4-t-C_4H_9-C_6H_4-CH_2CH(CH_3)CH_2$ | $-OCH_2-$ | |
| 712 | H | $-CO-OCH_2-$ | |
| 713 | $CH_3$ | $-CO-OCH_2-$ | |
| 714 | $C_2H_5$ | $-CO-OCH_2-$ | |
| 715 | $n-C_3H_7$ | $-CO-OCH_2-$ | |
| 716 | $iso-C_3H_7$ | $-CO-OCH_2-$ | |
| 717 | $n-C_4H_9$ | $-CO-OCH_2-$ | |
| 718 | $(CH_3)_2CHCH_2$ | $-CO-OCH_2-$ | |
| 719 | $CH_3CH_2CH(CH_3)$ | $-CO-OCH_2-$ | |
| 720 | $tert.C_4H_9$ | $-CO-OCH_2-$ | öl; IR (Film): 1727,1480, 1281,1150,771 $cm^{-1}$ |
| 721 | $CH_3CH_2C(CH_3)_2$ | $-CO-OCH_2-$ | |
| 722 | $(CH_3)_3CCH_2$ | $-CO-OCH_2-$ | |
| 723 | $(CH_3)_2CHCH_2CH_2$ | $-CO-OCH_2-$ | |
| 724 | $CH_3CH_2CH_2CH(C_2H_5)$ | $-CO-OCH_2-$ | |
| 725 | $n-C_5H_{11}$ | $-CO-OCH_2-$ | |

| Nr. | Z | Y | Daten |
|---|---|---|---|
| 726 | $n-C_6H_{13}$ | $-CO-OCH_2-$ | |
| 727 | $n-C_7H_{15}$ | $-CO-OCH_2-$ | |
| 728 | $n-C_8H_{17}$ | $-CO-OCH_2-$ | |
| 729 | $n-C_9H_{19}$ | $-CO-OCH_2-$ | |
| 730 | $n-C_{10}H_{21}$ | $-CO-OCH_2-$ | |
| 731 | $n-C_{15}H_{31}$ | $-CO-OCH_2-$ | |
| 732 | $n-C_{17}H_{35}$ | $-CO-OCH_2-$ | |
| 733 | $CH_2=CH$ | $-CO-OCH_2-$ | |
| 734 | $CH_2=C(CH_3)$ | $-CO-OCH_2-$ | |
| 735 | $CH_3CH=CH$ | $-CO-OCH_2-$ | |
| 736 | $CH_3CH=C(CH_3)$ | $-CO-OCH_2-$ | |
| 737 | $(CH_3)_2C=CH$ | $-CO-OCH_2-$ | |
| 738 | $CH_2=CHCH_2$ | $-CO-OCH_2-$ | |
| 739 | $CH_3CH=CHCH_2$ | $-CO-OCH_2-$ | |
| 740 | $(CH_3)_2C=CHCH_2$ | $-CO-OCH_2-$ | |
| 741 | $CH_3CH=CH-CH=CH$ | $-CO-OCH_2-$ | |
| 742 | $(CH_3)_2C=CHCH_2CH_2C(CH_3)=CH$ | $-CO-OCH_2-$ | |
| 743 | $(CH_3)_2CHCH_2CH_2CH_2CH(CH_3)CH_2$ | $-CO-OCH_2-$ | |
| 744 | $(CH_3)_2C=CHCH_2CH_2CH(CH_3)CH_2$ | $-CO-OCH_2-$ | |
| 745 | $HC\equiv C$ | $-CO-OCH_2-$ | |
| 746 | $C_6H_5-C\equiv C$ | $-CO-OCH_2-$ | |
| 747 | $CH_3OCH_2$ | $-CO-OCH_2-$ | |
| 748 | $C_2H_5OCH_2$ | $-CO-OCH_2-$ | |
| 749 | $CH_3CH(OCH_3)$ | $-CO-OCH_2-$ | |
| 750 | $CN-CH_2$ | $-CO-OCH_2-$ | |

| Nr. | Z | Y | Daten |
|---|---|---|---|
| 751 | Cyclopropyl | $-CO-OCH_2-$ | Öl; IR (Film): 2958,1726, 1399,1168,1082,772 $cm^{-1}$ |
| 752 | Cyclobutyl | $-CO-OCH_2-$ | |
| 753 | Cyclopentyl | $-CO-OCH_2-$ | |
| 754 | Cyclohexyl | $-CO-OCH_2-$ | |
| 755 | 1-Methylcyclopropyl | $-CO-OCH_2-$ | Öl; IR (Film): 1724,1323,1214,1197, 1157,771,755 $cm^{-1}$ |
| 756 | 2,2-Dichlor-1-Methyl-cyclopropyl | $-CO-OCH_2-$ | |
| 757 | 2,2-Dichlor-3,3-Dimethyl-cyclopropyl | $-CO-OCH_2-$ | |
| 758 | 2,2,3,3-Tetramethylcyclopropyl | $-CO-OCH_2-$ | |
| 759 | 2-(2'-Methyl-1'-propenyl-)-3,3-Dimethylcyclopropyl | $-CO-OCH_2-$ | |
| 760 | 2-(2',2'-Difluorvinyl)-3,3-Dimethylcyclopropyl | $-CO-OCH_2-$ | |
| 761 | 2-(2',2'-Dichlorvinyl)-3,3-Dimethylcyclopropyl | $-CO-OCH_2-$ | |
| 762 | 2-(2',2'Dibromvinyl)-3,3-Dimethylcyclopropyl | $-CO-OCH_2-$ | |
| 763 | 2-Phenylcyclopropyl | $-CO-OCH_2-$ | |
| 764 | 2-(4'-Chlorphenyl)cyclopropyl | $-CO-OCH_2-$ | |
| 765 | 2,2-Dichlor-3-Phenylcyclopropyl | $-CO-OCH_2-$ | |
| 766 | 2-Carbomethoxy-cyclopropyl | $-CO-OCH_2-$ | |
| 767 | $Cl_2CH$ | $-CO-OCH_2-$ | |
| 768 | $Cl_2CH$ | $-CO-OCH_2-$ | |
| 769 | $Cl_3C$ | $-CO-OCH_2-$ | |
| 770 | $BrCH_2$ | $-CO-OCH_2-$ | |
| 771 | $CF_3$ | $-CO-OCH_2-$ | |
| 772 | $CH_3CH(Cl)$ | $-CO-OCH_2-$ | |

| Nr. | Z | Y | Daten |
|---|---|---|---|
| 773 | $C_6H_5$ (=Phenyl) | $-CO-OCH_2-$ | Öl; IR (Film) 1722, 1451, 1314, 1270 1213, 1110, 713 $cm^{-1}$ |
| 774 | $2-CH_3-C_6H_4$ | $-CO-OCH_2-$ | |
| 775 | $3-CH_3-C_6H_4$ | $-CO-OCH_2-$ | |
| 776 | $4-CH_3-C_6H_4$ | $-CO-OCH_2-$ | |
| 777 | $2,3-(CH_3)_2-C_6H_3$ | $-CO-OCH_2-$ | |
| 778 | $2,4-(CH_3)_2-C_6H_3$ | $-CO-OCH_2-$ | |
| 779 | $2,6-(CH_3)_2-C_6H_3$ | $-CO-OCH_2-$ | |
| 780 | $3,4-(CH_3)_2-C_6H_3$ | $-CO-OCH_2-$ | |
| 781 | $3,5-(CH_3)_2-C_6H_3$ | $-CO-OCH_2-$ | |
| 782 | $2,4,6-(CH_3)_2-C_6H_2$ | $-CO-OCH_2-$ | |
| 783 | $4-t-C_4H_9-C_6H_4$ | $-CO-OCH_2-$ | |
| 784 | $2-C_6H_5-C_6H_4$ | $-CO-OCH_2-$ | |
| 785 | $4-C_6H_5-C_6H_4$ | $-CO-OCH_2-$ | |
| 786 | $2-Cl-C_6H_4$ | $-CO-OCH_2-$ | |
| 787 | $3-Cl-C_6H_4$ | $-CO-OCH_2-$ | |
| 788 | $4-Cl-C_6H_4$ | $-CO-OCH_2-$ | |
| 789 | $2,4-Cl_2-C_6H_3$ | $-CO-OCH_2-$ | |
| 790 | $2,5-Cl_2-C_6H_3$ | $-CO-OCH_2-$ | |
| 791 | $2,6-Cl_2-C_6H_3$ | $-CO-OCH_2-$ | |
| 792 | $3,4-Cl_2-C_6H_3$ | $-CO-OCH_2-$ | |
| 793 | $3,5-Cl_2-C_6H_3$ | $-CO-OCH_2-$ | |
| 794 | $2,4,5-Cl_3-C_6H_2$ | $-CO-OCH_2-$ | |
| 795 | $2,3,4,5,6-Cl_5-C_6$ | $-CO-OCH_2-$ | |

| Nr. | Z | Y | Daten |
|---|---|---|---|
| 796 | $6-F,2-Cl-C_6H_3$ | $-CO-OCH_2-$ | |
| 797 | $2-F-C_6H_4$ | $-CO-OCH_2-$ | |
| 798 | $3-F-C_6H_4$ | $-CO-OCH_2-$ | |
| 799 | $4-F-C_6H_4$ | $-CO-OCH_2-$ | |
| 800 | $2,4-F_2-C_6H_3$ | $-CO-OCH_2-$ | |
| 801 | $2,6-F_2-C_6H_3$ | $-CO-OCH_2-$ | |
| 802 | $2,3,4,5,6-F_5-C_6$ | $-CO-OCH_2-$ | |
| 803 | $2-Br-C_6H_4$ | $-CO-OCH_2-$ | |
| 804 | $3-Br-C_6H_4$ | $-CO-OCH_2-$ | |
| 805 | $4-Br-C_6H_4$ | $-CO-OCH_2-$ | |
| 806 | $2-CF_3-C_6H_4$ | $-CO-OCH_2-$ | |
| 807 | $3-CF_3-C_6H_4$ | $-CO-OCH_2-$ | |
| 808 | $4-CF_3-C_6H_4$ | $-CO-OCH_2-$ | |
| 809 | $2-OCH_3-C_6H_4$ | $-CO-OCH_2-$ | |
| 810 | $3-OCH_3-C_6H_4$ | $-CO-OCH_2-$ | |
| 811 | $4-OCH_3-C_6H_4$ | $-CO-OCH_2-$ | |
| 812 | $3,4-(OCH_3)_2-C_6H_3$ | $-CO-OCH_2-$ | |
| 813 | $3,4,5-(OCH_3)_3-C_6H_2$ | $-CO-OCH_2-$ | |
| 814 | $4-t-C_4H_9O-C_6H_4$ | $-CO-OCH_2-$ | |
| 815 | 2-Phenoxy-$C_6H_4$ | $-CO-OCH_2-$ | |
| 816 | 3-Phenoxy-$C_6H_4$ | $-CO-OCH_2-$ | |
| 817 | 4-Phenoxy-$C_6H_4$ | $-CO-OCH_2-$ | |
| 818 | $C_6H_5-CH_2$ | $-CO-OCH_2-$ | |
| 819 | $2-F-C_6H_4-CH_2$ | $-CO-OCH_2-$ | |
| 820 | $3-F-C_6H_4-CH_2$ | $-CO-OCH_2-$ | |

| Nr. | Z | Y | Daten |
|---|---|---|---|
| 821 | 4-F-$C_6H_4$-$CH_2$ | -CO-$OCH_2$- | |
| 822 | 2-Cl-$C_6H_4$-$CH_2$ | -CO-$OCH_2$- | |
| 823 | 3-Cl-$C_6H_4$-$CH_2$ | -CO-$OCH_2$- | |
| 824 | 4-Cl-$C_6H_4$-$CH_2$ | -CO-$OCH_2$- | |
| 825 | 2-Cl,6F-$C_6H_3$-$CH_2$ | -CO-$OCH_2$- | |
| 826 | 2,4-$Cl_2$-$C_6H_3$-$CH_2$ | -CO-$OCH_2$- | |
| 827 | 2,6-$Cl_2$-$C_6H_3$-$CH_2$ | -CO-$OCH_2$- | |
| 828 | 3,5-$Cl_2$-$C_6H_3$-$CH_2$ | -CO-$OCH_2$- | |
| 829 | 2,4,6-$Cl_3$-$C_6H_2$-$CH_2$ | -CO-$OCH_2$- | |
| 830 | 2-$CH_3$-$C_6H_4$-$CH_2$ | -CO-$OCH_2$- | |
| 831 | 3-$CH_3$-$C_6H_4$-$CH_2$ | -CO-$OCH_2$- | |
| 832 | 4-$CH_3$-$C_6H_4$-$CH_2$ | -CO-$OCH_2$- | |
| 833 | 4-t-$C_4H_9$-$C_6H_4$-$CH_2$ | -CO-$OCH_2$- | |
| 834 | 2,4-$(CH_3)_2$-$C_6H_3$-$CH_2$ | -CO-$OCH_2$- | |
| 835 | 2,6-$(CH_3)_2$-$C_6H_3$-$CH_2$ | -CO-$OCH_2$- | |
| 836 | 2,4,6-$(CH_3)_3$-$C_6H_2$-$CH_2$ | -CO-$OCH_2$- | |
| 837 | 2-$OCH_3$-$C_6H_4$-$CH_2$ | -CO-$OCH_2$- | |
| 838 | 3-$OCH_3$-$C_6H_4$-$CH_2$ | -CO-$OCH_2$- | |
| 839 | 4-$OCH_3$-$C_6H_4$-$CH_2$ | -CO-$OCH_2$- | |
| 840 | 2-$CF_3$-$C_6H_4$-$CH_2$ | -CO-$OCH_2$- | |
| 841 | 3-$CF_3$-$C_6H_4$-$CH_2$ | -CO-$OCH_2$- | |
| 842 | 4-$CF_3$-$C_6H_4$-$CH_2$ | -CO-$OCH_2$- | |
| 843 | 4-O-t-$C_4H_9$-$C_6H_4$-$CH_2$ | -CO-$OCH_2$- | |
| 844 | 4-O-$C_6H_5$-$C_6H_4$-$CH_2$ | -CO-$OCH_2$- | |
| 845 | 4-$C_6H_5$-$C_6H_4$-$CH_2$ | -CO-$OCH_2$- | |

| Nr. | Z | Y | Daten |
|---|---|---|---|
| 846 | $2\text{-Br-}C_6H_4\text{-}CH_2$ | $\text{-CO-O}CH_2\text{-}$ | |
| 847 | $3\text{-Br-}C_6H_4\text{-}CH_2$ | $\text{-CO-O}CH_2\text{-}$ | |
| 848 | $4\text{-Br-}C_6H_4\text{-}CH_2$ | $\text{-CO-O}CH_2\text{-}$ | |
| 849 | $C_6H_5\text{-CH}(CH_3)$ | $\text{-CO-O}CH_2\text{-}$ | |
| 850 | $C_6H_5\text{-CH}(C_2H_5)$ | $\text{-CO-O}CH_2\text{-}$ | |
| 851 | $C_6H_5\text{-CH(iso-}C_3H_7)$ | $\text{-CO-O}CH_2\text{-}$ | |
| 852 | $C_6H_5\text{-CH(OH)}$ | $\text{-CO-O}CH_2\text{-}$ | |
| 853 | $2\text{-O}CH_3\text{-}C_6H_4\text{-CH(OH)}$ | $\text{-CO-O}CH_2\text{-}$ | |
| 854 | $4\text{-O}CH_3\text{-}C_6H_4\text{-CH(OH)}$ | $\text{-CO-O}CH_2\text{-}$ | |
| 855 | $3,4\text{-}(OCH_3)_2\text{-}C_6H_3\text{-CH(OH)}$ | $\text{-CO-O}CH_2\text{-}$ | |
| 856 | $2\text{-O}CH_3\text{-}C_6H_4\text{-CH}(OCH_3)$ | $\text{-CO-O}CH_2\text{-}$ | |
| 857 | $4\text{-O}CH_3\text{-}C_6H_4\text{-CH}(OCH_3)$ | $\text{-CO-O}CH_2\text{-}$ | |
| 858 | $3,4\text{-}(OCH_3)_2\text{-}C_6H_3\text{-CH}(OCH_3)$ | $\text{-CO-O}CH_2\text{-}$ | |
| 859 | $C_6H_5\text{-}CH_2CH_2$ | $\text{-CO-O}CH_2\text{-}$ | |
| 860 | $C_6H_5\text{-}CH_2CH(CH_3)$ | $\text{-CO-O}CH_2\text{-}$ | |
| 861 | $4\text{-t-}C_4H_9\text{-}C_6H_4\text{-}CH_2CH_2$ | $\text{-CO-O}CH_2\text{-}$ | |
| 862 | $4\text{-t-}C_4H_9\text{-}C_6H_4\text{- }CH_2CH(CH_3)$ | $\text{-CO-O}CH_2\text{-}$ | |
| 863 | $2\text{-Cl-}C_6H_4\text{-}CH_2CH_2$ | $\text{-CO-O}CH_2\text{-}$ | |
| 864 | $3\text{-Cl-}C_6H_4\text{-}CH_2CH_2$ | $\text{-CO-O}CH_2\text{-}$ | |
| 865 | $4\text{-Cl-}C_6H_4\text{-}CH_2CH_3$ | $\text{-CO-O}CH_2\text{-}$ | |
| 866 | $C_6H_5\text{-CH=CH}$ | $\text{-CO-O}CH_2\text{-}$ | öl; IR (Film) 1719,1637,1311,1204 1164,768 $cm^{-1}$ |
| 867 | $2\text{-Cl-}C_6H_4\text{-CH=CH}$ | $\text{-CO-O}CH_2\text{-}$ | |
| 868 | $3\text{-Cl-}C_6H_4\text{-CH=CH}$ | $\text{-CO-O}CH_2\text{-}$ | |

| Nr. | Z | Y | Daten |
|---|---|---|---|
| 869 | $4-Cl-C_6H_4-CH=CH$ | $-CO-OCH_2-$ | |
| 870 | $2,4-Cl_2-C_6H_3-CH=CH$ | $-CO-OCH_2-$ | |
| 871 | $2-F-C_6H_4-CH=CH$ | $-CO-OCH_2-$ | |
| 872 | $4-F-C_6H_4-CH=CH$ | $-CO-OCH_2$ | |
| 873 | $2-CH_3-C_6H_4-CH=CH$ | $-CO-OCH_2-$ | |
| 874 | $4-CH_3-C_6H_4-CH=CH$ | $-CO-OCH_2-$ | |
| 875 | $4-t-C_4H_9-C_6H_4-CH=CH$ | $-CO-OCH_2-$ | |
| 876 | $2-OCH_3-C_6H_4-CH=CH$ | $-CO-OCH_2-$ | |
| 877 | $4-OCH_3-C_6H_4-CH=CH$ | $-CO-OCH_2-$ | |
| 878 | 4-Phenoxy-$C_6H_4-CH=CH$ | $-CO-OCH_2-$ | |
| 879 | $C_6H_5-(CH_2)_3$ | $-CO-OCH_2-$ | |
| 880 | $C_6H_5-CH_2CH(CH_3)CH_2$ | $-CO-OCH_2-$ | |
| 881 | $C_6H_5-(CH_2)_4$ | $-CO-OCH_2-$ | |
| 882 | $C_6H_5-(CH_2)_5$ | $-CO-OCH_2-$ | |
| 883 | $C_6H_5-(CH_2)_6$ | $-CO-OCH_2-$ | |
| 884 | $4-t-C_4H_9-C_6H_4-CH_2CH(CH_3)CH_2$ | $-CO-OCH_2-$ | |
| 885 | $C_6H_5$ | $-C\equiv C-$ | |
| 886 | $2-Cl-C_6H_4$ | $-C\equiv C-$ | |
| 887 | $3-Cl-C_6H_4$ | $-C\equiv C-$ | |
| 888 | $4-Cl-C_6H_4$ | $-C\equiv C-$ | |
| 889 | $C_6H_5OCH_2$ | $-C\equiv C-$ | |
| 890 | $2-Cl-C_6H_5OCH_2$ | $-C\equiv C-$ | |
| 891 | $3-Cl-C_6H_5OCH_2$ | $-C\equiv C-$ | |
| 892 | $4-Cl-C_6H_5OCH_2$ | $-C\equiv C-$ | |
| 893 | $CH_3OOC$ | $-C\equiv C-$ | |

| Nr. | Z | Y | Daten |
|---|---|---|---|
| 894 | t-$C_4H_9OOC$ | $-C{\equiv}C-$ | |
| 895 | $C_6H_5$ | $-CH_2-$ | |
| 896 | 2-F-$C_6H_4$ | $-CH_2-$ | |
| 897 | 3-F-$C_6H_4$ | $-CH_2-$ | |
| 898 | 4-F-$C_6H_4$ | $-CH_2-$ | |
| 899 | 2-Cl-$C_6H_4$ | $-CH_2-$ | |
| 900 | 3-Cl-$C_6H_4$ | $-CH_2-$ | |
| 901 | 4-Cl-$C_6H_4$ | $-CH_2-$ | |
| 902 | 2Cl,6F-$C_6H_3$ | $-CH_2-$ | |
| 903 | 2,4-$Cl_2$-$C_6H_3$ | $-CH_2-$ | |
| 904 | 2,6-$Cl_2$-$C_6H_3$ | $-CH_2-$ | |
| 905 | 3,5-$Cl_2$-$C_6H_3$ | $-CH_2-$ | |
| 906 | 2,4,6-$Cl_3$-$C_6H_2$ | $-CH_2-$ | |
| 907 | 2-$CH_3$-$C_6H_4$ | $-CH_2-$ | |
| 908 | 3-$CH_3$-$C_6H_4$ | $-CH_2-$ | |
| 909 | 4-$CH_3$-$C_6H_4$ | $-CH_2-$ | |
| 910 | 4-t-$C_4H_9$-$C_6H_4$ | $-CH_2-$ | |
| 911 | 2,4-$(CH_3)_2$-$C_6H_3$ | $-CH_2-$ | |
| 912 | 2,6-$(CH_3)_2$-$C_6H_3$ | $-CH_2-$ | |
| 913 | 2,4,6-$(CH_3)_3$-$C_6H_2$ | $-CH_2-$ | |
| 914 | 2-$OCH_3$-$C_6H_4$ | $-CH_2-$ | |
| 915 | 3-$OCH_3$-$C_6H_4$ | $-CH_2-$ | |
| 916 | 4-$OCH_3$-$C_6H_4$ | $-CH_2-$ | |
| 917 | 2-$CF_3$-$C_6H_4$ | $-CH_2-$ | |
| 918 | 3-$CF_3$-$C_6H_4$ | $-CH_2-$ | |

| Nr. | Z | Y | Daten |
|---|---|---|---|
| 919 | 4-$CF_3$-$C_6H_4$ | -$CH_2$- | |
| 920 | 4-O-t-$C_4H_9$-$C_6H_4$ | -$CH_2$- | |
| 921 | 4-O-$C_6H_5$-$C_6H_4$ | -$CH_2$- | |
| 922 | 4-$C_6H_5$-$C_6H_4$ | -$CH_2$- | |
| 923 | 2-Br-$C_6H_4$ | -$CH_2$- | |
| 924 | 4-Br-$C_6H_4$ | -$CH_2$- | |
| 925 | Br | -$CH_2$- | Daten siehe Beschreibung |
| 926 | $Br^-(C_6H_5)_3P^+$ | -$CH_2$- | Daten siehe Beschreibung |
| 927 | $Cl^-(C_6H_5)_3P^+$ | -$CH_2$- | |
| 928 | $Br_2$ | $>CH-$ | |
| 929 | $Cl_2$ | $>CH-$ | |
| 930 | O=CH | - | |
| 931 | iso-$C_3H_7$ | O | |
| 932 | $CH_3CH_2CH(CH_3)$ | O | |
| 933 | tert.$C_4H_9$ | O | |
| 934 | $CH_3CH_2C(CH_3)_2$ | O | |
| 935 | $CH_3CH_2CH_2CH(C_2H_5)$ | O | |
| 936 | $CH_2$=CH-$CH_2$ | O | |
| 937 | $CH_3$CH=CH-$CH_2$ | O | |
| 938 | $(CH_3)_2$C=CH-$CH_2$ | O | |
| 939 | $CH_3OCH_2$ | O | |
| 940 | $CNCH_2$ | O | |
| 941 | Cyclopropyl | O | |
| 942 | Cyclobutyl | O | |
| 943 | Cyclopentyl | O | |

| Nr. | Z | Y | Daten |
|---|---|---|---|
| 944 | Cyclohexyl | O | |
| 945 | 1-Methylcyclohexyl | O | |
| 946 | 1-Methylcyclopropyl | O | |
| 947 | $Cl_3C$ | O | |
| 948 | $CF_3C$ | O | |
| 949 | $C_6H_5$ (=Phenyl) | O | |
| 950 | $2-CH_3-C_6H_4$ | O | |
| 951 | $3-CH_3-C_6H_4$ | O | |
| 952 | $4-CH_3-C_6H_4$ | O | |
| 953 | $2,3-(CH_3)_2-C_6H_3$ | O | |
| 954 | $2,4-(CH_3)_2-C_6H_3$ | O | |
| 955 | $2,6-(CH_3)_2-C_6H_3$ | O | |
| 956 | $3,4-(CH_3)_2-C_6H_3$ | O | |
| 957 | $3,5-(CH_3)_2-C_6H_3$ | O | |
| 958 | $2,4,6-(CH_3)_2-C_6H_2$ | O | |
| 959 | $4-t-C_4H_9-C_6H_4$ | O | |
| 960 | $2-C_6H_5-C_6H_4$ | O | |
| 961 | $4-C_6H_5-C_6H_4$ | O | |
| 962 | $2-Cl-C_6H_4$ | O | |
| 963 | $3-Cl-C_6H_4$ | O | |
| 964 | $4-Cl-C_6H_4$ | O | |
| 965 | $2,4-Cl_2-C_6H_3$ | O | |
| 966 | $2,5-Cl_2-C_6H_3$ | O | |
| 967 | $2,6-Cl_2-C_6H_3$ | O | |
| 968 | $3,4-Cl_2-C_6H_3$ | O | |

| Nr. | Z | Y | Daten |
|---|---|---|---|
| 969 | 3,5-$Cl_2$-$C_6H_3$ | O | |
| 970 | 2,4,5-$Cl_3$-$C_6H_2$ | O | |
| 971 | 2,3,4,5,6-$Cl_5$-$C_6$ | O | |
| 972 | 6-F,2-Cl-$C_6H_3$ | O | |
| 973 | 2-F-$C_6H_4$ | O | |
| 974 | 3-F-$C_6H_4$ | O | |
| 975 | 4-F-$C_6H_4$ | O | |
| 976 | 2,4-$F_2$-$C_6H_3$ | O | |
| 977 | 2,6-$F_2$-$C_6H_3$ | O | |
| 978 | 2,3,4,5,6-$F_5$-$C_6$ | O | |
| 979 | 2-Br-$C_6H_4$ | O | |
| 980 | 3-Br-$C_6H_4$ | O | |
| 981 | 4-Br-$C_6H_4$ | O | |
| 982 | 2-$CF_3$-$C_6H_4$ | O | |
| 983 | 3-$CF_3$-$C_6H_4$ | O | |
| 984 | 4-$CF_3$-$C_6H_4$ | O | |
| 985 | 2-$OCH_3$-$C_6H_4$ | O | |
| 986 | 3-$OCH_3$-$C_6H_4$ | O | |
| 987 | 4-$OCH_3$-$C_6H_4$ | O | |
| 988 | 3,4-$(OCH_3)_2$-$C_6H_3$ | O | |
| 989 | 3,4,5-$(OCH_3)_3$-$C_6H_2$ | O | |
| 990 | 4-t-$C_4H_9O$-$C_6H_4$ | O | |
| 991 | 2-Phenoxy-$C_6H_4$ | O | |
| 992 | 3-Phenoxy-$C_6H_4$ | O | |
| 993 | 4-Phenoxy-$C_6H_4$ | O | |

| Nr. | Z | Y | Daten |
|---|---|---|---|
| 994 | $C_6H_5$ | $-SCH_2-$ | |
| 995 | $2-F-C_6H_4$ | $-SCH_2-$ | |
| 996 | $3-F-C_6H_4$ | $-SCH_2-$ | |
| 997 | $4-F-C_6H_4$ | $-SCH_2-$ | |
| 998 | $2-Cl-C_6H_4$ | $-SCH_2-$ | |
| 999 | $3-Cl-C_6H_4$ | $-SCH_2-$ | |
| 1000 | $4-Cl-C_6H_4$ | $-SCH_2-$ | |
| 1001 | $2Cl,6F-C_6H_3$ | $-SCH_2-$ | |
| 1002 | $2,4-Cl_2-C_6H_3$ | $-SCH_2-$ | |
| 1003 | $2,6-Cl_2-C_6H_3$ | $-SCH_2-$ | |
| 1004 | $3,5-Cl_2-C_6H_3$ | $-SCH_2-$ | |
| 1005 | $2,4,6-Cl_3-C_6H_2$ | $-SCH_2-$ | |
| 1006 | $2-CH_3-C_6H_4$ | $-SCH_2-$ | |
| 1007 | $3-CH_3-C_6H_4$ | $-SCH_2-$ | |
| 1008 | $4-CH_3-C_6H_4$ | $-SCH_2-$ | |
| 1009 | $4-t-C_4H_9-C_6H_4$ | $-SCH_2-$ | |
| 1010 | $2,4-(CH_3)_2-C_6H_3$ | $-SCH_2-$ | |
| 1011 | $2,6-(CH_3)_2-C_6H_3$ | $-SCH_2-$ | |
| 1012 | $2,4,6-(CH_3)_3-C_6H_2$ | $-SCH_2-$ | |
| 1013 | $2-OCH_3-C_6H_4$ | $-SCH_2-$ | |
| 1014 | $3-OCH_3-C_6H_4$ | $-SCH_2-$ | |
| 1015 | $4-OCH_3-C_6H_4$ | $-SCH_2-$ | |
| 1016 | $2-CF_3-C_6H_4$ | $-SCH_2-$ | |
| 1017 | $3-CF_3-C_6H_4$ | $-SCH_2-$ | |
| 1018 | $4-CF_3-C_6H_4$ | $-SCH_3-$ | |

| Nr. | Z | Y | Daten |
|---|---|---|---|
| 1019 | $4-O-t-C_4H_9-C_6H_4$ | $-SCH_2-$ | |
| 1020 | $4-O-C_6H_5-C_6H_4$ | $-SCH_2-$ | |
| 1021 | $4-C_6H_5-C_6H_4$ | $-SCH_2-$ | |
| 1022 | $2-Br-C_6H_4$ | $-SCH_2-$ | |
| 1023 | $4-Br-C_6H_4$ | $-SCH_2-$ | |
| 1024 | $C_6H_5$ | -S- | |
| 1025 | $2-Cl-C_6H_4$ | -S- | |
| 1026 | $3-Cl-C_6H_4$ | -S- | |
| 1027 | $4-Cl-C_6H_4$ | -S- | |
| 1028 | $2-CH_3-C_6H_4$ | -S- | |
| 1029 | $4-CH_3-C_6H_4$ | -S- | |
| 1030 | $4-C_6H_5-C_6H_4$ | -S- | |
| 1031 | $CH_3$ | -S- | |
| 1032 | $C_6H_5O$ | $-CH_2CH_2O-$ | |
| 1033 | $2-Cl-C_6H_4O$ | $-CH_2CH_2O-$ | |
| 1034 | $3-Cl-C_6H_4O$ | $-CH_2CH_2O-$ | |
| 1035 | $4-Cl-C_6H_4O$ | $-CH_2CH_2O-$ | |
| 1036 | $2-CH_3-C_6H_4O$ | $-CH_2CH_2O-$ | |
| 1037 | $4-CH_3-C_6H_4O$ | $-CH_2CH_2O-$ | |
| 1038 | $2-OCH_3-C_6H_4O$ | $-CH_2CH_2O-$ | |
| 1039 | $4-OCH_3-C_6H_4O$ | $-CH_2CH_2O-$ | |
| 1040 | $2-CF_3-C_6H_4O$ | $-CH_2CH_2O-$ | |
| 1041 | $4-CF_3-C_6H_4O$ | $-CH_2CH_2O-$ | |
| 1042 | $C_6H_5OCH_2$ | $-CH_2CH_2O-$ | |
| 1043 | $2-Cl-C_6H_4OCH_2$ | $-CH_2CH_2O-$ | |

| Nr. | Z | Y | Daten |
|---|---|---|---|
| 1044 | $4\text{-}Cl\text{-}C_6H_4OCH_2$ | $-CH_2CH_2O-$ | |
| 1045 | $2\text{-}CH_3\text{-}C_6H_4OCH_2$ | $-CH_2CH_2O-$ | |
| 1046 | $4\text{-}CH_3\text{-}C_6H_4OCH_2$ | $-CH_2CH_2O-$ | |
| 1047 | $2\text{-}OCH_3\text{-}C_6H_4OCH_2$ | $-CH_2CH_2O-$ | |
| 1048 | $4\text{-}OCH_3\text{-}C_6H_4OCH_2$ | $-CH_2CH_2O-$ | |
| 1049 | $4\text{-}t\text{-}C_4H_9\text{-}C_6H_4OCH_2$ | $-CH_2CH_2O-$ | |
| 1050 | $C_6H_5OCH_2CH_2$ | $-CH_2CH_2O-$ | |
| 1051 | $2\text{-}Cl\text{-}C_6H_4OCH_2CH_2$ | $-CH_2CH_2O-$ | |
| 1052 | $4\text{-}Cl\text{-}C_6H_4OCH_2CH_2$ | $-CH_2CH_2O-$ | |
| 1053 | $4\text{-}F\text{-}C_6H_4OCH_2CH_2$ | $-CH_2CH_2O-$ | |
| 1054 | $2\text{-}CH_3\text{-}C_6H_4OCH_2CH_2$ | $-CH_2CH_2O-$ | |
| 1055 | $4\text{-}CH_3\text{-}C_6H_4OCH_2CH_2$ | $-CH_2CH_2O-$ | |
| 1056 | $2\text{-}OCH_3\text{-}C_6H_4OCH_2CH_2$ | $-CH_2CH_2O-$ | |
| 1057 | $4\text{-}OCH_3\text{-}C_6H_4OCH_2CH_2$ | $-CH_2CH_2O-$ | |
| 1058 | $4\text{-}t\text{-}C_4H_9\text{-}C_6H_4\text{-}OCH_2CH_2$ | $CH_2CH_2O-$ | |
| 1059 | $C_6H_5O(CH_2)_3$ | $-CH_2CH_2O-$ | |
| 1060 | $2\text{-}Cl\text{-}C_6H_4O(CH_2)_3$ | $-CH_2CH_2O-$ | |
| 1061 | $4\text{-}Cl\text{-}C_6H_4O(CH_2)_3$ | $-CH_2CH_2O-$ | |
| 1062 | $C_6H_5O(CH_2)_4$ | $-CH_2CH_2O-$ | |
| 1063 | $2\text{-}Cl\text{-}C_6H_4\text{-}O(CH_2)_4$ | $-CH_2CH_2O-$ | |
| 1064 | $4\text{-}Cl\text{-}C_6H_4O(CH_2)_4$ | $-CH_2CH_2O-$ | |
| 1065 | $C_6H_5O(CH_2)_5$ | $-CH_2CH_2O-$ | |
| 1066 | $2\text{-}Cl\text{-}C_6H_4O(CH_2)_5$ | $-CH_2CH_2O-$ | |
| 1067 | $4\text{-}Cl\text{-}C_6H_4O(CH_2)_5$ | $-CH_2CH_2O-$ | |
| 1068 | $C_6H_5O(CH_2)_6$ | $-CH_2CH_2O-$ | |

| Nr. | Z | Y | Daten |
|---|---|---|---|
| 1069 | $C_6H_5OCH_2CH_2O$ | $-CH_2CH_2O-$ | |
| 1070 | $C_6H_5$ | -CO-O- | |
| 1071 | $2-Cl-C_6H_4$ | -CO-O- | |
| 1072 | $3-Cl-C_6H_4$ | -CO-O- | |
| 1073 | $4-Cl-C_6H_4$ | -CO-O- | |
| 1074 | $2-CH_3-C_6H_4$ | -CO-O- | |
| 1075 | $4-CH_3-C_6H_4$ | -CO-O- | |
| 1076 | $4-C_6H_5-C_6H_4$ | -CO-O- | |
| 1077 | $C_6H_5$ | -CO-O- | |
| 1078 | $2-Cl-C_6H_4$ | -CO-O- | |
| 1079 | $3-Cl-C_6H_4$ | -CO-O- | |
| 1080 | $4-Cl-C_6H_4$ | -CO-O- | |
| 1081 | $2-CH_3-C_6H_4$ | -CO-O- | |
| 1082 | $CH_3$ | -CO-O- | |
| 1083 | $t-C_4H_9$ | -CO-O- | |
| 1084 | $CH_2C_6H_5$ | -CO-O- | |
| 1085 | $C_6H_5$ | -HN-CO-O- | |
| 1086 | $2-Cl-C_6H_4$ | -HN-CO-O- | |
| 1087 | $4-Cl-C_6H_4$ | -HN-CO-O- | |
| 1088 | $C_6H_{11}$ | -NH-CO-O- | |
| 1089 | $n-C_4H_9$ | -NH-CO-O- | |
| 1090 | 2-Furyl | -CH=CH- | |
| 1091 | 5-Nitro-2-furyl | -CH=CH- | |
| 1092 | 5-Chlor-2-furyl | -CH=CH- | |
| 1093 | 2-(2'-Furyl)-ethenyl | -CH=CH- | |

| Nr. | Z | Y | Daten |
|---|---|---|---|
| 1094 | 2-(5'-Nitro-2'-furyl)ethenyl | -CH=CH- | |
| 1095 | 3-Furyl | -CH=CH- | |
| 1096 | 5-Nitro-3-furyl | -CH=CH- | |
| 1097 | 5-Chlor-3-furyl | -CH=CH- | |
| 1098 | 2-(3'-Furyl)ethenyl | -CH=CH- | |
| 1099 | 2-(5'-Nitro-3'-furyl)ethenyl | -CH=CH- | |
| 1100 | Benzofuran-2-yl | -CH=CH- | |
| 1101 | Benzofuran-3-yl | -CH=CH- | |
| 1102 | 2-Thienyl | -CH=CH- | |
| 1103 | 5-Nitro-2-thienyl | -CH=CH- | |
| 1104 | 5-Chlor-2-thienyl | -CH=CH- | |
| 1105 | 2-(2'-Thienyl)ethenyl | -CH=CH- | |
| 1106 | 2-(5'-Nitro-2'-thienyl)ethenyl | -CH=CH- | |
| 1107 | 3-Thienyl | -CH=CH- | |
| 1108 | 5-Nitro-3-thienyl | -CH=CH- | |
| 1109 | 5-Chlor-3-thienyl | -CH=CH- | |
| 1110 | 2-(3'-thienyl)ethenyl | -CH=CH- | |
| 1111 | 2-(5'-Nitro-3'-thienyl)ethenyl | -CH=CH- | |
| 1112 | Benzothien-2-yl | -CH=CH- | |
| 1113 | Benzothien-3-yl | -CH=CH- | |
| 1114 | N-Methyl-pyrrol-2-yl | -CH=CH- | |
| 1115 | N-Methyl-pyrrol-3-yl | -CH=CH- | |
| 1116 | N-Methyl-pyrazol-3-yl | -CH=CH- | |
| 1117 | N-Methyl-pyrazol-4-yl | -CH=CH- | |
| 1118 | N-Methyl-pyrazol-5-yl | -CH=CH- | |

| Nr. | Z | Y | Daten |
|---|---|---|---|
| 1119 | N-Methyl-imidazol-2-yl | -CH=CH- | |
| 1120 | N-Methyl-imidazol-4-yl | -CH=CH- | |
| 1121 | N-Methyl-imidazol-5-yl | -CH=CH- | |
| 1122 | 1-Methyl-1,2,3-triazol-4-yl | -CH=CH- | |
| 1123 | 1-Methyl-1,2,3-triazol-5-yl | -CH=CH- | |
| 1124 | 1-Methyl-1,2,4-triazol-3-yl | -CH=CH- | |
| 1125 | 1-Methyl-1,2,4-triazol-5-yl | -CH=CH- | |
| 1126 | 1-Methyl-tetrazol-5-yl | -CH=CH- | |
| 1127 | Isoxazol-3-yl | -CH=CH- | |
| 1128 | Isoxazol-4-yl | -CH=CH- | |
| 1129 | Isoxazol-5-yl | -CH=CH- | |
| 1130 | Benzisoxazol-3-yl | -CH=CH- | |
| 1131 | Benzoxazol-2-yl | -CH=CH- | |
| 1132 | Oxazol-2-yl | -CH=CH- | |
| 1133 | Oxazol-4-yl | -CH=CH- | |
| 1134 | Oxazol-5-yl | -CH=CH- | |
| 1135 | Thiazol-2-yl | -CH=CH- | |
| 1136 | Thiazol-4-yl | -CH=CH- | |
| 1137 | Thiazol-5-yl | -CH=CH- | |
| 1138 | Benzthiazol-2-yl | -CH=CH- | |
| 1139 | Benzisothiazol-3-yl | -CH=CH- | |
| 1140 | Isothiazol-3-yl | -CH=CH- | |
| 1141 | Isothiazol-4-yl | -CH=CH- | |
| 1142 | Isothiazol-5-yl | -CH=CH- | |
| 1143 | 1,2,3-Thiadiazol-4-yl | -CH=CH- | |

| Nr. | Z | Y | Daten |
|---|---|---|---|
| 1144 | 1,2,4-Thiadiazol-5-yl | -CH=CH- | |
| 1145 | 1,3,4-Thiadiazol-3-yl | -CH=CH- | |
| 1146 | 2-Furyl | $-CH_2CH_2-$ | |
| 1147 | 5-Nitro-2-furyl | $-CH_2CH_2-$ | |
| 1148 | 5-Chlor-2-furyl | $-CH_2CH_2-$ | |
| 1149 | 2-(2'-Furyl)-ethenyl | $-CH_2CH_2-$ | |
| 1150 | 2-(5'-Nitro-2'-furyl)ethenyl | $-CH_2CH_2-$ | |
| 1151 | 3-Furyl | $-CH_2CH_2-$ | |
| 1152 | 5-Nitro-3-furyl | $-CH_2CH_2-$ | |
| 1153 | 5-Chlor-3-furyl | $-CH_2CH_2-$ | |
| 1154 | 2-(3'-Furyl)ethenyl | $-CH_2CH_2-$ | |
| 1155 | 2-(5'-Nitro-3'-furyl)ethenyl | $-CH_2CH_2-$ | |
| 1156 | Benzofuran-2-yl | $-CH_2CH_2-$ | |
| 1157 | Benzofuran-3-yl | $-CH_2CH_2-$ | |
| 1158 | 2-Thienyl | $-CH_2CH_2-$ | |
| 1159 | 5-Nitro-2-thienyl | $-CH_2CH_2-$ | |
| 1160 | 5-Chlor-2-thienyl | $-CH_2CH_2-$ | |
| 1161 | 2-(2'-Thienyl)ethenyl | $-CH_2CH_2-$ | |
| 1162 | 2-(5'-Nitro-2'-thienyl)ethenyl | $-CH_2CH_2-$ | |
| 1163 | 3-Thienyl | $-CH_2CH_2-$ | |
| 1164 | 5-Nitro-3-thienyl | $-CH_2CH_2-$ | |
| 1165 | 5-Chlor-3-thienyl | $-CH_2CH_2-$ | |
| 1166 | 2-(3'-thienyl)ethenyl | $-CH_2CH_2-$ | |
| 1167 | 2-(5'-Nitro-3'-thienyl)ethenyl | $-CH_2CH_2-$ | |
| 1168 | Benzothien-2-yl | $-CH_2CH_2-$ | |

| Nr. | Z | Y | Daten |
|---|---|---|---|
| 1169 | Benzothien-3-yl | $-CH_2CH_2-$ | |
| 1170 | N-Methyl-pyrrol-2-yl | $-CH_2CH_2-$ | |
| 1171 | N-Methyl-pyrrol-3-yl | $-CH_2CH_2-$ | |
| 1172 | N-Methyl-pyrazol-3-yl | $-CH_2CH_2-$ | |
| 1173 | N-Methyl-pyrazol-4-yl | $-CH_2CH_2-$ | |
| 1174 | N-Methyl-pyrazol-5-yl | $-CH_2CH_2-$ | |
| 1175 | N-Methyl-imidazol-2-yl | $-CH_2CH_2-$ | |
| 1176 | N-Methyl-imidazol-4-yl | $-CH_2CH_2-$ | |
| 1177 | N-Methyl-imidazol-5-yl | $-CH_2CH_2-$ | |
| 1178 | 1-Methyl-1,2,3-triazol-4-yl | $-CH_2CH_2-$ | |
| 1179 | 1-Methyl-1,2,3-triazol-5-yl | $-CH_2CH_2-$ | |
| 1180 | 1-Methyl-1,2,4-triazol-3-yl | $-CH_2CH_2-$ | |
| 1181 | 1-Methyl-1,2,4-triazol-5-yl | $-CH_2CH_2-$ | |
| 1182 | 1-Methyl-tetrazol-5-yl | $-CH_2CH_2-$ | |
| 1183 | Isoxazol-3-yl | $-CH_2CH_2-$ | |
| 1184 | Isoxazol-4-yl | $-CH_2CH_2-$ | |
| 1185 | Isoxazol-5-yl | $-CH_2CH_2-$ | |
| 1186 | Benzisoxazol-3-yl | $-CH_2CH_2-$ | |
| 1187 | Benzoxazol-2-yl | $-CH_2CH_2-$ | |
| 1188 | Oxazol-2-yl | $-CH_2CH_2-$ | |
| 1189 | Oxazol-4-yl | $-CH_2CH_2-$ | |
| 1190 | Oxazol-5-yl | $-CH_2CH_2-$ | |
| 1191 | Thiazol-2-yl | $-CH_2CH_2-$ | |
| 1192 | Thiazol-4-yl | $-CH_2CH_2-$ | |
| 1193 | Thiazol-5-yl | $-CH_2CH_2-$ | |

| Nr. | Z | Y | Daten |
|---|---|---|---|
| 1194 | Benzthiazol-2-yl | $-CH_2CH_2-$ | |
| 1195 | Benzisothiazol-3-yl | $-CH_2CH_2-$ | |
| 1196 | Isothiazol-3-yl | $-CH_2CH_2-$ | |
| 1197 | Isothiazol-4-yl | $-CH_2CH_2-$ | |
| 1198 | Isothiazol-5-yl | $-CH_2CH_2-$ | |
| 1199 | 1,2,3-Thiadiazol-4-yl | $-CH_2CH_2-$ | |
| 1200 | 1,2,4-Thiadiazol-5-yl | $-CH_2CH_2-$ | |
| 1201 | 1,3,4-Thiadiazol-3-yl | $-CH_2CH_2-$ | |
| 1202 | 2-Furyl | $-CH_2O-$ | |
| 1203 | 5-Nitro-2-furyl | $-CH_2O-$ | |
| 1204 | 5-Chlor-2-furyl | $-CH_2O-$ | |
| 1205 | 2-(2'-Furyl)-ethenyl | $-CH_2O-$ | |
| 1206 | 2-(5'-Nitro-2'-furyl)ethenyl | $-CH_2O-$ | |
| 1207 | 3-Furyl | $-CH_2O-$ | |
| 1208 | 5-Nitro-3-furyl | $-CH_2O-$ | |
| 1209 | 5-Chlor-3-furyl | $-CH_2O-$ | |
| 1210 | 2-(3'-Furyl)ethenyl | $-CH_2O-$ | |
| 1211 | 2-(5'-Nitro-3'-furanyl)ethenyl | $-CH_2O-$ | |
| 1212 | Benzofuran-2-yl | $-CH_2O-$ | |
| 1213 | Benzofuran-3-yl | $-CH_2O-$ | |
| 1214 | 2-Thienyl | $-CH_2O-$ | |
| 1215 | 5-Nitro-2-thienyl | $-CH_2O-$ | |
| 1216 | 5-Chlor-2-thienyl | $-CH_2O-$ | |
| 1217 | 2-(2'-Thienyl)ethenyl | $-CH_2O-$ | |
| 1218 | 2-(5'-Nitro-2'-thienyl)ethenyl | $-CH_2O-$ | |

| Nr. | Z | Y | Daten |
|---|---|---|---|
| 1219 | 3-Thienyl | $-CH_2O-$ | |
| 1220 | 5-Nitro-3-thienyl | $-CH_2O-$ | |
| 1221 | 5-Chlor-3-thienyl | $-CH_2O-$ | |
| 1222 | 2-(3'-thienyl)ethenyl | $-CH_2O-$ | |
| 1223 | 2-(5'-Nitro-3'-thienyl)ethenyl | $-CH_2O-$ | |
| 1224 | Benzothien-2-yl | $-CH_2O-$ | |
| 1225 | Benzothien-3-yl | $-CH_2O-$ | |
| 1226 | N-Methyl-pyrrol-2-yl | $-CH_2O-$ | |
| 1227 | N-Methyl-pyrrol-3-yl | $-CH_2O-$ | |
| 1228 | N-Methyl-pyrazol-3-yl | $-CH_2O-$ | |
| 1229 | N-Methyl-pyrazol-4-yl | $-CH_2O-$ | |
| 1230 | N-Methyl-pyrazol-5-yl | $-CH_2O-$ | |
| 1231 | N-Methyl-imidazol-2-yl | $-CH_2O-$ | |
| 1232 | N-Methyl-imidazol-4-yl | $-CH_2O-$ | |
| 1233 | N-Methyl-imidazol-5-yl | $-CH_2O-$ | |
| 1234 | 1-Methyl-1,2,3-triazol-4-yl | $-CH_2O-$ | |
| 1235 | 1-Methyl-1,2,3-triazol-5-yl | $-CH_2O-$ | |
| 1236 | 1-Methyl-1,2,4-triazol-3-yl | $-CH_2O-$ | |
| 1237 | 1-Methyl-1,2,4-triazol-5-yl | $-CH_2O-$ | |
| 1238 | 1-Methyl-tetrazol-5-yl | $-CH_2O-$ | |
| 1239 | Isoxazol-3-yl | $-CH_2O-$ | |
| 1240 | Isoxazol-4-yl | $-CH_2O-$ | |
| 1241 | Isoxazol-5-yl | $-CH_2O-$ | |
| 1242 | Benzisoxazol-3-yl | $-CH_2O-$ | |
| 1243 | Benzoxazol-2-yl | $-CH_2O-$ | |

| Nr. | Z | Y | Daten |
|---|---|---|---|
| 1244 | Oxazol-2-yl | $-CH_2O-$ | |
| 1245 | Oxazol-4-yl | $-CH_2O-$ | |
| 1246 | Oxazol-5-yl | $-CH_2O-$ | |
| 1247 | Thiazol-2-yl | $-CH_2O-$ | |
| 1248 | Thiazol-4-yl | $-CH_2O-$ | |
| 1249 | Thiazol-5-yl | $-CH_2O-$ | |
| 1250 | Benzthiazol-2-yl | $-CH_2O-$ | |
| 1251 | Benzisothiazol-3-yl | $-CH_2O-$ | |
| 1252 | Isothiazol-3-yl | $-CH_2O-$ | |
| 1253 | Isothiazol-4-yl | $-CH_2O-$ | |
| 1254 | Isothiazol-5-yl | $-CH_2O-$ | |
| 1255 | 1,2,3-Thiadiazol-4-yl | $-CH_2O-$ | |
| 1256 | 1,2,4-Thiadiazol-5-yl | $-CH_2O-$ | |
| 1257 | 1,3,4-Thiadiazol-3-yl | $-CH_2O-$ | |
| 1258 | 2-Furyl | -O- | |
| 1259 | 5-Nitro-2-furyl | -O- | |
| 1260 | 5-Chlor-2-furyl | -O- | |
| 1261 | 3-Furyl | -O- | |
| 1262 | 5-Nitro-3-furyl | -O- | |
| 1263 | 5-Chlor-3-furyl | -O- | |
| 1264 | Benzofuran-2-yl | -O- | |
| 1265 | Benzofuran-3-yl | -O- | |
| 1266 | 2-Thienyl | -O- | |
| 1267 | 5-Nitro-2-thienyl | -O- | |
| 1268 | 5-Chlor-2-thienyl | -O- | |

| Nr. | Z | Y | Daten |
|---|---|---|---|
| 1269 | 3-Thienyl | -O- | |
| 1270 | 5-Nitro-3-thienyl | -O- | |
| 1271 | 5-Chlor-3-thienyl | -O- | |
| 1272 | Benzothien-2-yl | -O- | |
| 1273 | Benzothien-3-yl | -O- | |
| 1274 | N-Methyl-pyrrol-2-yl | -O- | |
| 1275 | N-Methyl-pyrrol-3-yl | -O- | |
| 1276 | N-Methyl-pyrazol-3-yl | -O- | |
| 1277 | N-Methyl-pyrazol-4-yl | -O- | |
| 1278 | N-Methyl-pyrazol-5-yl | -O- | |
| 1279 | N-Methyl-imidazol-2-yl | -O- | |
| 1280 | N-Methyl-imidazol-4-yl | -O- | |
| 1281 | N-Methyl-imidazol-5-yl | -O- | |
| 1282 | 1-Methyl-1,2,3-triazol-4-yl | -O- | |
| 1283 | 1-Methyl-1,2,3-triazol-5-yl | -O- | |
| 1284 | 1-Methyl-1,2,4-triazol-3-yl | -O- | |
| 1285 | 1-Methyl-1,2,4-triazol-5-yl | -O- | |
| 1286 | 1-Methyl-tetrazol-5-yl | -O- | |
| 1287 | Isoxazol-3-yl | -O- | |
| 1288 | Isoxazol-4-yl | -O- | |
| 1289 | Isoxazol-5-yl | -O- | |
| 1290 | Benzisoxazol-3-yl | -O- | |
| 1291 | Benzoxazol-2-yl | -O- | |
| 1292 | Oxazol-2-yl | -O- | |
| 1293 | Oxazol-4-yl | -O- | |

| Nr. | Z | Y | Daten |
|---|---|---|---|
| 1294 | Oxazol-5-yl | -O- | |
| 1295 | Thiazol-2-yl | -O- | |
| 1296 | Thiazol-4-yl | -O- | |
| 1297 | Thiazol-5-yl | -O- | |
| 1298 | Benzthiazol-2-yl | -O- | |
| 1299 | Benzisothiazol-3-yl | -O- | |
| 1300 | Isothiazol-3-yl | -O- | |
| 1301 | Isothiazol-4-yl | -O- | |
| 1302 | Isothiazol-5-yl | -O- | |
| 1303 | 1,3,-Thiazolo[4,5-b]pyridin-2-yl | -O- | |
| 1304 | 2-Furyl | -S- | |
| 1305 | 5-Nitro-2-furyl | -S- | |
| 1306 | 5-Chlor-2-furyl | -S- | |
| 1307 | 3-Furyl | -S- | |
| 1308 | 5-Nitro-3-furyl | -S- | |
| 1309 | 5-Chlor-3-furyl | -S- | |
| 1310 | Benzofuran-2-yl | -S- | |
| 1311 | Benzofuran-3-yl | -S- | |
| 1312 | 2-Thienyl | -S- | |
| 1313 | 5-Nitro-2-thienyl | -S- | |
| 1314 | 5-Chlor-2-thienyl | -S- | |
| 1315 | 3-Thienyl | -S- | |
| 1316 | 5-Nitro-3-thienyl | -S- | |
| 1317 | 5-Chlor-3-thienyl | -S- | |
| 1318 | Benzothien-2-yl | -S- | |

| Nr. | Z | Y | Daten |
|---|---|---|---|
| 1319 | Benzothien-3-yl | -S- | |
| 1320 | N-Methyl-pyrrol-2-yl | -S- | |
| 1321 | N-Methyl-pyrrol-3-yl | -S- | |
| 1322 | N-Methyl-pyrazol-3-yl | -S- | |
| 1323 | N-Methyl-pyrazol-4-yl | -S- | |
| 1324 | N-Methyl-pyrazol-5-yl | -S- | |
| 1325 | N-Methyl-imidazol-2-yl | -S- | |
| 1326 | N-Methyl-imidazol-4-yl | -S- | |
| 1327 | N-Methyl-imidazol-5-yl | -S- | |
| 1328 | 1-Methyl-1,2,3-triazol-4-yl | -S- | |
| 1329 | 1-Methyl-1,2,3-triazol-5-yl | -S- | |
| 1330 | 1-Methyl-1,2,4-triazol-3-yl | -S- | |
| 1331 | 1-Methyl-1,2,4-triazol-5-yl | -S- | |
| 1332 | 1-Methyl-tetrazol-5-yl | -S- | |
| 1333 | Isoxazol-3-yl | -S- | |
| 1334 | Isoxazol-4-yl | -S- | |
| 1335 | Isoxazol-5-yl | -S- | |
| 1336 | Benzisoxazol-3-yl | -S- | |
| 1337 | Benzoxazol-2-yl | -S- | |
| 1338 | Oxazol-2-yl | -S- | |
| 1339 | Oxazol-4-yl | -S- | |
| 1340 | Oxazol-5-yl | -S- | |
| 1341 | Thiazol-2-yl | -S- | |
| 1342 | Oxazol-4-yl | -S- | |
| 1343 | Oxazol-5-yl | -S- | |

| Nr. | Z | Y | Daten |
|---|---|---|---|
| 1344 | Thiazol-2-yl | -S- | |
| 1345 | Thiazol-4-yl | -S- | |
| 1346 | Thiazol-5-yl | -S- | |
| 1347 | Benzthiazol-2-yl | -S- | |
| 1348 | Benzisothiazol-3-yl | -S- | |
| 1349 | Isothiazol-3-yl | -S- | |
| 1350 | Isothiazol-4-yl | -S- | |
| 1351 | Isothiazol-5-yl | -S- | |
| 1352 | 1,3,-Thiazolo[4,5-b]pyridin-2-yl | -S- | |
| 1353 | 2-Furyl | $-OCH_2-$ | |
| 1354 | 5-Nitro-2-furyl | $-OCH_2-$ | |
| 1355 | 5-Chlor-2-furyl | $-OCH_2-$ | |
| 1356 | 3-Furyl | $-OCH_2-$ | |
| 1357 | 5-Nitro-3-furyl | $-OCH_2-$ | |
| 1358 | 5-Chlor-3-furyl | $-OCH_2-$ | |
| 1359 | Benzofuran-2-yl | $-OCH_2-$ | |
| 1360 | Benzofuran-3-yl | $-OCH_2-$ | |
| 1361 | 2-Thienyl | $-OCH_2-$ | |
| 1362 | 5-Nitro-2-thienyl | $-OCH_2-$ | |
| 1363 | 5-Chlor-2-thienyl | $-OCH_2-$ | |
| 1364 | 3-Thienyl | $-OCH_2-$ | |
| 1365 | 5-Nitro-3-thienyl | $-OCH_2-$ | |
| 1366 | 5-Chlor-3-thienyl | $-OCH_2-$ | |
| 1367 | Benzothien-2-yl | $-OCH_2-$ | |
| 1368 | Benzothien-3-yl | $-OCH_2-$ | |

| Nr. | Z | Y | Daten |
| --- | --- | --- | --- |
| 1369 | N-Methyl-pyrrol-2-yl | $-OCH_2-$ | |
| 1370 | N-Methyl-pyrrol-3-yl | $-OCH_2-$ | |
| 1371 | N-Methyl-pyrazol-3-yl | $-OCH_2-$ | |
| 1372 | N-Methyl-pyrazol-4-yl | $-OCH_2-$ | |
| 1373 | N-Methyl-pyrazol-5-yl | $-OCH_2-$ | |
| 1374 | N-Methyl-imidazol-2-yl | $-OCH_2-$ | |
| 1375 | N-Methyl-imidazol-4-yl | $-OCH_2-$ | |
| 1376 | N-Methyl-imidazol-5-yl | $-OCH_2-$ | |
| 1377 | 1-Methyl-1,2,3-triazol-4-yl | $-OCH_2-$ | |
| 1378 | 1-Methyl-1,2,3-triazol-5-yl | $-OCH_2-$ | |
| 1379 | 1-Methyl-1,2,4-triazol-3-yl | $-OCH_2-$ | |
| 1380 | 1-Methyl-1,2,4-triazol-5-yl | $-OCH_2-$ | |
| 1381 | 1-Methyl-tetrazol-5-yl | $-OCH_2-$ | |
| 1382 | Isoxazol-3-yl | $-OCH_2-$ | |
| 1383 | Isoxazol-4-yl | $-OCH_2-$ | |
| 1384 | Isoxazol-5-yl | $-OCH_2-$ | |
| 1385 | Benzisoxazol-3-yl | $-OCH_2-$ | |
| 1386 | Benzoxazol-2-yl | $-OCH_2-$ | |
| 1387 | Oxazol-2-yl | $-OCH_2-$ | |
| 1389 | Oxazol-4-yl | $-OCH_2-$ | |
| 1390 | Oxazol-5-yl | $-OCH_2-$ | |
| 1391 | Thiazol-2-yl | $-OCH_2-$ | |
| 1392 | Thiazol-4-yl | $-OCH_2-$ | |
| 1393 | Thiazol-5-yl | $-OCH_2-$ | |
| 1394 | Benzthiazol-2-yl | $-OCH_2-$ | |

| Nr. | Z | Y | Daten |
|---|---|---|---|
| 1395 | Benzisothiazol-3-yl | $-OCH_2-$ | |
| 1396 | Isothiazol-3-yl | $-OCH_2-$ | |
| 1397 | Isothiazol-4-yl | $-OCH_2-$ | |
| 1398 | Isothiazol-5-yl | $-OCH_2-$ | |
| 1399 | 2-Furyl | $-SCH_2-$ | |
| 1400 | 5-Nitro-2-furyl | $-SCH_2-$ | |
| 1401 | 5-Chlor-2-furyl | $-SCH_2-$ | |
| 1402 | 3-Furyl | $-SCH_2-$ | |
| 1403 | 5-Nitro-3-furyl | $-SCH_2-$ | |
| 1404 | 5-Chlor-3-furyl | $-SCH_2-$ | |
| 1405 | Benzofuran-2-yl | $-SCH_2-$ | |
| 1406 | Benzofuran-3-yl | $-SCH_2-$ | |
| 1407 | 2-Thienyl | $-SCH_2-$ | |
| 1408 | 5-Nitro-2-thienyl | $-SCH_2-$ | |
| 1409 | 5-Chlor-2-thienyl | $-SCH_2-$ | |
| 1410 | 3-Thienyl | $-SCH_2-$ | |
| 1411 | 5-Nitro-3-thienyl | $-SCH_2-$ | |
| 1412 | 5-Chlor-3-thienyl | $-SCH_2-$ | |
| 1413 | Benzothien-2-yl | $-SCH_2-$ | |
| 1414 | Benzothien-3-yl | $-SCH_2-$ | |
| 1415 | N-Methyl-pyrrol-2-yl | $-SCH_2-$ | |
| 1416 | N-Methyl-pyrrol-3-yl | $-SCH_2-$ | |
| 1417 | N-Methyl-pyrazol-3-yl | $-SCH_2-$ | |
| 1418 | N-Methyl-pyrazol-4-yl | $-SCH_2-$ | |
| 1419 | N-Methyl-pyrazol-5-yl | $-SCH_2-$ | |

| Nr. | Z | Y | Daten |
|---|---|---|---|
| 1420 | N-Methyl-imidazol-2-yl | $-SCH_2-$ | |
| 1421 | N-Methyl-imidazol-4-yl | $-SCH_2-$ | |
| 1422 | N-Methyl-imidazol-5-yl | $-SCH_2-$ | |
| 1423 | 1-Methyl-1,2,3-triazol-4-yl | $-SCH_2-$ | |
| 1424 | 1-Methyl-1,2,3-triazol-5-yl | $-SCH_2-$ | |
| 1425 | 1-Methyl-1,2,4-triazol-3-yl | $-SCH_2-$ | |
| 1426 | 1-Methyl-1,2,4-triazol-5-yl | $-SCH_2-$ | |
| 1427 | 1-Methyl-tetrazol-5-yl | $-SCH_2-$ | |
| 1428 | Isoxazol-3-yl | $-SCH_2-$ | |
| 1429 | Isoxazol-4-yl | $-SCH_2-$ | |
| 1430 | Isoxazol-5-yl | $-SCH_2-$ | |
| 1431 | Benzisoxazol-3-yl | $-SCH_2-$ | |
| 1432 | Benzoxazol-2-yl | $-SCH_2-$ | |
| 1433 | Oxazol-2-yl | $-SCH_2-$ | |
| 1434 | Oxazol-4-yl | $-SCH_2-$ | |
| 1435 | Oxazol-5-yl | $-SCH_2-$ | |
| 1436 | Thiazol-2-yl | $-SCH_2-$ | |
| 1437 | Thiazol-4-yl | $-SCH_2-$ | |
| 1438 | Thiazol-5-yl | $-SCH_2-$ | |
| 1439 | Benzthiazol-2-yl | $-SCH_2-$ | Öl; $^1H$-NMR ($CDCl_3$): $\delta$ = 3.78(3H),4.58(2H), 5.83(1H),6.63(1H),7.1-7.9(8H) |
| 1440 | Benzisothiazol-3-yl | $-SCH_2-$ | |
| 1441 | Isothiazol-3-yl | $-SCH_2-$ | |
| 1442 | Isothiazol-4-yl | $-SCH_2-$ | |
| 1443 | Isothiazol-5-yl | $-SCH_2-$ | |
| 1444 | 1,2,3-Thiadiazol-4-yl | $-SCH_2-$ | |

| Nr. | Z | Y | Daten |
|---|---|---|---|
| 1445 | 1,2,4-Thiadiazol-5-yl | $-SCH_2-$ | |
| 1446 | 1,3,4-Thiadiazol-3-yl | $-SCH_2-$ | |
| 1447 | 2-Furyl | $-CO-OCH_2-$ | |
| 1448 | 5-Nitro-2-furyl | $-CO-OCH_2-$ | |
| 1449 | 5-Chlor-2-furyl | $-CO-OCH_2-$ | |
| 1450 | 2-(2'-Furyl)-ethenyl | $-CO-OCH_2-$ | |
| 1451 | 2-(5'-Nitro-2'-furyl)ethenyl | $-CO-OCH_2-$ | |
| 1452 | 3-Furyl | $-CO-OCH_2-$ | |
| 1453 | 5-Nitro-3-furyl | $-CO-OCH_2-$ | |
| 1454 | 5-Chlor-3-furyl | $-CO-OCH_2-$ | |
| 1455 | 2-(3'-Furyl)ethenyl | $-CO-OCH_2-$ | |
| 1456 | 2-(5'-Nitro-3'-furanyl)ethenyl | $-CO-OCH_2-$ | |
| 1457 | Benzofuran-2-yl | $-CO-OCH_2-$ | |
| 1458 | Benzofuran-3-yl | $-CO-OCH_2-$ | |
| 1459 | 2-Thienyl | $-CO-OCH_2-$ | |
| 1460 | 5-Nitro-2-thienyl | $-CO-OCH_2-$ | |
| 1461 | 5-Chlor-2-thienyl | $-CO-OCH_2-$ | |
| 1462 | 2-(2'-Thienyl)ethenyl | $-CO-OCH_2-$ | |
| 1463 | 2-(5'-Nitro-2'-thienyl)ethenyl | $-CO-OCH_2-$ | |
| 1464 | 3-Thienyl | $-CO-OCH_2-$ | |
| 1465 | 5-Nitro-3-thienyl | $-CO-OCH_2-$ | |
| 1466 | 5-Chlor-3-thienyl | $-CO-OCH_2-$ | |
| 1467 | 2-(3'-thienyl)ethenyl | $-CO-OCH_2-$ | |
| 1468 | 2-(5'-Nitro-3'-thienyl)ethenyl | $-CO-OCH_2-$ | |
| 1469 | Benzothien-2-yl | $-CO-OCH_2-$ | |

| Nr. | Z | Y | Daten |
|---|---|---|---|
| 1470 | Benzothien-3-yl | $-CO-OCH_2-$ | |
| 1471 | N-Methyl-pyrrol-2-yl | $-CO-OCH_2-$ | |
| 1472 | N-Methyl-pyrrol-3-yl | $-CO-OCH_2-$ | |
| 1473 | N-Methyl-pyrazol-3-yl | $-CO-OCH_2-$ | |
| 1474 | N-Methyl-pyrazol-4-yl | $-CO-OCH_2-$ | |
| 1475 | N-Methyl-pyrazol-5-yl | $-CO-OCH_2-$ | |
| 1476 | N-Methyl-imidazol-2-yl | $-CO-OCH_2-$ | |
| 1477 | N-Methyl-imidazol-4-yl | $-CO-OCH_2-$ | |
| 1478 | N-Methyl-imidazol-5-yl | $-CO-OCH_2-$ | |
| 1479 | 1-Methyl-1,2,3-triazol-4-yl | $-CO-OCH_2-$ | |
| 1480 | 1-Methyl-1,2,3-triazol-5-yl | $-CO-OCH_2-$ | |
| 1481 | 1-Methyl-1,2,4-triazol-3-yl | $-CO-OCH_2-$ | |
| 1482 | 1-Methyl-1,2,4-triazol-5-yl | $-CO-OCH_2-$ | |
| 1483 | 1-Methyl-tetrazol-5-yl | $-CO-OCH_2-$ | |
| 1484 | Isoxazol-3-yl | $-CO-OCH_2-$ | |
| 1485 | Isoxazol-4-yl | $-CO-OCH_2-$ | |
| 1486 | Isoxazol-5-yl | $-CO-OCH_2-$ | |
| 1487 | Benzisoxazol-3-yl | $-CO-OCH_2-$ | |
| 1488 | Benzoxazol-2-yl | $-CO-OCH_2-$ | |
| 1489 | Oxazol-2-yl | $-CO-OCH_2-$ | |
| 1490 | Oxazol-4-yl | $-CO-OCH_2-$ | |
| 1491 | Oxazol-5-yl | $-CO-OCH_2-$ | |
| 1492 | Thiazol-2-yl | $-CO-OCH_2-$ | |
| 1493 | Thiazol-4-yl | $-CO-OCH_2-$ | |
| 1494 | Thiazol-5-yl | $-CO-OCH_2-$ | |

| Nr. | Z | Y | Daten |
|---|---|---|---|
| 1495 | Benzthiazol-2-yl | $-CO-OCH_2-$ | |
| 1496 | Benzisothiazol-3-yl | $-CO-OCH_2-$ | |
| 1497 | Isothiazol-3-yl | $-CO-OCH_2-$ | |
| 1498 | Isothioazol-4-yl | $-CO-OCH_2-$ | |
| 1499 | Isothiazol-5-yl | $-CO-OCH_2-$ | |
| 1500 | 1,2,3-Thiadiazol-4-yl | $-CO-OCH_2-$ | |
| 1501 | 1,2,4-Thiadiazol-5-yl | $-CO-OCH_2-$ | |
| 1502 | 1,3,4-Thiadiazol-3-yl | $-CO-OCH_2-$ | |
| 1503 | 2-Furyl | -NH-CO-O- | |
| 1504 | 5-Nitro-2-furyl | -NH-CO-O- | |
| 1505 | 5-Chlor-2-furyl | -NH-CO-O- | |
| 1506 | 3-Furyl | -NH-CO-O- | |
| 1507 | 5-Nitro-3-furyl | -NH-CO-O- | |
| 1508 | 5-Chlor-3-furyl | -NH-CO-O- | |
| 1509 | Benzofuran-2-yl | -NH-CO-O- | |
| 1510 | Benzofuran-3-yl | -NH-CO-O- | |
| 1511 | 2-Thienyl | -NH-CO-O- | |
| 1512 | 5-Nitro-2-thienyl | -NH-CO-O- | |
| 1513 | 5-Chlor-2-thienyl | -NH-CO-O- | |
| 1514 | 3-Thienyl | -NH-CO-O- | |
| 1515 | 5-Nitro-3-thienyl | -NH-CO-O- | |
| 1516 | 5-Chlor-3-thienyl | -NH-CO-O- | |
| 1517 | Benzothien-2-yl | -NH-CO-O- | |
| 1518 | Benzothien-3-yl | -NH-CO-O- | |
| 1519 | N-Methyl-pyrrol-2-yl | -NH-CO-O- | |

| Nr. | Z | Y | Daten |
|---|---|---|---|
| 1520 | N-Methyl-pyrrol-3-yl | -NH-CO-O- | |
| 1521 | N-Methyl-pyrazol-3-yl | -NH-CO-O- | |
| 1522 | N-Methyl-pyrazol-4-yl | -NH-CO-O- | |
| 1523 | N-Methyl-pyrazol-5-yl | -NH-CO-O- | |
| 1524 | N-Methyl-imidazol-2-yl | -NH-CO-O- | |
| 1525 | N-Methyl-imidazol-4-yl | -NH-CO-O- | |
| 1526 | N-Methyl-imidazol-5-yl | -NH-CO-O- | |
| 1527 | 1-Methyl-1,2,3-triazol-4-yl | -NH-CO-O- | |
| 1528 | 1-Methyl-1,2,3-triazol-5-yl | -NH-CO-O- | |
| 1529 | 1-Methyl-1,2,4-triazol-3-yl | -NH-CO-O- | |
| 1530 | 1-Methyl-1,2,4-triazol-5-yl | -NH-CO-O- | |
| 1531 | 1-Methyl-tetrazol-5-yl | -NH-CO-O- | |
| 1532 | Isoxazol-3-yl | -NH-CO-O- | |
| 1533 | Isoxazol-4-yl | -NH-CO-O- | |
| 1534 | Isoxazol-5-yl | -NH-CO-O- | |
| 1535 | Benzisoxazol-3-yl | -NH-CO-O- | |
| 1536 | Benzoxazol-2-yl | -NH-CO-O- | |
| 1537 | Oxazol-2-yl | -NH-CO-O- | |
| 1538 | Oxazol-4-yl | -NH-CO-O- | |
| 1539 | Oxazol-5-yl | -NH-CO-O- | |
| 1540 | Thiazol-2-yl | -NH-CO-O- | |
| 1541 | Thiazol-4-yl | -NH-CO-O- | |
| 1542 | Thiazol-5-yl | -NH-CO-O- | |
| 1543 | Benzthiazol-2-yl | -NH-CO-O- | |
| 1544 | Benzisothiazol-3-yl | -NH-CO-O- | |

| Nr. | Z | Y | Daten |
|---|---|---|---|
| 1545 | Isothiazol-3-yl | -NH-CO-O- | |
| 1546 | Isothiazol-4-yl | -NH-CO-O- | |
| 1547 | Isothiazol-5-yl | -NH-CO-O- | |
| 1548 | 2-Furyl | -CO-O- | |
| 1549 | 5-Nitro-2-furyl | -CO-O- | |
| 1550 | 5-Chlor-2-furyl | -CO-O- | |
| 1551 | 2-(2'-Furyl)-ethenyl | -CO-O- | |
| 1552 | 2-(5'-Nitro-2'-furyl)ethenyl | -CO-O- | |
| 1553 | 3-Furyl | -CO-O- | |
| 1554 | 5-Nitro-3-furyl | -CO-O- | |
| 1555 | 5-Chlor-3-furyl | -CO-O- | |
| 1556 | 2-(3'-Furyl)ethenyl | -CO-O- | |
| 1557 | 2-(5'-Nitro-3'-furanyl)ethenyl | -CO-O- | |
| 1558 | Benzofuran-2-yl | -CO-O- | |
| 1559 | Benzofuran-3-yl | -CO-O- | |
| 1560 | 2-Thienyl | -CO-O- | |
| 1561 | 5-Nitro-2-thienyl | -CO-O- | |
| 1562 | 5-Chlor-2-thienyl | -CO-O- | |
| 1563 | 2-(2'-Thienyl)ethenyl | -CO-O- | |
| 1564 | 2-(5'-Nitro-2'-thienyl)ethenyl | -CO-O- | |
| 1565 | 3-Thienyl | -CO-O- | |
| 1566 | 5-Nitro-3-thienyl | -CO-O- | |
| 1567 | 5-Chlor-3-thienyl | -CO-O- | |
| 1568 | 2-(3'-thienyl)ethenyl | -CO-O- | |
| 1569 | 2-(5'-Nitro-3'-thienyl)ethenyl | -CO-O- | |

| Nr. | Z | Y | Daten |
|---|---|---|---|
| 1570 | Benzothien-2-yl | -CO-O- | |
| 1571 | Benzothien-3-yl | -CO-O- | |
| 1572 | N-Methyl-pyrrol-2-yl | -CO-O- | |
| 1573 | N-Methyl-pyrrol-3-yl | -CO-O- | |
| 1574 | N-Methyl-pyrazol-3-yl | -CO-O- | |
| 1575 | N-Methyl-pyrazol-4-yl | -CO-O- | |
| 1576 | N-Methyl-pyrazol-5-yl | -CO-O- | |
| 1577 | N-Methyl-imidazol-2-yl | -CO-O- | |
| 1578 | N-Methyl-imidazol-4-yl | -CO-O- | |
| 1579 | N-Methyl-imidazol-5-yl | -CO-O- | |
| 1580 | 1-Methyl-1,2,3-triazol-4-yl | -CO-O- | |
| 1581 | 1-Methyl-1,2,3-triazol-5-yl | -CO-O- | |
| 1582 | 1-Methyl-1,2,4-triazol-3-yl | -CO-O- | |
| 1583 | 1-Methyl-1,2,4-triazol-5-yl | -CO-O- | |
| 1584 | 1-Methyl-tetrazol-5-yl | -CO-O- | |
| 1585 | Isoxazol-3-yl | -CO-O- | |
| 1586 | Isoxazol-4-yl | -CO-O- | |
| 1587 | Isoxazol-5-yl | -CO-O- | |
| 1588 | Benzisoxazol-3-yl | -CO-O- | |
| 1589 | Benzoxazol-2-yl | -CO-O- | |
| 1590 | Oxazol-2-yl | -CO-O- | |
| 1591 | Oxazol-4-yl | -CO-O- | |
| 1592 | Oxazol-5-yl | -CO-O- | |
| 1593 | Thiazol-2-yl | -CO-O- | |
| 1594 | Thiazol-4-yl | -CO-O- | |

| Nr. | Z | Y | Daten |
|---|---|---|---|
| 1620 | N-Methyl-pyrrol-2-yl | -O-CO- | |
| 1621 | N-Methyl-pyrrol-3-yl | -O-CO- | |
| 1622 | N-Methyl-pyrazol-3-yl | -O-CO- | |
| 1623 | N-Methyl-pyrazol-4-yl | -O-CO- | |
| 1625 | N-Methyl-imidazol-2-yl | -O-CO- | |
| 1626 | N-Methyl-imidazol-4-yl | -O-CO- | |
| 1627 | N-Methyl-imidazol-5-yl | -O-CO- | |
| 1628 | 1-Methyl-1,2,3-triazol-4-yl | -O-CO- | |
| 1629 | 1-Methyl-1,2,3-triazol-5-yl | -O-CO- | |
| 1630 | 1-Methyl-1,2,4-triazol-3-yl | -O-CO- | |
| 1631 | 1-Methyl-1,2,4-triazol-5-yl | -O-CO- | |
| 1632 | 1-Methyl-tetrazol-5-yl | -O-CO- | |
| 1633 | Isoxazol-3-yl | -O-CO- | |
| 1634 | Isoxazol-4-yl | -O-CO- | |
| 1635 | Isoxazol-5-yl | -O-CO- | |
| 1636 | Benzoisoxazol-3-yl | -O-CO- | |
| 1637 | Benzoxazol-2-yl | -O-CO- | |
| 1638 | Oxazol-2-yl | -O-CO- | |
| 1639 | Oxazol-4-yl | -O-CO- | |
| 1640 | Oxazol-5-yl | -O-CO- | |
| 1641 | Thiazol-2-yl | -O-CO- | |
| 1642 | Thiazol-4-yl | -O-CO- | |
| 1643 | Thiazol-5-yl | -O-CO- | |
| 1644 | Benzthiazol-2-yl | -O-CO- | |

| Nr. | Z | Y | Daten |
|---|---|---|---|
| 1595 | Thiazol-5-yl | -CO-O- | |
| 1596 | Benzthiazol-2-yl | -CO-O- | |
| 1597 | Benzisothiazol-3-yl | -CO-O- | |
| 1598 | Isothiazol-3-yl | -CO-O- | |
| 1599 | Isothioazol-4-yl | -CO-O- | |
| 1600 | Isothiazol-5-yl | -CO-O- | |
| 1601 | 1,2,3-Isodiazol-4-yl | -CO-O- | |
| 1602 | 1,2,4-Thiadiazol-5-yl | -CO-O- | |
| 1603 | 1,3,4-Thiadiazol-3-yl | -CO-O- | |
| 1604 | 2-Furyl | -O-CO- | |
| 1605 | 5-Nitro-2-furyl | -O-CO- | |
| 1606 | 5-Chlor-2-furyl | -O-CO- | |
| 1607 | 3-Furyl | -O-CO- | |
| 1608 | 5-Nitro-3-furyl | -O-CO- | |
| 1609 | 5-Chlor-3-furyl | -O-CO- | |
| 1610 | Benzofuran-2-yl | -O-CO- | |
| 1611 | Benzofuran-3-yl | -O-CO- | |
| 1612 | 2-Thienyl | -O-CO- | |
| 1613 | 5-Nitro-2-thienyl | -O-CO- | |
| 1614 | 5-Chlor-2-thienyl | -O-CO- | |
| 1615 | 3-Thienyl | -O-CO- | |
| 1616 | 5-Nitro-3-thienyl | -O-CO- | |
| 1617 | 5-Chlor-3-thienyl | -O-CO- | |
| 1618 | Benzothien-2-yl | -O-CO- | |
| 1619 | Benzothien-3-yl | -O-CO- | |

| Nr. | Z | Y | Daten |
|---|---|---|---|
| 1645 | Benzisothiazol-3-yl | -O-CO- | |
| 1646 | Isothiazol-3-yl | -O-CO- | |
| 1647 | Isothiazol-4-yl | -O-CO- | |
| 1648 | Isothiazol-5-yl | -O-CO- | |
| 1649 | 1,2,3-Thiadiazol-4-yl | -O-CO- | |
| 1650 | 1,2,4-Thiadiazol-5-yl | -O-CO- | |
| 1651 | 1,3,4-Thiadiazol-3-yl | -O-CO- | |
| 1652 | 6-Chlor-benzothiazol-2-yl | $-SCH_2-$ | Öl; $^1H$-NMR ($CDCl_3$): $\delta$ = 3.78(3H), 4.53(2H), 5.84(1H), 6.62(1H), 7.1-7.8(7H) |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten :

Erysiphe graminis (echter Mehltau) in Getreide,
Erysiphe cichoracearum und Sphaerotheca fuliginea an Kürbisgewächsen,
Podosphaera leucotricha an Äpfeln
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 460 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 632 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der

Lösung in Wasser erhält man eine wäßrige Dispersion.

III. 20 Gew.-Teile der Verbindung Nr. 649 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 773 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 866 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 866 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung Nr. 460 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 632 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 649 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid ;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,

2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde α-Phenyl-acrylsäuremethylester (A) – bekannt aus C.A. Reg. No. 1865-29-8 – benutzt.

Anwendungsbeispiel 1

Wirksamkeit gegen Pyricularia oryzae (protektiv)

Blätter von in Töpfen gewachsenen Reiskeimlingen der Sorte "Bahia" wurden mit wäßrigen Emulsionen, die 80% Wirkstoff und 20% Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99% relativer Luftfeuchtigkeit aufgestellt. Nach 6 Tagen wurde das Ausmaß des Krankheitsbefalls ermittelt.

Das Ergebnis zeigt, daß die Wirkstoffe 460, 632, 649, 773 und 866 bei der Anwendung als 0,05%ige (Gew.%) Spritzbrühe eine bessere fungizide Wirkung zeigen (95%) als der bekannte Vergleichswirkstoff A (60%).

Anwendungsbeispiel 2

Wirksamkeit gegen Pyrenophora teres

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70% relativer Luftfeuchtigkeit für weitere 5 Tage kultiviert. Dann wurde das Ausmaß des Befalls ermittelt.

Das Ergebnis zeigt, daß die Wirkstoffe 649 und 1439 bei der Anwendung als 0,05%ige Spritzbrühe eine bessere fungizide Wirkung zeigen (90%) als der bekannte Vergleichswirkstoff A (0%).

Anwendungsbeispiel 3

Wirksamkeit gegen Weizenmehltau

Blätter von in Töpfen gewachsenen Weizenkeimlingen der Sorte "Kanzler" wurden mit wäßriger Spritzbrühe, die 80% Wirkstoff und 20% Emulgiermittel in der Trockensubstanz enthielt, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80% relativer Luftfeuchtigkeit aufgestellt. Nach 7 Tagen wurde das Ausmaß der Mehltauentwicklung ermittelt.

Das Ergebnis zeigt, daß die Wirkstoffe 632 und 649 bei der Anwendung als 0,025%ige Spritzbrühe eine bessere fungizide Wirkung zeigen (90%) als der bekannte Vergleichswirkstoff A (40%).

## Ansprüche

1. α-Aryl-acrylsäureester der allgemeinen Formel I

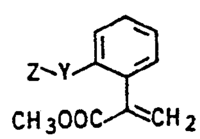

(I)

in der

Y gegebenenfalls substituiertes $C_1$-$C_4$-Alkylen, gegebenenfalls substituiertes $C_2$-$C_4$-Alkenylen, $C_2$-$C_4$-Alkinylen, O,$S(O)_m$ (m = 0, 1, 2), gegebenenfalls $C_1$-$C_4$-alkylsubstituiertes N, Oxycarbonyl, Carbonyloxy, $C_1$-$C_{10}$-

Oxycarbonylalkylen, $C_1$-$C_{10}$-Carbonyloxyalkylen, $C_2$-$C_{10}$-Oxyalkylenoxy, $C_1$-$C_{10}$-Oxyalkylen, $C_1$-$C_{10}$-Alkylenoxy, $C_1$-$C_{10}$-Thioalkylen, Azo, gegebenenfalls $C_1$-$C_4$-alkylsubstituiertes Carbonylamino, gegebenenfalls $C_1$-$C_4$-alkylsubstituiertes Aminocarbonyl, gegebenenfalls $C_1$-$C_4$-alkylsubstituiertes Aminocarbonyloxy
und

Z Wasserstoff, Halogen, $C_1$-$C_{18}$-Alkyl, $C_2$-$C_{18}$-Alkenyl, $C_3$-$C_8$-Cycloalkyl, $C_2$-$C_4$-Alkinyl, Aryl, Aryl-$C_1$-$C_{10}$-alkyl, Aryl-$C_2$-$C_{10}$-alkenyl, Aryloxy, Aryloxy-$C_1$-$C_{10}$-alkyl, $C_1$-$C_4$-Alkoxy-$C_1$-$C_{10}$-alkyl, Halogen-$C_1$-$C_{10}$-alkyl, Aryloxy-$C_1$-$C_4$-alkoxy, $C_1$-$C_4$-Alkoxycarbonyl, die gegebenenfalls substituiert sind mit Halogen, Cyano, Nitro, $C_1$-$C_4$-Alkyl, $C_2$-$C_4$-Alkenyl, $C_1$-$C_4$-Alkoxy, $C_1$-$C_4$-Halogenalkyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten gegebenenfalls einen aromatischen oder heteroaromatischen Ring bilden, der gegebenenfalls an eine Etheneinheit geknüpft ist, außer den Verbindungen, in denen Y O und Z H oder $CH_3$ und in denen Z-Y $CH_3OOC$ bedeuten.

2. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel I behandelt,

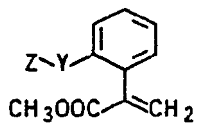

(I)

in der

Y gegebenenfalls substituiertes $C_1$-$C_4$-Alkylen, gegebenenfalls substituiertes $C_2$-$C_4$-Alkenylen, $C_2$-$C_4$-Alkinylen, O,S(O)$_m$ (m = 0-2), gegebenenfalls $C_1$-$C_4$-alkylsubstituiertes N, Oxycarbonyl, Carbonyloxy, $C_1$-$C_{10}$-Oxycarbonylalkylen, $C_1$-$C_{10}$-Carbonyloxyalkylen, $C_2$-$C_{10}$-Oxyalkylenoxy, $C_1$-$C_{10}$-Oxyalkylen, $C_1$-$C_{10}$-Alkylenoxy, $C_1$-$C_{10}$-Thioalkylen, Azo, gegebenenfalls $C_1$-$C_4$-alkyl-substituiertes Carbonylamino, gegebenenfalls $C_1$-$C_4$-alkyl-substituiertes Aminocarbonyl, gegebenenfalls $C_1$-$C_4$-alkyl-substituiertes Aminocarbonyloxy
und

Z Wasserstoff, Halogen, $C_1$-$C_{18}$-Alkyl, $C_2$-$C_{18}$-Alkenyl, $C_3$-$C_8$-Cycloalkyl, $C_2$-$C_4$-Alkinyl, Aryl, Aryl-$C_1$-$C_{10}$-alkyl, Aryl-$C_2$-$C_{10}$-alkenyl, Aryloxy, Aryloxy-$C_1$-$C_{10}$-alkyl, $C_1$-$C_4$-Alkoxy-$C_1$-$C_{10}$-alkyl, Halogen-$C_1$-$C_{10}$-alkyl, Aryloxy-$C_1$-$C_4$-alkoxy, $C_1$-$C_4$-Alkoxycarbonyl, die gegebenenfalls substituiert sind mit Halogen, Cyano, Nitro, $C_1$-$C_4$-Alkyl, $C_2$-$C_4$-Alkenyl, $C_1$-$C_4$-Alkoxy, $C_1$-$C_4$-Halogenalkyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten gegebenenfalls einen aromatischen oder heteroaromatischen Ring bilden der gegebenenfalls an eine Etheneinheit geknüpft ist, außer den Verbindungen, in denen Y O und Z H oder $CH_3$ und in denen Z-Y $CH_3OOC$ bedeuten.

3. Fungizid, enthaltend einen inerten Trägerstoff und eine fungizid wirksame Menge einer Verbindung der Formel I

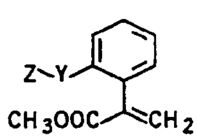

(I)

in der

Y gegebenenfalls substituiertes $C_1$-$C_4$-Alkylen, gegebenenfalls substituiertes $C_2$-$C_4$-Alkenylen, $C_2$-$C_4$-Alkinylen, O,S(O)m (m = 0-2), gegebenenfalls $C_1$-$C_4$-alkylsubstituiertes N, Oxycarbonyl, Carbonyloxy, $C_1$-$C_{10}$-Oxycarbonylalkylen, $C_1$-$C_{10}$-Carbonyloxyalkylen, $C_2$-$C_{10}$-Oxyalkylenoxy, $C_1$-$C_{10}$-Oxyalkylen, $C_1$-$C_{10}$-Alkylenoxy, $C_1$-$C_{10}$-Thioalkylen, Azo, gegebenenfalls $C_1$-$C_4$-alkylsubstituiertes Carbonylamino, gegebenenfalls $C_1$-$C_4$-alkylsubstituiertes Aminocarbonyl, gegebenenfalls $C_1$-$C_4$-alkylsubstituiertes Aminocarbonyloxy
und

Z Wasserstoff, Halogen, $C_1$-$C_{18}$-Alkyl, $C_2$-$C_{18}$-Alkenyl, $C_3$-$C_8$-Cycloalkyl, $C_2$-$C_4$-Alkinyl, Aryl, Aryl-$C_1$-$C_{10}$-al-

kyl, Aryl-$C_2$-$C_{10}$-alkenyl, Aryloxy, Aryloxy-$C_1$-$C_{10}$-alkyl, $C_1$-$C_4$-Alkoxy-$C_1$-$C_{10}$-alkyl, Halogen-$C_1$-$C_{10}$-alkyl, Aryloxy-$C_1$-$C_4$-alkoxy, $C_1$-$C_4$-Alkoxycarbonyl, die gegebenenfalls substituiert sind mit Halogen, Cyano, Nitro, $C_1$-$C_4$-Alkyl, $C_2$-$C_4$-Alkenyl, $C_1$-$C_4$-Alkoxy, $C_1$-$C_4$Halogenalkyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten gegebenenfalls einen aromatischen oder heteroaromatischen Ring bilden, der gebenenfalls an eine Ethenyleinheit geknüpft ist, außer den Verbindungen, in denen Y O und Z H oder $CH_3$ und in denen Z-Y $CH_3OOC$ bedeuten.

4. Verfahren zur Herstellung eines fungiziden Mittels, dadurch gekennzeichnet, daß man eine oder mehrere Verbindungen der Formel I gemäß Anspruch 1 mit einem festen oder flüssigen Trägerstoff sowie gegebenenfalls mit einem oder mehreren oberflächenaktiven Mitteln mischt.

5. Verbindung der Formel I gemäß Anspruch 1, in der Y O bedeutet und Z Benzyl bedeutet.

6. Verbindung der Formel I gemäß Anspruch 1, in der Y $-CO-OCH_2$ bedeutet und Z Phenyl bedeutet.

7. Verbindung der Formel I gemäß Anspruch 1, in der Y $-CO-OCH_2$ bedeutet und Z Styryl bedeutet.

8. Verbindung der Formel I gemäß Anspruch 1, in der Y $-CO-OCH_2$ bedeutet und Z 1-Methylcyclopropyl bedeutet.

9. 2-(2'-Brommethyl)phenyl-acrylsäuremethylester

## Claims

1. α-Arylacrylates of the general formula I

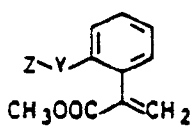

(I)

where

Y is substituted or unsubstituted $C_1$-$C_4$-alkylene, substituted or unsubstituted $C_2$-$C_4$-alkenylene, $C_2$-$C_4$-alkylene, O,$S(O)_m$ (m = 0, 1 or 2), unsubstituted or $C_1$-$C_4$-alkyl-substituted N, oxycarbonyl, carbonyloxy, $C_1$-$C_{10}$-oxycarbonylalkylene, $C_1$-$C_{10}$-carbonyloxyalkylene, $C_2$-$C_{10}$-oxyalkyleneoxy, $C_1$-$C_{10}$-oxyalkylene, $C_1$-$C_{10}$-alkyleneoxy, $C_1$-$C_{10}$-thioalkylene, azo, unsubstituted or $C_1$-$C_4$-alkyl-substituted carbonylamino, unsubstituted or $C_1$-$C_4$-alkyl-substituted aminocarbonyl, or unsubstituted or $C_1$-$C_4$-alkyl-substituted aminocarbonyloxy,

and

Z is hydrogen, halogen, $C_1$-$C_{18}$-alkyl, $C_2$-$C_{18}$-alkenyl, $C_3$-$C_8$-cycloalkyl, $C_2$-$C_4$-alkynyl, aryl, aryl-$C_1$-$C_{10}$-alkyl, aryl-$C_2$-$C_{10}$-alkenyl, aryloxy, aryloxy-$C_1$-$C_{10}$-alkyl, $C_1$-$C_4$-alkoxy-$C_1$-$C_{10}$-alkyl, halo-$C_1$-$C_{10}$-alkyl, aryloxy-$C_1$-$C_4$-alkoxy, $C_1$-$C_4$-alkoxycarbonyl, which are unsubstituted or substituted by halogen, cyano, nitro, $C_1$-$C_4$-alkyl, $C_2$-$C_4$-alkenyl, $C_1$-$C_4$-alkoxy, $C_1$-$C_4$-haloalkyl, $C_2$-$C_4$-haloalkenyl, $C_1$-$C_4$-alkoxycarbonyl, unsubstituted or substituted phenyl or unsubstituted or substituted phenoxy, or is a C-bonded, unsubstituted or substituted 5-membered heterocyclic structure which contains one to four identical or different heteroatoms, specifically nitrogen, oxygen or sulfur, in which structure two adjacent substituents may form an aromatic or heteroaromatic ring which may be bonded to an ethylene unit, with the exception of the compounds in which Y is O and Z is H or $CH_3$, and in which Z-Y is $CH_3OOC$.

2. A method for controlling fungi, wherein the fungi, or the materials, plants, seed or the soil threatened by fungus attack are treated with a fungicidally effective amount of a compound of the formula I

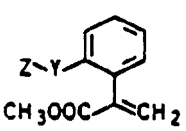

(I)

where

Y is substituted or unsubstituted $C_1$-$C_4$-alkylene, substituted or unsubstituted $C_2$-$C_4$-alkenylene, $C_2$-$C_4$-al-

kynylene, O,S(O)$_m$ (m = 0, 1 or 2), unsubstituted or $C_1$-$C_4$-alkyl-substituted N, oxycarbonyl, carbonyloxy, $C_1$-$C_{10}$-oxycarbonylalkylene, $C_1$-$C_{10}$-carbonyloxyalkylene, $C_2$-$C_{10}$-oxyalkyleneoxy, $C_1$-$C_{10}$-oxyalkylene, $C_1$-$C_{10}$-alkyleneoxy, $C_1$-$C_{10}$-thioalkylene, azo, unsubstituted or $C_1$-$C_4$-alkyl-substituted carbonylamino, unsubstituted or $C_1$-$C_4$-alkyl-substituted aminocarbonyl, or unsubstituted or $C_1$-$C_4$-alkyl-substituted aminocarbonyloxy, and

Z is hydrogen, halogen, $C_1$-$C_{18}$-alkyl, $C_2$-$C_{18}$-alkenyl, $C_3$-$C_8$-cycloalkyl, $C_2$-$C_4$-alkynyl, aryl, aryl-$C_1$-$C_{10}$-alkyl, aryl-$C_2$-$C_{10}$-alkenyl, aryloxy, aryloxy-$C_1$-$C_{10}$-alkyl, $C_1$-$C_4$-alkoxy-$C_1$-$C_{10}$-alkyl, halo-$C_1$-$C_{10}$-alkyl, aryloxy-$C_1$-$C_4$-alkoxy, $C_1$-$C_4$-alkoxycarbonyl, which are unsubstituted or substituted by halogen, cyano, nitro, $C_1$-$C_4$-alkyl, $C_2$-$C_4$-alkenyl, $C_1$-$C_4$-alkoxy, $C_1$-$C_4$-haloalkyl, $C_2$-$C_4$-haloalkenyl, $C_1$-$C_4$-alkoxycarbonyl, unsubstituted or substituted phenyl or unsubstituted or substituted phenoxy, or is a C-bonded, unsubstituted or substituted 5-membered heterocyclic structure which contains one to four identical or different heteroatoms, specifically nitrogen, oxygen or sulfur, in which structure two adjacent substituents may form an aromatic or heteroaromatic ring which may be bonded to an ethylene unit, with the exception of compounds in which Y is O and Z is H or $CH_3$, and in which Z-Y is $CH_3OOC$.

3. A fungicide containing an inert carrier and a fungicidally effective amount of a compound of the formula I

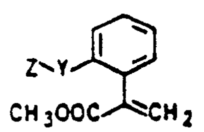

(I)

where

Y is substituted or unsubstituted $C_1$-$C_4$-alkylene, substituted or unsubstituted $C_2$-$C_4$-alkenylene, $C_2$-$C_4$-alkynylene, O,S(O)$_m$ (m = 0, 1 or 2), unsubstituted or $C_1$-$C_4$-alkyl-substituted N, oxycarbonyl, carbonyloxy, $C_1$-$C_{10}$-oxycarbonylalkylene, $C_1$-$C_{10}$-carbonyloxyalkylene, $C_2$-$C_{10}$-oxyalkyleneoxy, $C_1$-$C_{10}$-oxyalkylene, $C_1$-$C_{10}$-alkyleneoxy, $C_1$-$C_{10}$-thioalkylene, azo, unsubstituted or $C_1$-$C_4$-alkyl-substituted carbonylamino, unsubstituted or $C_1$-$C_4$-alkyl-substituted aminocarbonyl, or unsubstituted or $C_1$-$C_4$-alkyl-substituted aminocarbonyloxy, and

Z is hydrogen, halogen, $C_1$-$C_{18}$-alkyl, $C_2$-$C_{18}$-alkenyl, $C_3$-$C_8$-cycloalkyl, $C_2$-$C_4$-alkynyl, aryl, aryl-$C_1$-$C_{10}$-alkyl, aryl-$C_2$-$C_{10}$-alkenyl, aryloxy, aryloxy-$C_1$-$C_{10}$-alkyl, $C_1$-$C_4$-alkoxy-$C_1$-$C_{10}$-alkyl, halo-$C_1$-$C_{10}$-alkyl, aryloxy-$C_1$-$C_4$-alkoxy, $C_1$-$C_4$-alkoxycarbonyl, which are unsubstituted or substituted by halogen, cyano, nitro, $C_1$-$C_4$-alkyl, $C_2$-$C_4$-alkenyl, $C_1$-$C_4$-alkoxy, $C_1$-$C_4$-haloalkyl, $C_2$-$C_4$-haloalkenyl, $C_1$-$C_4$-alkoxycarbonyl, unsubstituted or substituted phenyl or unsubstituted or substituted phenoxy, or is a C-bonded, unsubstituted or substituted 5-membered heterocyclic structure which contains one to four identical or different heteroatoms, specifically nitrogen, oxygen or sulfur, in which structure two adjacent substituents may form an aromatic or heteroaromatic ring which may be bonded to an ethylene unit, with the exception of compounds in which Y is O and Z is H or $CH_3$, and in which Z-Y is $CH_3OOC$.

4. A process for the preparation of a fungicide, wherein one or more compounds of the formula I as claimed in claim 1 are mixed with a solid or liquid carrier and, if required, with one or more surface-active agents.

5. A compound of the formula I as claimed in claim 1, where Y is oxygen and Z is benzyl.

6. A compound of the formula I as claimed in claim 1, where Y is $-CO-OCH_2$ and Z is phenyl.

7. A compound of the formula I as claimed in claim 1, where Y is $-CO-OCH_2$ and Z is styryl.

8. A compound of the formula I as claimed in claim 1, where Y is $-CO-OCH_2$ and Z is 1-methylcyclopropyl.

9. Methyl 2-(2'-bromomethyl)-phenylacrylate.

## Revendications

1. Esters d'acide α-aryl-acrylique de formule générale I

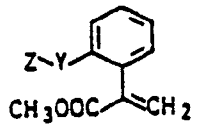

(I)

dans laquelle

Y représente alkylène en $C_1$-$C_4$ éventuellement substitué, alcénylène en $C_2$-$C_4$ éventuellement substitué, alcynylène en $C_2$-$C_4$, O,S(O)$_m$ (m = 0, 1, 2), N éventuellement substitué par alkyle en $C_1$-$C_4$, oxycarbonyle, carbonyloxy, oxycarbonylalkylène en $C_1$-$C_{10}$, carbonyloxyalkylène en $C_1$-$C_{10}$, oxyalkylènoxy en $C_2$-$C_{10}$, oxyalkylène en $C_1$-$C_{10}$, alkylènoxy en $C_1$-$C_{10}$, thioalkylène en $C_1$-$C_{10}$, azo, carbonylamino éventuellement substitué par alkyle en $C_1$-$C_4$, aminocarbonyle éventuellement substitué par alkyle en $C_1$-$C_4$, aminocarbonyloxy éventuellement substitué par alkyle en $C_1$-$C_4$

et

Z, hydrogène, halogène, alkyle en $C_1$-$C_{18}$, alcényle en $C_2$-$C_{18}$, cycloalkyle en $C_3$-$C_8$, alcynyle en $C_2$-$C_4$, aryle, aryl-alkyle en $C_1$-$C_{10}$, aryl-alcényle en $C_2$-$C_{10}$, aryloxy, aryloxy-alkyle en $C_1$-$C_{10}$, alcoxy en $C_1$-$C_4$-alkyle en $C_1$-$C_{10}$, halogène-alkyle en $C_1$-$C_{10}$, aryloxy-alcoxy en $C_1$-$C_4$, alcoxy en $C_1$-$C_4$-carbonyle, ces restes étant éventuellement substitués par halogène, cyano, nitro, alkyle en $C_1$-$C_4$, alcényle en $C_2$-$C_4$, alcoxy en $C_1$-$C_4$, halogènalkyle en $C_1$-$C_4$, halogènalcényle en $C_2$-$C_4$, alcoxy en $C_1$-$C_4$-carbonyle ; phényle éventuellement substitué, phénoxy éventuellement substitué, ou un hétérocycle à cinq chaînons éventuellement substitué, à liaison C, qui contient un à quatre hétéro-atomes identiques ou différents, à savoir azote, oxygène ou soufre, dans lequel deux substituants contigus forment éventuellement un cycle aromatique ou hétéroaromatique, lié éventuellement à une unité éthylène, exceptés les composés dans lesquels Y représente O et Z représente H ou $CH_3$ et dans lesquels Z-Y représente $CH_3OOC$.

2. Procédé de lutte contre les champignons, caractérisé par le fait que l'on traite les champignons ou les matériaux, plantes, semences ou le sol avec une quantité efficace du point de vue fongicide d'un composé de formule I

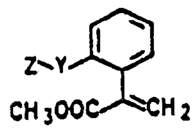

(I)

dans laquelle

Y représente alkylène en $C_1$-$C_4$ éventuellement substitué, alcénylène en $C_2$-$C_4$ éventuellement substitué, alcynylène en $C_2$-$C_4$, O,S(O)$_m$ (m = 0, 1, 2), N éventuellement substitué par alkyle en $C_1$-$C_4$, oxycarbonyle, carbonyloxy, oxycarbonylalkylène en $C_1$-$C_{10}$, carbonyloxyalkylène en $C_1$-$C_{10}$, oxyalkylènoxy en $C_2$-$C_{10}$, oxyalkylène en $C_1$-$C_{10}$, alkylènoxy en $C_1$-$C_{10}$, thioalkylène en $C_1$-$C_{10}$, azo, carbonylamino éventuellement substitué par alkyle en $C_1$-$C_4$, aminocarbonyle éventuellement substitué par alkyle en $C_1$-$C_4$, aminocarbonyloxy éventuellement substitué par alkyle en $C_1$-$C_4$

et

Z, hydrogène, halogène, alkyle en $C_1$-$C_{18}$, alcényle en $C_2$-$C_{18}$, cycloalkyle en $C_3$-$C_8$, alcynyle en $C_2$-$C_4$, aryle, aryl-alkyle en $C_1$-$C_{10}$, aryl-alcényle en $C_2$-$C_{10}$, aryloxy, aryloxy-alkyle en $C_1$-$C_{10}$, alcoxy en $C_1$-$C_4$-alkyle en $C_1$-$C_{10}$, halogène-alkyle en $C_1$-$C_{10}$, aryloxy-alcoxy en $C_1$-$C_4$, alcoxy en $C_1$-$C_4$-carbonyle, ces restes étant éventuellement substitués par halogène, cyano, nitro, alkyle en $C_1$-$C_4$, alcényle en $C_2$-$C_4$, alcoxy en $C_1$-$C_4$, halogènalkyle en $C_1$-$C_4$, halogènalcényle en $C_2$-$C_4$, alcoxy en $C_1$-$C_4$-carbonyle ; phényle éventuellement substitué, phénoxy éventuellement substitué, ou un hétérocycle à cinq chaînons éventuellement substitué, à liaison C, qui contient un à quatre hétéro-atomes identiques ou différents, à savoir azote, oxygène ou soufre, dans lequel deux substituants contigus forment éventuellement un cycle aromatique ou hétéroaromatique, lié éventuellement à une unité éthylène, exceptés les composés dans lesquels Y représente O et Z représente H ou $CH_3$ et dans lesquels Z-Y représente $CH_3OOC$.

3. Fongicide, contenant un véhicule inerte et une quantité efficace du point de vue fongicide d'un composé de formule I

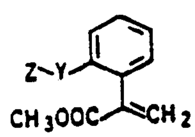

(I)

dans laquelle

Y représente alkylène en $C_1$-$C_4$ éventuellement substitué, alcénylène en $C_2$-$C_4$ éventuellement substitué, alcynylène en $C_2$-$C_4$, O,S(O)$_m$ (m = 0, 1, 2), N éventuellement substitué par alkyle en $C_1$-$C_4$, oxycarbonyle, car-